(19) Europäisches Patentamt
European Patent Office
Office européen des brevets

(11) Veröffentlichungsnummer: **0 298 452 B1**

(12) **EUROPÄISCHE PATENTSCHRIFT**

(45) Veröffentlichungstag der Patentschrift: **30.06.93**

(21) Anmeldenummer: **88110789.0**

(22) Anmeldetag: **06.07.88**

Die Akte enthält technische Angaben, die nach dem Eingang der Anmeldung eingereicht wurden und die nicht in dieser Patentschrift enthalten sind.

(51) Int. Cl.5: **C07D 405/04**, C07D 311/58, C07D 311/62, C07D 213/89, A61K 31/44, A61K 31/47, A61K 31/505, A61K 31/35

(54) **In 4-Stellung durch Aryl oder N-Heteroaryl substituierte 2H-1-Benzopyran-Derivate.**

(30) Priorität: **06.07.87 GB 8715839**
**29.04.88 GB 8810212**

(43) Veröffentlichungstag der Anmeldung:
**11.01.89 Patentblatt 89/02**

(45) Bekanntmachung des Hinweises auf die Patenterteilung:
**30.06.93 Patentblatt 93/26**

(84) Benannte Vertragsstaaten:
**AT BE CH DE ES FR GB GR IT LI LU NL SE**

(56) Entgegenhaltungen:
**EP-A- 0 107 423**
**EP-A- 0 218 373**
**EP-A- 0 296 975**

**CHEMICAL ABSTRACTS, Band 101, 1984, Seite 630, Nr. 210927n, Columbus, Ohio, US; I. SHARMA et al.: "A study of Grignard reaction on 3,4-diarylcoumarins: part II - effect of substituents on chromene formation in Grignard reaction of 4-(2-hydroxyphenyl)-3-phenylcoumarin"**

(73) Patentinhaber: **F. HOFFMANN-LA ROCHE AG**
**Postfach 3255**
**CH-4002 Basel(CH)**

(72) Erfinder: **Attwood, Michael Richard**
**1A Benslow Lane**
**Hitchin, Hertfordshire(GB)**
Erfinder: **Jones, Philip Stephen**
**35 Chapman Road**
**Stevenage, Hertfordshire(GB)**
Erfinder: **Redshaw, Sally**
**29 Hertford Road**
**Stevenage, Hertfordshire(GB)**

(74) Vertreter: **Lederer, Franz, Dr. et al**
**Patentanwalt Dr. Franz Lederer Lucile-Grahn-Strasse 22**
**W-8000 München 80 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 298 452 B1

## Beschreibung

Die vorliegende Erfindung betrifft Benzopyranderivate, ein Verfahren zu deren Herstellung sowie Arzneimittel enthaltend diese Derivate.

Die erfindungsgemässen Benzopyranderivate sind Verbindungen der allgemeinen Formeln

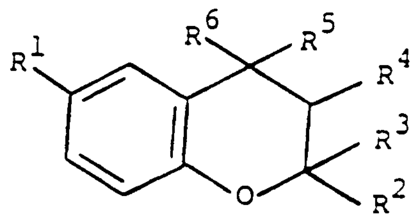

I

worin $R^1$ Wasserstoff, Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl, $R^4$ Wasserstoff oder Hydroxy und $R^5$ Wasserstoff oder $R^4$ und $R^5$ zusammen eine Kohlenstoff-Kohlenstoff-Bindung und $R^6$ eine Arylgruppe, welche eine Hydroxygruppe in in 2-Stellung trägt, eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist 2-Hydroxy-3-pyridyl, 2-Hydroxy-4-methyl-3-pyridyl, 3-Hydroxy-4-pyridyl, 2-Pyrazinyl-1-oxid, 2-Pyrimidinyl-1-oxid, 6-Py-rimidinyl-1-oxid oder 2-Chinolyl-1-oxid bedeuten, wobei die als "nieder" bezeichneten Reste 1-7 Kohlenstoffatome aufweisen und "Aroyl" gegebenenfalls durch Halogen oder Nitro substituiertes Benzoyl und "Aryl" gegebenenfalls durch Halogen, Cyan oder nieder Alkyl ein- oder mehrfach substituiertes Phenyl oder Naphthyl bedeuten, und pharmazeutisch verwendbare Säureadditionssalze der Verbindungen der Formel I, welche basisch sind.

Diese Benzopyranderivate sind neu und besitzen wertvolle pharmakodynamische Eigenschaften.

In EP-A-107 423, EP-A-218 373 und EP-A-296 975 sind Benzopyranderivate beschrieben, welche ebenfalls eine blutdrucksenkende Wirkung entfalten. Die anmeldegemässen Benzopyranderivate unterscheiden sich durch die Anwesenheit eines Arylrestes oder eines über ein Kohlenstoffatom gebundenen Heteroarylrestes in 4-Stellung des Benzopyrans in signifikanter Weise von den vorbekannten Verbindungen. Ausserdem sind in C.A. 101 (1984), 210927n Benzopyranderivate beschrieben, welche sich durch die Anwesenheit eines Phenylrestes in 3-Stellung des Benzopyrans ebenfalls in signifikanter Weise von den anmeldegemässen Verbindungen unterscheiden.

Gegenstand der vorliegenden Erfindung sind die Verbindungen der obigen Formel I und deren vorerwähnte Salze per se, ein Verfahren und Zwischenprodukte zu deren Herstellung, Arzneimittel enthaltend die Verbindungen der Formel I und deren vorerwähnte Salze sowie die Verwendung der Verbindungen der Formel I und deren vorerwähnten Salze zur Herstellung eines Arzneimittels zur Bekämpfung bzw. Verhütung von Bluthochdruck, dekompensierter Herzinsuffizienz, Angina pectoris, peripheren und cerebralen Gefässerkrankungen und Störungen der glatten Muskulatur.

Der in der vorliegenden Beschreibung verwendete Ausdruck "nieder Alkyl" - allein oder in Kombination - bedeutet einen geradkettigen oder verzweigten Alkylrest mit 1-7, vorzugsweise 1-4, Kohlenstoffatomen, wie Methyl, Aethyl, n-Propyl, Isopropyl, n-Butyl, Isobutyl, s-Butyl, t-Butyl, n-Pentyl und dergleichen, Methylthio, Aethylthio und dergleichen sind Beispiele von nieder Alkylthiogruppen und Methylsulfonyl, Aethylsulfonyl und dergleichen sind Beispiele von nieder Alkylsulfonylgruppen. Der Ausdruck "nieder Alkoxy" - allein oder in Kombination - bedeutet eine nieder Alkylgruppe, wie sie weiter oben definiert wurde, welche über ein Sauerstoffatom gebunden ist. Beispiele von nieder Alkoxygruppen sind Methoxy, Aethoxy, n-Propoxy, Isopropoxy, n-Butoxy und dergleichen, und Beispiele von nieder Alkoxycarbonylgruppen sind Methoxycarbonyl, Aethoxycarbonyl und dergleichen. Der Ausdruck "$C_{2-7}$-Alkanoyl" bedeutet eine primäre oder sekundäre Alkanoylgruppe mit 2 bis 7 Kohlenstoffatomen, wie Acetyl, Propionyl, Butyryl, Isobutyryl und dergleichen. Beispiele für "Aroyl" sind p-Nitrobenzoyl oder o-, m- oder p-Jodbenzoyl. Methylcarbamoyl, Aethylcarbamoyl und dergleichen sind Beispiele von Mono(nieder alkyl)carbamoylgruppen und Dimethylcarbamoyl, Diäthylcarbamoyl und dergleichen sind Beispiele von Di(nieder alkyl)-carbamoylgruppen.

Beispiele der mit $R^6$ bezeichneten Arylgruppen sind 2-Hydroxyphenyl, 4-Chlor-2-hydroxyphenyl, 2-Hydroxy-6-methylphenyl, 4-Cyan-2-hydroxyphenyl, 2-Hydroxynaphthyl und dergleichen. Beispiele der mit

$R^6$ bezeichneten 2-Pyridyl-1-oxidgruppe sind 2-Pyridyl-N-oxid, 3-Chlor-2-pyridyl-N-oxid, 4-Chlor-2-pyridyl-N-oxid, 5-Chlor-2-pyridyl-N-oxid, 6-Chlor-2-pyridyl-N-oxid, 5-Amino-2-pyridyl-N-oxid, 6-Amino-2-pyridyl-N-oxid, 5-Hydroxy-2-pyridyl-N-oxid, 5-Benzyloxy-2-pyridyl-N-oxid, 5-Phenyl-2-pyridyl-N-oxid, 5-(4-Methylphenyl)-2-pyridyl-N-oxid, 3-Methyl-2-pyridyl-N-oxid, 4-Methyl-2-pyridyl-N-oxid, 5-Methyl-2-pyridyl-N-oxid, 6-Methyl-2-pyridyl-N-oxid, 4-Methoxy-2-pyridyl-N-oxid, 5-Methoxycarbonyl-2-pyridyl-N-oxid und dergleichen.

Wenn $R^2$ und $R^3$ in Formel I verschiedene Bedeutungen besitzen, können die Verbindungen in racemischer oder optisch reiner Form vorliegen. Im weiteren können die Verbindungen der Formel I in racemischer oder optisch reiner Form vorliegen, wenn $R^4$ und $R^5$ je Wasserstoff oder $R^4$ Hydroxy und $R^5$ Wasserstoff bedeuten. Falls $R^2$ und $R^3$ verschiedene Bedeutungen besitzen und entweder $R^4$ und $R^5$ je Wasserstoff oder $R^4$ Hydroxy und $R^5$ Wasserstoff bedeuten, können die Verbindungen der Formel I in verschiedenen diastereomeren Formen vorliegen. Im weiteren kann cis/trans-Isomerie bei solchen Verbindungen der Formel I auftreten, worin $R^4$ Hydroxy und $R^5$ Wasserstoff bedeuten. Wenn $R^6$ in Formel I 2-Hydroxy-3-pyridyl, 2-Hydroxy-4-methyl-3-pyridyl oder 3-Hydroxy-4-pyridyl bedeutet, können die Verbindungen in tautomeren Formen vorliegen. Die vorliegende Erfindung umfasst alle diese möglichen Formen.

Eine besondere Untergruppe von Verbindungen der Formel I sind solche, worin $R^1$ Wasserstoff, Halogen, Nitro, Cyan, nieder Alkyl, nieder Alkoxycarbonyl, nieder Alkylthio, nieder Aklylsulfonyl, $C_{2-7}$-Alkanoyl, Carbamoyl, Mono(nieder alkyl)carbamoyl oder Di(nieder alkyl)carbamoyl, $R^2$ und $R^3$ je Wasserstoff oder nieder Alkyl und $R^4$ und $R^5$ je Wasserstoff oder zusammen eine Kohlenstoff-Kohlenstoff-Bindung bedeuten.

In den erfindungsgemässen Verbindungen bedeutet $R^1$ vorzugsweise Nitro, Cyan oder $C_{2-7}$-Alkanoyl, besonders bevorzugt Nitro, Cyan oder Acetyl. $R^2$ und $R^3$ bedeuten vorzugsweise nieder Alkyl, besonders bevorzugt Methyl. Vorzugsweise bedeuten $R^4$ und $R^5$ Wasserstoff oder zusammen eine zusätzliche Kohlenstoff-Kohlenstoff-Bindung. $R^6$ bedeutet vorzugsweise eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist.

Aus dem obigen folgt, dass besonders bevorzugte Verbindungen der Formel I solche sind, worin $R^1$ Nitro, Cyan oder Acetyl, $R^2$ und $R^3$ je Methyl, $R^4$ und $R^5$ je Wasserstoff oder zusammen eine Kohlenstoff-Kohlenstoff-Bindung und $R^6$ eine 2-Pyridyl-1-oxidgruppe bedeuten, welche gegebenenfalls durch Halogen, Amino, Hydroxy, Benzyloxy, Phenyl, nieder-Alkylphenyl, nieder Alkyl, nieder Alkoxy oder nieder Alkoxycarbonyl substituiert ist.

Ganz speziell bevorzugte Verbindungen der Formel I sind:
2-(3,4-Dihydro-2,2-dimethyl-6-nitro-2H-1-benzopyran-4-yl)pyridin-N-oxid,
2-(6-Acetyl-3,4-dihydro-2,2-dimethyl-2H-1-benzopyran-4-yl)pyridin-N-oxid,
2-(6-Cyan-3,4-dihydro-2,2-dimethyl-2H-1-benzopyran-4-yl)pyridin-N-oxid und
2-(6-Cyan-3,4-dihydro-2,2-dimethyl-2H-1-benzopyran-4-yl)-5-phenylpyridin-N-oxid.

Weitere bevorzugte Verbindungen der Formel I sind:
2-(6-Acetyl-3,4-dihydro-2,2-dimethyl-2H-1-benzopyran-4-yl)-3-methylpyridin-N-oxid,
2-(3,4-Dihydro-2,2-dimethyl-6-nitro-2H-1-benzopyran-4-yl)-3-methylpyridin-N-oxid,
2-(6-Cyan-2,2-dimethyl-2H-1-benzopyran-4-yl)pyridin-N-oxid,
2-(3,4-Dihydro-2,2-dimethyl-2H-1-benzopyran-4-yl)pyridin-N-oxid,
2-(3,4-Dihydro-2,2-dimethyl-6-methylthio-2H-1-benzopyran-4-yl)pyridin-N-oxid,
2-(3,4-Dihydro-2,2-dimethyl-6-methylsulfonyl-2H-1-benzopyran-4-yl)pyridin-N-oxid,
2-(2,2-Dimethyl-2H-1-benzopyran-4-yl)pyridin-N-oxid,
2-(6-Brom-3,4-dihydro-2,2-dimethyl-2H-1-benzopyran-4-yl)pyridin-N-oxid,
2-[6-(Methoxycarbonyl)-2,2-dimethyl-2H-1-benzopyran-4-yl] pyridin-N-oxid,
2-[3,4-Dihydro-6-(methoxycarbonyl)-2,2-dimethyl-2H-1-benzopyran-4-yl] pyridin-N-oxid,
2-(6-Carbamoyl-3,4-dihydro-2,2-dimethyl-2H-1-benzopyran-4-yl)pyridin-N-oxid,
2-(6-Cyan-3,4-dihydro-2,2-dimethyl-2H-1-benzopyran-4-yl)pyrimidin-1-oxid,
2-(6-Cyan-2,2-dimethyl-2H-1-benzopyran-4-yl)chinolin-1-oxid,
2-(6-Cyan-3,4-dihydro-2,2-dimethyl-2H-1-benzopyran-4-yl)chinolin-1-oxid,
4-(2-Hydroxyphenyl)-2,2-dimethyl-2H-1-benzopyran-6-carbonitril,
4-(5-Cyan-2-hydroxyphenyl)-2,2-dimethyl-2H-1-benzopyran-6-carbonitril und
3-(6-Cyan-3,4-dihydro-2,2-dimethyl-2H-1-benzopyran-4-yl)-2(1H)-pyridon.

Beispiele weiterer interessanter Verbindungen der Formel I sind:
2-(6-Chlor-3,4-dihydro-2,2-dimethyl-2H-1-benzopyran-4-yl)pyridin-N-oxid,
2-(2,2,6-Trimethyl-2H-1-benzopyran-4-yl)pyridin-N-oxid,
2-[6-(Trifluormethyl)-2,2-dimethyl-2H-1-benzopyran-4-yl]pyridin-N-oxid,
2-[6-(t-Butyl)-3,4-dihydro-2,2-dimethyl-2H-1-benzopyran-4-yl]pyridin-N-oxid,

2-(6-Benzoyl-3,4-dihydro-2,2-dimethyl-2H-1-benzopyran-4-yl)-pyridin-N-oxid,
2-[3,4-Dihydro-2,2-dimethyl-6-(4-nitrobenzoyl)-2H-1-benzopyran-4-yl]pyridin-N-oxid,
2-[3,4-Dihydro-2,2-dimethyl-6-(2-jodbenzoyl)-2H-1-benzopyran-4-yl]pyridin-N-oxid,
2-[3,4-Dihydro-2,2-dimethyl-6-(3-jodbenzoyl)-2H-1-benzopyran-4-yl]pyridin-N-oxid,
2-[3,4-Dihydro-2,2-dimethyl-6-(4-jodbenzoyl)-2H-1-benzopyran-4-yl]pyridin-N-oxid,
2-(6-Cyan-2-äthyl-2-methyl-2H-1-benzopyran-4-yl)pyridin-N-oxid,
2-(6-Acetyl-2-methyl-2H-1-benzopyran-4-yl)pyridin-N-oxid,
2-Chlor-6-(6-cyan-3,4-dihydro-2,2-dimethyl-2H-1-benzopyran-4-yl)pyridin-N-oxid,
4-Chlor-2-(6-cyan-3,4-dihydro-2,2-dimethyl-2H-1-benzopyran-4-yl)pyridin-N-oxid,
2-(6-Cyan-3,4-dihydro-2,2-dimethyl-2H-1-benzopyran-4-yl)-4-methoxypyridin-N-oxid
2-Amino-6-(6-cyan-2,2-dimethyl-2H-1-benzopyran-4-yl)pyridin-N-oxid,
2-Amino-6-(6-cyan-3,4-dihydro-2,2-dimethyl-2H-1-benzopyran-4-yl)pyridin-N-oxid,
2-(6-Cyan-3,4-dihydro-2,2-dimethyl-2H-1-benzopyran-4-yl)-6-methylpyridin-N-oxid,
2-(6-Cyan-2,2-dimethyl-2H-1-benzopyran-4-yl)-4-methylpyridin-N-oxid,
2-(6-Cyan-3,4-dihydro-2,2-dimethyl-2H-1-benzopyran-4-yl)-4-methylpyridin-N-oxid,
2-(6-Cyan-3,4-dihydro-2,2-dimethyl-2H-1-benzopyran-4-yl)-5-(methoxycarbonyl)pyridin-N-oxid,
5-Amino-2-(6-cyan-2,2-dimethyl-2H-1-benzopyran-4-yl)pyridin-N-oxid,
5-Amino-2-(6-cyan-3,4-dihydro-2,2-dimethyl-2H-1-benzopyran-4-yl)pyridin-N-oxid,
2-(6-Cyan-2,2-dimethyl-2H-1-benzopyran-4-yl)-5-hydroxypyridin-N-oxid,
5-Chlor-2-(6-cyan-2,2-dimethyl-2H-1-benzopyran-4-yl)pyridin-N-oxid,
5-Chlor-2-(6-cyan-3,4-dihydro-2,2-dimethyl-2H-1-benzopyran-4-yl)pyrdin-N-oxid,
2-(6-Cyan-2,2-dimethyl-2H-1-benzopyran-4-yl)-5-phenylpyridin-N-oxid,
2-(6-Acetyl-2,2-dimethyl-2H-1-benzopyran-4-yl)pyridine-N-oxid,
2-(6-Cyan-2,2-dimethyl-2H-1-benzopyran-4-yl)-5-methylpyridin-N-oxid,
2-(6-Cyan-3,4-dihydro-2,2-dimethyl-2H-1-benzopyran-4-yl)-5-methylpyridin-N-oxid,
2-(6-Brom-2,2-dimethyl-2H-1-benzopyran-4-yl)pyridin-N-oxid,
2-(6-Cyan-2,2-dimethyl-2H-1-benzopyran-4-yl)-5-(4-methylphenyl)pyridin-N-oxid,
2-(6-Acetyl-2-methyl-2-phenyl-2H-1-benzopyran-4-yl)pyridin-N-oxid,
5-Benzyloxy-2-(6-cyan-3,4-dihydro-2,2-dimethyl-2H-1-benzopyran-4-yl)pyridin-N-oxid,
2-(6-Cyan-3,4-dihydro-2,2-dimethyl-2H-1-benzopyran-4-yl)-5-hydroxypyridin-N-oxid,
rac-trans-2-(6-Cyan-3,4-dihydro-3-hydroxy-2,2-dimethyl-2H-1-benzopyran-4-yl)pyridin-N-oxid,
rac-cis-2-(6-Cyan-3,4-dihydro-3-hydroxy-2,2-dimethyl-2H-1-benzopyran-4-yl)pyridin-N-oxid,
6-(6-Cyan-3,4-dihydro-2,2-dimethyl-2H-1-benzopyran-4-yl)pyrimidin-1-oxid,
2-(6-Cyan-3,4-dihydro-2,2-dimethyl-2H-1-benzopyran-4-yl)pyrazin-1-oxid,
4-(6-Acetyl-2,2-dimethyl-2H-1-benzopyran-4-yl)-3-pyridinol,
4-(2,2-Dimethyl-2H-1-benzopyran-4-yl)-3-pyridinol,
(-)-2-(6-Cyan-3,4-dihydro-2,2-dimethyl-2H-1-benzopyran-4-yl)pyridin-N-oxid und
(+)-2-(6-Cyan-3,4-dihydro-2,2-dimethyl-2H-1-benzopyran-4-yl)pyridin-N-oxid.

Die Verbindungen der Formel I und die pharmazeutisch verwendbaren Säureadditionssalze derjenigen Verbindungen, welche basisch sind, können erfindungsgemäss dadurch hergestellt werden, dass man

a) zur Herstellung einer Verbindung der Formel I, worin $R^6$ eine Aryl gruppe mit einer Hydroxygruppe in 2-Stellung 2-Hydroxy-3-pyridyl, 2-Hydroxy-4-methyl-3-pyridyl oder 3-Hydroxy-4-pyridyl bedeutet, die nieder Alkoxygruppe in einer Verbindung der allgemeinen Formel

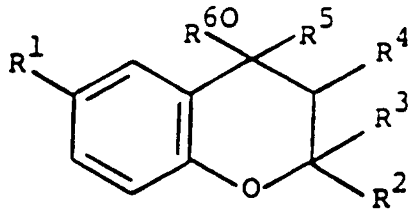

II

worin $R^1$, $R^2$, $R^3$, $R^4$ und $R^5$ die oben angegebene Bedeutung besitzen und $R^{60}$ eine Arylgruppe mit einer nieder Alkoxygruppe in 2-Stellung, 2-nieder Alkoxy-3-pyridyl, 2-nieder Alkoxy-4-methyl-3-pyridyl oder 3-nieder Alkoxy-4-pyridyl bedeutet,
in eine Hydroxygruppe überführt, oder

b) zur Herstellung einer Verbindung der Formel I, worin $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet, eine Verbindung der allgemeinen Formel

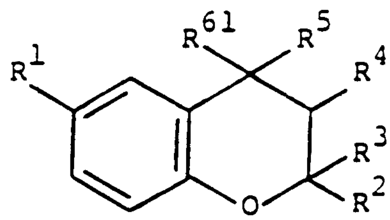

III

worin $R^1$, $R^2$, $R^3$, $R^4$ und $R^5$ die oben angegebene Bedeutung besitzen und $R^{61}$ eine 2-Pyridylgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl, 2-Pyrimidinyl, 6-Pyrimidinyl oder 2-Chinolyl bedeutet,
oxidiert, oder
c) zur Herstellung einer Verbindung der Formel I, worin $R^6$ 2-Hydroxy-3-pyridyl oder 2-Hydroxy-4-methyl-3-pyridyl bedeutet, eine Verbindung der allgemeinen Formel

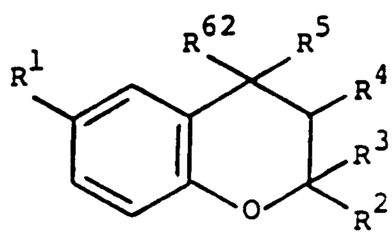

IV

worin $R^1$, $R^2$, $R^3$, $R^4$ und $R^5$ die oben angegebene Bedeutung besitzen und $R^{62}$ 3-Pyridyl-1-oxid oder 4-Methyl-3-pyridyl-1-oxid bedeutet,
mit einem nieder Alkansäureanhydrid umsetzt und das erhaltene Produkt hydrolysiert, oder
d) zur Herstellung einer Verbindung der Formel I, worin $R^4$ und $R^5$ je Wasserstoff und $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeuten, eine Verbindung der allgemeinen Formel

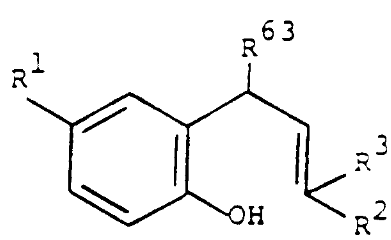

V

worin $R^1$, $R^2$, und $R^3$ die oben angegebene Bedeutung besitzen und $R^{63}$ 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet,
cyclisiert, oder
e) zur Herstellung einer Verbindung der Formel I, worin $R^1$ nieder Alkylsulfonyl bedeutet, eine Verbindung der Formel I, worin $R^1$ nieder Alkylthio bedeutet, oxidiert, oder

f) zur Herstellung einer Verbindung der Formel I, worin $R^1$ $C_{2-7}$-Alkanoyl oder Aroyl bedeutet, eine Verbindung der Formel I, worin $R^1$ Wasserstoff bedeutet, $C_{2-7}$-alkanoyliert oder aroyliert, oder

g) zur Herstellung einer Verbindung der Formel I, worin $R^6$ eine 2-Pyridyl-1-oxidgruppe mit einer nieder Alkoxygruppe in o- oder p-Stellung zur 1-Oxidgruppe bedeutet, eine Verbindung der Formel I, worin $R^6$ eine 2-Pyridyl-1-oxidgruppe mit einem Halogenatom in o- oder p-Stellung zur 1-Oxidgruppe bedeutet, mit einem Alkalimetall-nieder-alkoxid bei erhöhter Temperatur umsetzt, oder

h) zur Herstellung einer Verbindung der Formel I, worin $R^4$ und $R^5$ je Wasserstoff und $R^6$ eine Hydroxy-substituierte 2-Pyridyl-1-oxidgruppe bedeuten, eine Verbindung der Formel I, worin $R^4$ und $R^5$ je Wasserstoff und $R^6$ eine Benzyloxy-substituierte 2-Pyridyl-1-oxidgruppe bedeuten, katalytisch hydriert, oder

i) zur Herstellung einer Verbindung der Formel I, worin $R^4$ und $R^5$ zusammen eine Kohlenstoff-Kohlenstoff-Bindung und $R^6$ eine 3-Hydroxy-4-pyridylgruppe bedeuten, ein 3-[N,N-Di(nieder alkyl)-carbamoyloxy]pyridin mit einer Verbindung der allgemeinen Formel

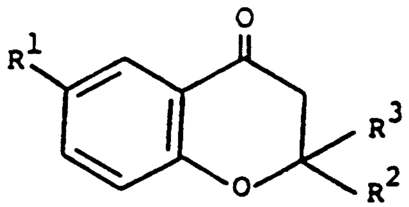

VI

worin $R^1$, $R^2$ und $R^3$ die oben angegebene Bedeutung besitzen,
in Gegenwart einer Alkalimetallalkylverbindung umsetzt, und/oder

j) erwünschtenfalls, ein erhaltenes Gemisch von diastereomeren Racematen in die entsprechenden diastereomeren Racemate oder optisch reinen Diastereomeren auftrennt, und/oder

k) erwünschtenfalls, ein erhaltenes Racemat in die beiden optischen Antipoden auftrennt, und/oder

l) erwünschtenfalls, ein erhaltenes cis/trans-Gemisch in die cis- und trans-Isomeren auftrennt, und

m) erwünschtenfalls, eine erhaltene basische Verbindung der Formel I in ein pharmazeutisch verwendbares Säureadditionssalz überführt.

Die Ueberführung einer nieder Alkoxygruppe, vorzugsweise einer Methoxygruppe, in einer Verbindung der Formel II in eine Hydroxygruppe gemäss Verfahrensvariante a) erfolgt in an sich bekannter Weise. Eine geeignete Ausführungsform betrifft die Behandlung einer Verbindung der Formel II mit einem Alkalimetall-nieder-alkanthiolat, z.B. Natriummethanthiolat, zweckmässigerweise in einem inerten organischen Lösungsmittel, wie Dimethylformamid, und bei erhöhter Temperatur, z.B. ungefähr 100 °C. Die Ueberführung kann jedoch auch unter Verwendung anderer Reagentien, wie Lithiumjodid, einem Tri(nieder alkyl)silylhalogenid, z .B. Trimethylsilyljodid, Bortribromid und dergleichen durchgeführt werden.

Bekannte Verfahren können für die Oxidation einer Verbindung der Formel III gemäss Verfahrensvariante b) verwendet werden. So kann beispielsweise eine Verbindung der Formel III durch Umsetzen mit Wasserstoffperoxid, einer organischen Persäure, wie Peressigsäure, Perbenzoesäure, m-Chlorperbenzoesäure, Perphthalsäure und dergleichen, einem Perester, Natriummetaperjodat, Natriumperborat in Essigsäure und dergleichen, oxidiert werden. Die Oxidation wird zweckmässigerweise in einem inerten organischen Lösungsmittel, wie einem halogenierten Kohlenwasserstoff, z. B. Chloroform, Dichlormethan, Dichloräthan und dergleichen durchgeführt. Wenn Wasserstoffperoxid als Oxidationsmittel verwendet wird, kann die Oxidation auch in Essigsäure und dergleichen durchgeführt werden. Zweckmässigerweise wird die Oxidation bei einer Temperatur zwischen ungefähr 0 °C und ungefähr 30 °C, vorzugsweise bei ungefähr Raumtemperatur, durchgeführt.

Verfahrensvariante c), d.h. die Umsetzung einer Verbindung der Formel IV mit einem nieder Alkansäureanhydrid und nachfolgender Hydrolyse der erhaltenen Produkts, kann nach an sich bekannten Methoden durchgeführt werden. Beispielsweise kann man eine Verbindung der Formel IV mit einem Anhydrid, vorzugsweise Essigsäureanhydrid, bei erhöhter Temperatur, vorzugsweise bei der Rückflusstemperatur des Reaktionsgemisches, umsetzen. Das erhaltene Produkt, nämlich eine Verbindung, die der Formel I entspricht, worin $R^6$ jedoch 2-Alkanoyloxy-3-pyridyl oder 2-Alkanoyloxy-4-methyl-3-pyridyl bedeutet, kann danach durch Umsetzen mit einer Säure oder einer Base in bekannter Weise hydrolysiert werden. Die Säurehydrolyse kann unter Verwendung einer wässerigen Mineralsäure, z.B. Salzsäure, Bromwasserstoffsäure, Schwefelsäure und dergleichen, oder einer organischen Säure, z.B. p-Toluolsulfonsäure und dergleichen, zweckmässigerweise in einem Wasser enthaltenden inerten organischen Lösungsmittel, wie Tetrahydrofuran, Dioxan und dergleichen, und bei ungefähr Raumtemperatur durchgeführt werden. Die basische Hydrolyse kann unter Verwendung eines Alkalimetallhydroxids, wie Natriumhydroxid, oder eines Alkalimetall-nieder-alkoxids, z.B. Natriummäthoxid, Natriumethoxid und degleichen, zweckmässigerweise in einem inerten organischen Lösungsmittel, wie einem niederen Alkanol, z.B. Methanol, Aethanol und dergleichen, bei Raumtemperatur durchgeführt werden.

Die Cyclisation einer Verbindung der Formel V gemäss Verfahrensvariante d) kann zweckmässigerweise durch Umsetzen mit einer Säure erfolgen, vorteilhafterweise einer anorganischen Säure, wie Schwefelsäure, und zweckmässigerweise in einem inerten organischen Lösungsmittel, wie einem halogenierten Kohlenwasserstoff, z.B. Dichlormethan, Chloroform, Dichloräthan und dergleichen. Es ist zweckmässig, die Reaktion bei ungefähr Raumtemperatur durchzuführen. Die Cyclisation erfolgt sehr leicht, und unter gewissen Umständen mag es von Vorteil oder sogar nötig sein, die Reaktion in situ durchzuführen.

Die Oxidation einer Verbindung der Formel I, worin $R^1$ nieder Alkylthio bedeutet, gemäss Verfahrensvariante e) kann in bekannter Weise durchgeführt werden. Z.B. kann die Oxidation durch Umsetzen mit Wasserstoffperoxid, einer organischen Persäure, wie Peressigsäure, Perbenzoesäure, m-Chlorperbenzoesäure, Perphthalsäure und dergleichen, einem Perester, Natriummetaperjodat, Natriumperborat in Essigsäure und dergleichen, erfolgen. Die Oxidation wird zweckmässigerweise in einem inerten organischen Lösungsmittel, wie einem halogenierten Kohlenwasserstoff, z.B. Chloroform, Dichlormethan, Dichloräthan und dergleichen, durchgführt. Wenn Wasserstoffperoxid als Oxidationsmittel verwendet wird, kann die Reaktion auch in Essigsäure und dergleichen durchgeführt werden. Der Einfachheit halber wird die Oxidation bei einer Temperatur zwischen ungefähr 0 °C und ungefähr 30 °C, vorzugsweise bei ungefähr Raumtemperatur, durchgeführt.

Die $C_{2-7}$-Alkanoylierung oder Aroylierung gemäss Verfahrensvariante f) kann unter den gut bekannten Reaktionsbedingungen einer Friedel-Crafts-Reaktion erfolgen. So kann beispielsweise eine Verbindung der Formel I, worin $R^1$ Wasserstoff bedeutet, mit einem geeigneten $C_{2-7}$-Alkanoylhalogenid, z.B. Acetylchlorid, oder einem Aroylhalogenid, z.B. Benzoylchlorid, 2-Jodbenzoylchlorid, 4-Nitrobenzoylchlorid und dergleichen, in Gegenwart eines Lewis-Säurekatalysator, z.B. Aluminiumchlorid, zweckmässigerweise in einem inerten organischen Lösungsmittel, z.B. Nitromethan, umgesetzt werden. Zweckmässigerweise wird die Reaktion bei einer Temperatur zwischen ungefähr 0 °C und der Raumtemperatur durchgeführt, obwohl sie auch bei einer höheren Temperatur durchgeführt werden kann, falls notwendig.

Der Ersatz eines Halogenatoms durch eine nieder Alkoxygruppe gemäss Verfahrensvariante g) erfolgt in bekannter Weise. Die Umsetzung der entsprechenden Verbindung der Formel I, vorzugsweise einer, worin das Halogenatom das Chloratom ist, mit einem Alkalimetall-nieder-alkoxid, z.B. Natriummethoxid, Natriumäthoxid und dergleichen, wird zweckmässigerweise in einem inerten organischen Lösungsmittel, vorzugsweise einem nieder Alkanol, welches dem verwendeten Alkalimetall-nieder-alkoxid entspricht, durchgeführt. Die Reaktion erfolgt vorzugsweise bei der Rückflusstemperatur des Reaktionsgemisches und unter einer Inertgasatmosphäre, z.B. Stickstoff.

Die katalytische Hydrierung gemäss Verfahrensvariante h) kann in bekannter Weise durchgeführt werden. Die katalytische Hydrierung erfolgt zweckmässigerweise in einem inerten organischen Lösungsmittel, wie einem nieder Alkanol, z.B. Methanol, unter Verwendung eines Edelmetallkatalysators, wie eines Palladium- oder Platinkatalysators, welche auch auf einem geeigneten Trägermaterial aufgetragen sein kann, z.B. Palladium auf Kohle. Es ist zweckmässig, die katalytische Hydrierung bei ungefähr Raumtemperatur und unter Atmosphärendruck durchzuführen.

Der Verfahrensaspekt i) wird zweckmässigerweise so durchgeführt, dass man zuerst ein 3-[N,N-di-(nieder alkyl)carbamoyloxy]pyridin, speziell 3-(N,N-Diäthylcarbamoyloxy)pyridin, mit einer Alkalimetallalkylverbindung, speziell N-Butyllithium, umsetzt und danach eine Verbindung der Formel VI zugibt. Die Umsetzung wird zweckmässigerweise in einem inerten organischen Lösungsmittel oder Lösungsmittelgemisch durchgeführt, wie einem aliphatischen Kohlenwasserstoff, z.B. Hexan, oder einem cyclischen Aether, z.B. Tetrahydrofuran, oder einem Gemisch davon. Die Reaktion erfolgt vorteilhafterweise bei einer Temperatur zwischen ungefähr -78 °C und Raumtemperatur.

Die Auftrennung eines Gemisches diastereomerer Racemate gemäss Verfahrensvariante j), die Auftrennung eines Racemates gemäss Verfahrensvariante k) und die Auftrennung eines cis/trans-Gemisches gemäss Verfahrensvariante l) können nach bekannten Methoden durchgeführt werden, z.B. durch Chromatographie unter Verwendung eines geeigneten Lösungsmittelsystems, wobei für den Fall der Auftrennung eines Racemates das Medium für die Chromatographie chiral sein muss, z.B. $\beta$-Cyclodextrin an Silicagel gebunden. Im weiteren kann ein saures Racemat auch unter Verwendung einer chiralen Base, z.B. Chinin, und ein basisches Racemat unter Verwendung einer chiralen Säure, z.B. Camphersulfonsäure, aufgetrennt werden. Ein weiteres Verfahren zur Auftrennung von Diastereomeren und von cis/trans-Isomeren ist die Kristallisation aus einem geeigneten Lösungsmittelsystem.

Gemäss Verfahrensvariante m) wird eine basische Verbindung der Formel I , d.h. eine Verbindung, worin $R^6$ eine aminosubstituierte 2-Pyridyl-1-oxidgruppe bedeutet, in ein pharmazeutisch verwendbares Säureadditionssalz übergeführt. Dies kann dadurch bewerkstelligt werden, indem man eine basische Verbindung der Formel I mit einer anorganischen Säure, wie einer Halogenwasserstoffsäure, z.B. Chlorwasserstoffsäure, Bromwasserstoffsäure oder Jodwasserstoffsäure, Schwefelsäure, Phosphorsäure, Salpetersäure und dergleichen oder einer organischen Säure, wie Essigsäure, Maleinsäure, Fumarsäure, Weinsäure, Citronensäure, Salicylsäure, Methansulfonsäure, p-Toluolsulfonsäure und dergleichen, umsetzt

Die Ausgangsstoffe der Formeln II, III und IV sind neu und ebenfalls Gegenstand der vorliegenden Erfindung. Diejenigen Ausgangsstoffe der Formeln II, III und IV, worin $R^4$ und $R^5$ je Wasserstoff oder zusammen eine Kohlenstoff-Kohlenstoff-Bindung bedeuten, können gemäss dem Reaktionschema I hergestellt werden. In diesem Schema besitzen $R^2$, $R^3$ und $R^{62}$ die oben angegebene Bedeutung, wahrend $R^7$ nieder Alkoxy, $R^{10}$ Wasserstoff, Halogen, Trifluormethyl, Nitro, Cyan, nieder Alkyl, nieder Alkylthio oder nieder Alkylsulfonyl und $R^{64}$ eine Arylgruppe mit einer nieder Alkoxygruppe in 2-Stellung, 2-nieder-Alkoxy-3-pyridyl, 2-nieder-Alkoxy-4-methyl-3-pyridyl, 3-nieder-Alkoxy-4-pyridyl, eine 2-Pyridylgruppe, welche gegebenenfalls durch Halogen, Amino, Hydroxy, Benzyloxy, Phenyl, nieder-Alkylphenyl, nieder-Alkyl, nieder-Alkoxy, nieder-Alkoxycarbonyl, Benzyloxycarbonyl oder Nitro substituiert ist, 2-Pyrazinyl, 2-Pyrimidinyl, 6-Pyrimidinyl, 2-Chinolyl, 3-Pyridyl oder 4-Methyl-3-pyridyl bedeuten. Es versteht sich von selbst, dass die in diesem Reaktionsschema vorkommenden Verbindungen der Formeln X und XI, worin $R^{64}$ eine Aryl gruppe mit einer nieder Alkoxygruppe in 2-Stellung oder 2-nieder-Alkoxy-3-pyridyl, 2-nieder-Alkoxy-4-methyl-3-pyridyl oder 3-nieder-Alkoxy-4-pyridyl bedeutet, dem Ausgangsmaterial der Formel II entsprechen, worin $R^4$ und $R^5$ je Wasserstoff oder $R^4$ und $R^5$ zusammen eine Kohlenstoff-Kohlenstoff-Bindung bedeuten, und dass die Verbindungen der Formeln X und XI, worin $R^{64}$ eine 2-Pyridylgruppe, welche gegebenenfalls durch Halogen, Amino, Hydroxy, Benzyloxy, Phenyl, nieder-Alkylphenyl, nieder-Alkyl, nieder-Alkoxy oder nieder-Alkoxycarbonyl substituiert ist, 2-Pyrazinyl, 2-Pyrimidinyl, 6-Pyrimidinyl oder 2-Chinolyl bedeutet, den Ausgansstoffen der Formel III entsprechen, worin $R^4$ und $R^5$ je Wasserstoff oder $R^4$ und $R^5$ zusammen eine Kohlenstoff-Kohlenstoff-Bindung bedeuten.

Reaktionsschema I

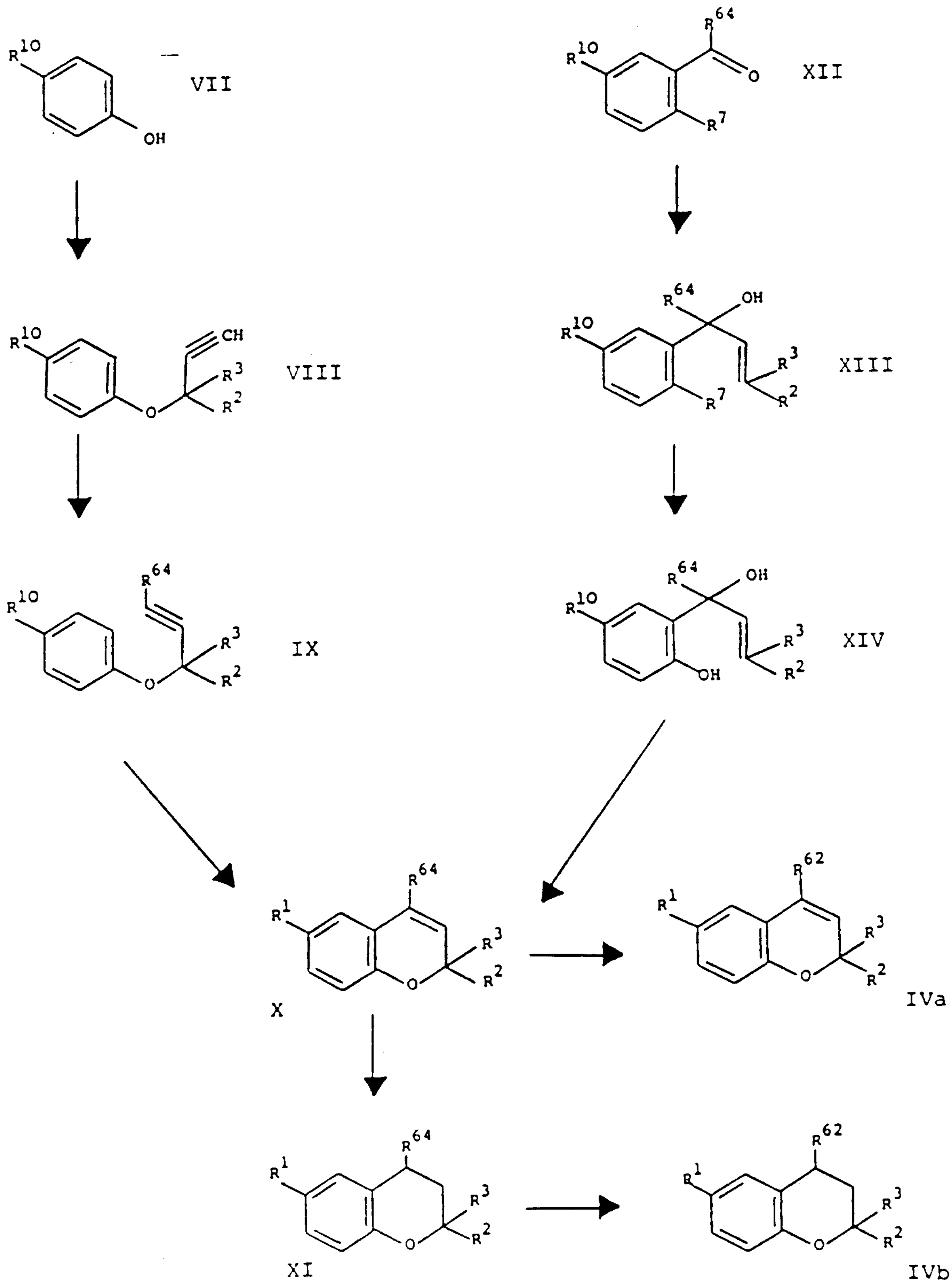

VII XII VIII XIII IX XIV X IVa XI IVb

Im Reaktionsschema I wird eine Verbindung der Formel VII, bei welcher es sich um eine bekannte Verbindung oder ein Analogon einer bekannten Verbindung handelt, mit einer Verbindung der allgemeinen Formel X-C($R^2$)($R^3$)-C≡CH, worin X Chlor, Brom oder Hydroxy bedeutet, zu einer Verbindung der Formel VIII umgesetzt. Die Umsetzung erfolgt in bekannter Weise. Wenn X Chlor oder Brom bedeutet, kann die Umsetzung beispielsweise in einem Gemisch von einem inerten organischen Lösungsmittel, wie einem

halogenierten aliphatischen Kohlenwasserstoff, z.B. Dichlormethan und dergleichen, und Wasser in Gegenwart einer Base, wie einem Alkalimetallhydroxid, z.B. Natriumhydroxid oder Kaliumhydroxid, und einem Phasentransferkatalysator, wie Benzyltrimethylammoniumhydroxid, zweckmässigerweise bei ungefähr Raumtemperatur durchgeführt werden. Wiederum beispielsweise kann die Reaktion, wenn X Hydroxy bedeutet, in einem inerten organischen Lösungsmittel, wie einem halogenierten aliphatischen Kohlenwasserstoff, z.B. Dichlormethan, in Gegenwart eines Kondensationsmittels, wie Diäthylazodicarboxylat/Triphenylphosphin, zweckmässigerweise bei ungefähr Raumtemperatur, durchgeführt werden.

Eine Verbindung der Formel VIII wird danach mit einer Verbindung der allgemeinen Formel $X^1$-$R^{64}$, worin $R^{64}$ die oben angegebene Bedeutung besitzt und $X^1$ Brom oder Jod bedeutet, in Gegenwart von Kupfer(I)jodid, einem Triarylphosphin, z.B. Triphenylphosphin, und Palladium(II)chlorid zu einer Verbindung der Formel IX umgesetzt. Die Umsetzung erfolgt zweckmässigerweise in Gegenwart eines Di(nieder alkyl)-amins, wie Diäthylamin, oder eines Tri(nieder alkyl)amins, wie Triäthylamin, und bei ungefähr Raumtemperatur. Unter gewissen Umständen mag es angebracht oder sogar nötig sein, die Reaktion unter einer Inertgasatmosphäre, z.B. Stickstoff, durchzuführen.

Eine Verbindung der Formel IX wird danach durch Erhitzen in einem inerten organischen Lösungsmittel, wie einem halogenierten aromatischen Kohlenwasserstoff, z.B. Chlorbenzol, 1,2-Dichlorbenzol und dergleichen, und vorzugsweise bei der Rückflusstemperatur des Reaktionsgemisches in eine Verbindung der Formel X übergeführt, worin $R^1$ Wasserstoff, Halogen, Trifluormethyl, Nitro, Cyan, nieder Alkyl, nieder Alkylthio oder nieder Alkylsulfonyl bedeutet.

Eine Verbindung der Formel X, worin $R^1$ Wasserstoff, Halogen, Trifluormethyl, Nitro, Cyan, nieder Alkyl, nieder Alkylthio oder nieder Alkylsulfonyl bedeutet, kann auch so hergestellt werden, dass man zunächst eine Verbindung der Formel XII, welche bekannt oder ein Analogon einer bekannten Verbindung ist, mit einer Verbindung der allgemeinen Formel $(R^2)(R^3)C=CH$-MgX, worin $R^2$, $R^3$ und X die oben angegebene Bedeutung besitzen, unter den üblichen Bedingungen einer Grignard-Reaktion zu einer Verbindung der Formel XIII umsetzt.

Die nieder Alkoxygruppe $R^7$ in einer erhaltenen Verbindung der Formel XIII wird danach in eine Hydroxygruppe übergeführt, wobei man eine Verbindung der Formel XIV erhält. Diese Ueberführung kann dadurch erfolgen, dass man eine Verbindung der Formel XIII mit einem Alkalimetall nieder alkanthiolat, z.B Natriummethanthiolat, zweckmässigerweise in einem inerten organischen Lösungsmittel, wie Dimethylformamid, und bei erhöhter Temperatur, z.B. ungefähr 100 °C, umsetzt. Die Ueberführung kann jedoch auch unter Verwendung anderer Reagentien, wie Lithiumjodid, einem Tri(nieder alkyl)silylhalogenid, z.B. Trimethylsilyljodid, Bortribromid und dergleichen, erfolgen. Es ist wohl klar, dass in einer Verbindung der Formel XIII, worin $R^{64}$ eine Aryl- oder 2-Pyridylgruppe mit einer nieder Alkoxygruppe in 2-Stellung bedeutet, die Alkoxygruppe während dieser Ueberführung ebenfalls in eine Hydroxygruppe übergeführt werden kann.

Eine so erhaltene Verbindung der Formel XIV wird danach durch Erhitzen, zweckmässigerweise in einem hoch siedenden aliphatischen Aether, wie Diäthylenglycoldimethyläther und dergleichen, in eine Verbindung der Formel X übergeführt.

Eine erhaltene Verbindung der Formel X, worin $R^1$ Wasserstoff bedeutet, kann in bekannter Weise in eine Verbindung der Formel X übergeführt werden, worin $R^1$ Halogen, Nitro, $C_{2-7}$-Alkanoyl, Aroyl oder tertiär-nieder Alkyl bedeutet. Beispielsweise kann eine Verbindung der Formel X, worin $R^1$ Wasserstoff bedeutet, durch Umsetzen mit elementarem Chlor oder Brom, zweckmässigerweise in einem inerten organischen Lösungsmittel, wie einem halogenierten Kohlenwasserstoff, z.B. Dichlormethan, Chloroform, Tetrachlorkohlenstoff und dergleichen, und in Gegenwart einer Base, z.B. eines tertiären Amins, wie Triäthylamin oder Pyridin, chloriert oder bromiert werden. Wiederum beispielsweise kann eine Verbindung der Formel X, worin $R^1$ Wasserstoff bedeutet, durch Umsetzen mit Nitroniumtetrafluorborat, zweckmässigerweise in einem inerten oganischen Lösungsmittel, wie Acetonitril, nitriert werden. Wiederum beispielsweise kann eine Verbindung der Formel X, worin $R^1$ Wasserstoff bedeutet, durch Umsetzen mit einem $C_{2-7}$-Alkanoylhalogenid, wie Acetylchlorid, oder einem Aroylhalogenid, wie Benzoylchlorid, in Gegenwart eines Katalysators, wie Aluminiumchlorid und dergleichen, $C_{2-7}$-alkanoyliert oder aroyliert werden. Eine Verbindung der Formel X, worin $R^1$ Wasserstoff bedeutet, kann beispielsweise auch durch Umsetzen mit einem tertiär-Alkanoylhalogenid, z.B. Pivaloylchlorid, in Gegenwart eines Katalysators, wie Aluminiumchlorid und dergleichen, zu einer Verbindung der Formel X umgesetzt werden, worin $R^1$ tertiär-nieder Alkyl bedeutet. Eine Verbindung der Formel X, worin $R^1$ Cyan bedeutet, kann in bekannter Weise in eine entsprechende Verbindung der Formel X, worin $R^1$ Carboxy bedeutet, übergeführt werden. Diese Ueberführung erfolgt beispielsweise durch Erhitzen mit einer wässerigen Alkalimetallhydroxidlösung, wie wässeriger Natriumhydroxidlösung. Die erhaltene Verbindung der Formel X, worin $R^1$ Carboxy bedeutet, kann nach bekannten Methoden in eine entsprechende Verbindung der Formel X übergeführt werden, worin $R^1$ nieder Alkoxycarbonyl, Carbamoyl, Mono(nieder alkyl)carbamoyl oder Di(nieder alkyl)carbamoyl bedeutet. Dies erfolgt

beispielsweise durch Ueberführung in das entsprechende Säurehalogenid, z.B. -chlorid, unter Verwendung eines geeigneten Halogenierungsmittels, z.B. Thionylchlorid, und Umsetzen des Halogenides mit einem geeignetem nieder Alkanol oder Ammoniak, einem nieder alkylamin, z.B. Methylamin, Aethylamin und dergleichen, oder einem Di(nieder alkyl)amin, z.B. Dimethylamin, Diäthylamin und dergleichen.

Eine Verbindung der Formel X, worin $R^1$ Wasserstoff, Halogen, Trifluormethyl, Cyan, nieder Alkyl, nieder Alkylthio oder nieder Alkylsulfonyl bedeutet, kann in eine entsprechende Verbindung der Formel XI übergeführt werden, worin $R^1$ Wasserstoff, Halogen, Trifluormethyl, Cyan, nieder Alkyl, nieder Alkylthio oder nieder Alkylsulfonyl bedeutet, und zwar durch Hydrieren in bekannter Weise in Gegenwart eines Edelmetallkatalysators, z.B. eines Palladium- oder Platinkatalysators. Beispielsweise wird die Hydrierung in einem inerten organischen Lösungsmittel, wie einem nieder Alkanol, z.B. Methanol, Aethanol und dergleichen, bei ungefähr Raumtemperatur und unter Atmosphärendruck durchgeführt.

Eine Verbindung der Formel XI, worin $R^1$ Wasserstoff bedeutet, kann in eine entsprechende Verbindung der Formel XI übergeführt werden, worin $R^1$ Halogen, Nitro, $C_{2-7}$-Alkanoyl, Aroyl oder tertiär-nieder Alkyl bedeutet. Diese Ueberführung kann in analoger Weise wie weiter oben für die Ueberführung einer Verbindung der Formel X, worin $R^1$ Wasserstoff bedeutet, in eine Verbindung der Formel X, worin $R^1$ Halogen, Nitro, $C_{2-7}$-Alkanoyl, Aroyl oder tertiär-nieder Alkyl bedeutet, angegeben, durchgeführt werden. Im weiteren kann eine Verbindung der Formel XI, worin $R^1$ Cyan bedeutet, in eine Verbindung der Formel XI übergeführt werden, worin $R^1$ Carboxy bedeutet, und diese Verbindung kann wiederum in eine entsprechende Verbindung der Formel XI übergeführt werden, worin $R^1$ nieder Alkoxycarbonyl, Carbamoyl, Mono(nieder alkyl)carbamoyl oder Di(nieder alkyl)carbamoyl bedeutet, und zwar wiederum in ähnlicher Weise wie früher im Zusammenhang mit der entsprechenden Verbindung der Formel X beschrieben. Eine Verbindung der Formel XI, worin $R^1$ Carbamoyl bedeutet, kann in bekannter Weise, beispielsweise durch Dehydrierung mit Phosphoroxychlorid, in eine entsprechende Verbindung der Formel I übergeführt werden, worin $R^1$ Cyan bedeutet.

Gewisse Substituenten an der mit $R^{64}$ bezeichneten 2-Pyridylgruppe in den Verbindungen der Formeln X und XI können funktionell in andere Substituenten umgewandelt werden. Beispielsweise kann eine Benzyloxycarbonyl-substituierte 2-Pyridylgruppe zur entsprechenden Carboxy-substituierten 2-Pyridylgruppe debenzyliert werden, beispielsweise durch Erhitzen mit Palladium auf Kohle und Ameisensäure, und die erhaltene Verbindung kann mit einem Diazoalkan zu einer nieder Alkoxycarbonyl-substituierten 2-Pyridylgruppe verestert werden. Im weiteren kann eine Nitro-substituierte 2-Pyridylgruppe in einer Verbindung der Formel X mit einem Reduktionssystem, wie Eisenpulver/Essigsäure, zur Amino-substituierten 2-Pyridylgruppe reduziet werden, wobei die Doppelbindung in der 3,4-Stellung des Moleküls nicht angegriffen wird. Wenn man eine Verbindung der Formel X, worin $R^{64}$ eine Nitro-substituierte 2-Pyridylgruppe bedeutet, katalytisch hydriert, wird selbstverständlich nicht nur die Nitro-substituierte 2-Pyridylgruppe zur entsprechenden Amino-substituierten 2-Pyridylgruppe reduziert, sondern auch die Doppelbindung in der 3,4-Stellung des Moleküls zu einer Einfachbindung. Andere funktionelle Modifikationen, welche durchgeführt werden können, umfassen auch die Umwandlung einer Amino-substituierten 2-Pyridylgruppe in eine Hydroxy-substituierte oder Chlor-substituierte 2-Pyridylgruppe, die Umwandlung einer Amino-substituierten 2-Pyridylgruppe in eine Jod-substituierte 2-Pyridylgruppe sowie die Umwandlung der letztgenannten Verbindung in eine nieder Alkylphenyl-substituierte 2-Pyridylgruppe, wobei alle diese funtionellen Abwandlungen nach an sich bekannten Methoden durchgeführt werden können.

Zur Herstellung eines Ausgangsmaterials der Formel IV wird eine Verbindung der Formel X oder XI, worin $R^{64}$ 3-Pyridyl oder 4-Methyl-3-pyridyl bedeutet, oxidiert. Diese Oxidation kann nach bekannten Methoden durchgeführt werden. Beispielsweise wird eine Verbindung der Formel X oder XI durch Umsetzen mit Wasserstoffperoxid, einer organischen Persäure, wie Peressigsäure, Perbenzoesäure, m-Chlorperbenzoesäure, Perphtalsäure und dergleichen, einem Perester, Natriummetaperjodat, Natriumperborat in Essigsäure und dergleichen, oxidiert. Die Oxidation wird zweckmässigerweise in einem inerten organischen Lösungsmittel, wie einem halogenierten Kohlenwasserstoff, z.B. Chloroform, Dichlormethan, Dichloräthan und dergleichen, durchgeführt. Wenn Wasserstoffperoxid als Oxidationsmittel verwendet wird, kann die Oxidation auch in Essigsäure und dergleichen durchgeführt werden. Zweckmässigerweise erfolgt die Oxidation bei einer Temperatur zwischen ungefähr 0 °C und ungefähr 30 °C, vorzugsweise bei ungefähr Raumtemperatur.

Ein weiteres Verfahren zur Herstellung der Ausgangsstoffe der Formel III, worin $R^4$ und $R^5$ je Wasserstoff bedeuten und $R^{61}$ eine 2-Pyridylgruppe mit einem Chloratom in der o- oder p-Stellung zum Stickstoffatom bedeutet, besteht darin, dass man eine Verbindung der Formel I, worin $R^4$ und $R^5$ je Wasserstoff und $R^6$ 2-Pyridyl-1-oxid bedeuten, bei erhöhter Temperatur beispielsweise ungefähr 80 °C, mit Phosphoroxychlorid umsetzt. Diese Umsetzung liefert ein Gemisch der oben erwähnten o-Chlor- und p-Chlorverbindungen, wobei dieses Gemisch chromatographisch in die einzelnen Verbindungen aufgetrennt

werden kann.

Noch eine andere Methode zur Herstellung der Ausgangsstoffe der Formel III, worin $R^4$ und $R^5$ zusammen eine Kohlenstoff-Kohlenstoff-Bindung bedeuten, umfasst die Umsetzung einer Verbindung der Formel I, worin $R^4$ und $R^5$ je Wasserstoff und $R^6$ 2-Pyridyl-1-oxid, welches gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet, mit einem nieder Alkansäureanhydrid, vorzugsweise Essigsäureanhydrid, bei erhöhter Temperatur, beispielsweise ungefähr 120 ° C.

Die Ausgangsstoffe der Formeln II, III und IV, worin $R^4$ Hydroxy und $R^5$ Wasserstoff bedeuten, können hergestellt werden, indem man zunächst eine Verbindung der Formel II, III oder IV, worin $R^4$ und $R^5$ zusammen eine Kohlenstoff-Kohlenstoff-Bindung bedeuten, in bekannter Weise in das entsprechende 3,4-Epoxid überführt. Beispielsweise kann diese Epoxidation in einem inerten organischen Lösungsmittel oder Lösungsmittelgemisch, wie einem nieder Alkanol, z.B. Methanol, Acetonitril oder einem Gemisch davon mit Wasserstoffperoxid in Gegenwart eines Alkalimetallwolframats, z.B. Natriumwolframat, bei erhöhter Temperatur, z.B. ungefähr 50 ° C, erfolgen. Danach wird das 3,4-Epoxid durch katalytische Hydrierung in bekannter Weise in das erwünschte Ausgangsmaterial, worin $R^4$ Hydroxy und $R^5$ Wasserstoff bedeuten, umgewandelt, beispielsweise in einem inerten organischen Lösungsmittel, wie einem nieder Alkanol, z.B. Aethanol, und in Gegenwart eines Edelmetallkatalysators, wie einem Platin- oder Palladiumkatalysator, welcher auf einem geeigneten Trägermaterial aufgetragen sein kann, z.B. Palladium auf Kohle. Zweckmässigerweise wird die katalytische Hydrierung bei ungefähr Raumtemperatur und unter Atmosphärendruck durchgeführt.

Wie im Falle der Verbindungen der Formel I können die Ausgangsmaterialien der Formeln II, III und IV in Abhängigkeit von der Bedeutung der Substituenten $R^2$ und $R^3$ und $R^4$ und $R^5$ als optische Isomere, Racemate, Diastereomere und cis/trans-Isomere vorliegen. Die Auftrennung eines Gemisches diastereomerer Racemate, die Auftrennung eines Racemates sowie die Tennung eines cis/trans-Gemisches kann in der Weise durchgeführt werden, wie sie weiter oben im Zusammenhang mit den Verbindungen der Formel I beschrieben wurden. Ein spezielles Verfahren zur Auftrennung einer racemischen Verbindung der Formel XI, worin $R^1$ Carboxy bedeutet, besteht darin, dass man eine solche Verbindung mit einer geeigneten chiralen Base, wie Chinin, umsetzt, die optisch aktiven Salze durch fraktionierte Kristallisation auftrennt und die optisch aktiven Verbindungen durch Umsetzen mit einer geeigneten Säure freisetzt.

Die Ausgangsstoffe der Formel V sind neu und ebenfalls Gegenstand der vorliegenden Erfindung. Sie können gemäss Reaktionsschema II hergestellt werden, in welchem $R^1$, $R^2$, $R^3$ und $R^{63}$ die oben angegebene Bedeutung besitzen, $R^8$ nieder Alkyl und $R^9$ nieder Alkylsulfonyl oder Arylsulfonyl bedeuten.

Reaktionsschema II

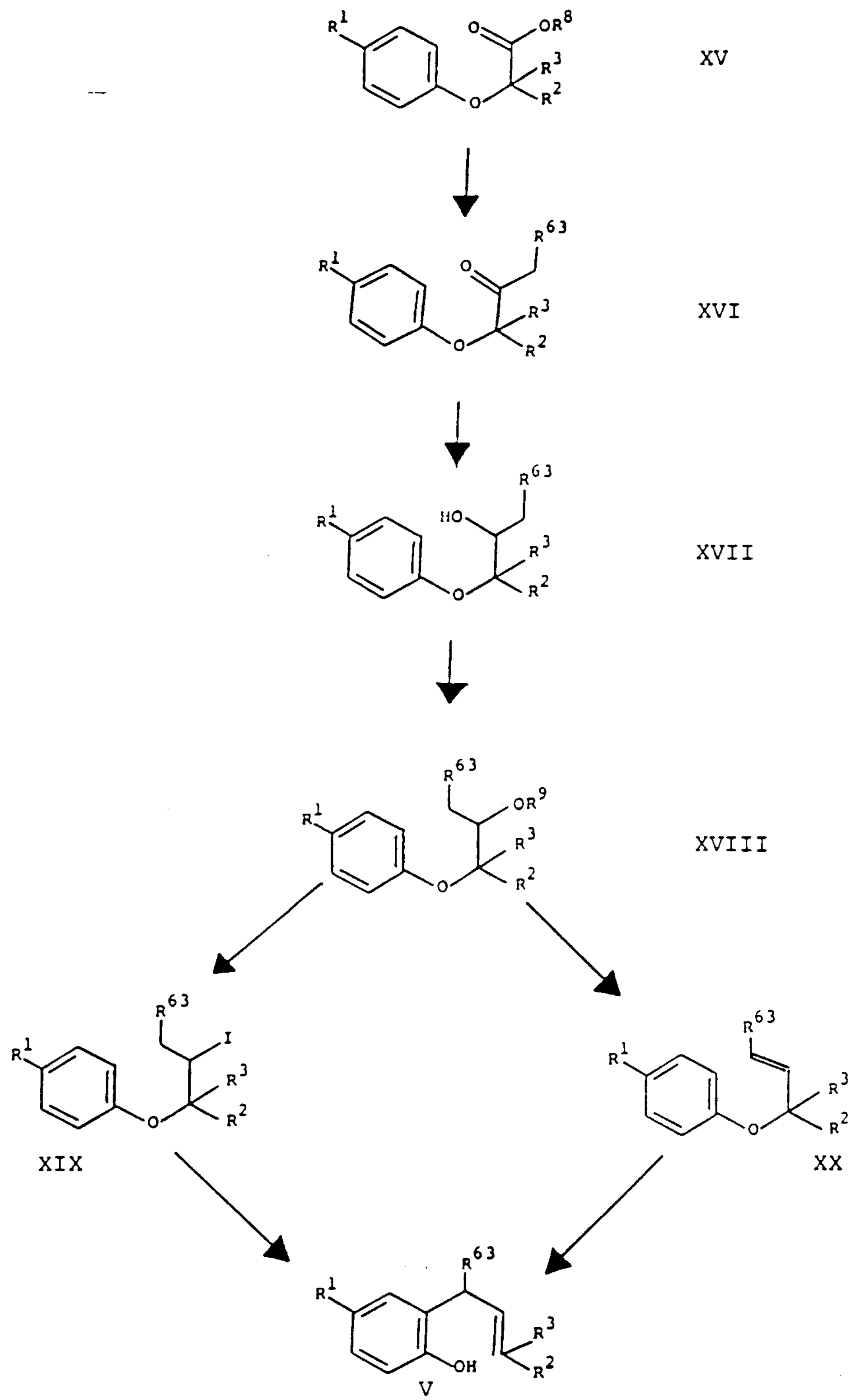

XV

XVI

XVII

XVIII

XIX

XX

V

Gemäss Reaktionsschema II wird eine Verbindung der Formel XV, bei der es sich um eine bekannte Verbindung oder ein Analogon einer bekannten Verbindung handelt, durch Umsetzen mit 2-Pyridyl-1-oxid, welches gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid mit einer Methylgruppe in 2-Stellung, z.B. 2-Picolin-N-oxid, in

Gegenwart einer starken Base, vorzugsweise eines Alkalimetallhydrids, wie Natriumhydrid, oder eines Alkalimetalldi(nieder alkyl)amids, wie Lithiumdiäthylamid, in eine Verbindung der Formel XVI übergeführt. Zweckmässigerweise führt man die Reaktion in einem inerten organischen Lösungsmittel, wie einem cyclischen Aether, z.B. Tetrahydrofuran, bei der Rückflusstemperatur des Reaktionsgemisches oder, falls ein Di(nieder alkyl)amid als starke Base verwendet wird, bei einer Temperatur zwischen ungefähr -78°C und ungefähr Raumtemperatur aus.

Eine Verbindung der Formel XVI wird danach in bekannter Weise zu einer Verbindung der Formel XVII reduziert. Z.B. führt man die Reduktion zweckmässigerweise unter Verwendung eines komplexen Metallhydrids, wie eines Alkalimetallborhydrids, z.B. Natriumborhydrid, in einem geeigneten inerten organischen Lösungsmittel, z.B. einem Alkohol, wie Aethanol, bei ungefähr Raumtemperatur durch.

Die nachfolgende Ueberführung einer Verbindung der Formel XVII in eine Verbindung der Formel XVIII erfolgt nach bekannten Methoden, beispielsweise durch Umsetzen mit einem nieder Alkansulfonylhalogenid, z.B. Methansulfonylchlorid und dergleichen, oder einem aromatischen Sulfonylhalogenid, z.B. Benzolsulfonylchlorid, p-Toluolsulfonylchlorid und dergleichen, in einem inerten organischen Lösungsmittel und in Gegenwart eines säurebindenden Mittels, wie einem tertiären Amin, z.B. Triäthylamin, Pyridin und dergleichen. Man kann das Amin im Ueberschuss verwenden, wobei es dann auch gleichzeitig als Lösungsmittel dienen kann. Die Umsetzung wird zweckmässigerweise bei ungefähr Raumtemperatur durchgeführt.

Im nächsten Schritt wird die Gruppe $OR^9$ in einer Verbindung der Formel XVIII in bekannter Weise durch ein Jodatom unter Bildung einer Verbindung der Formel XIX ersetzt. Dieser Schritt kann beispielsweise durch Umsetzen mit einem Alkalimetalljodid, wie Natriumjodid, in Aceton bei erhöhter Temperatur, zweckmässigerweise bei der Rückflusstemperatur des Reaktionsgemisches, erfolgen.

Das Jodatom in einer Verbindung der Formel XIX wird danach abgespalten, und das erhaltene Produkt einer Claisen-Umlagerung unterworfen, wobei man das erwünschte Ausgangsmaterial der Formel V erhält. Die Abspaltung und die Umlagerung werden durch Erhitzen einer Verbindung der Formel XIX mit einem geeigneten tertiären Amin, z.B. Diisopropyläthylamin und dergleichen, zweckmässigerweise bei der Rückflusstemperatur, in einem inerten organischen Lösungsmittel, wie einem nieder Alkanol, z.B. Methanol, Aethanol und dergleichen, in einem Schritt durchgeführt.

Eine alternative Methode ausgehend von einer Verbindung der Formel XVIII betrifft die Abspaltung der Gruppe $OR^9$ durch Umsetzen mit einer starken Base, wie einem Alkalimetallhydrid, z.B. Natriumhydrid, zweckmässigerweise in einem organischen Lösungsmittel, wie einem nieder Alkanol, z.B. Isopropanol, und bei ungefähr Raumtemperatur, wobei man eine Verbindung der Formel XX erhält.

Die Verbindung der Formel XX wird danach durch eine Claisen-Umlagerung in das erwünschte Ausgangsmaterial der Formel V übergeführt. Dieser Schritt erfolgt zweckmässigerweise durch erhitzen in einem hoch siedenden organischen Lösungsmittel, wie einem halogenierten aromatischen Kohlenwasserstoff, z.B. Chlorbenzol, 1,2-Dichlorbenzol und dergleichen.

Die in Verfahrensvariante i) als Ausgangsstoffe eingesetzten Verbindungen, nämlich die 3-[N,N-Di(nieder alkyl)carbamoyloxy]pyridin sowie die Verbindungen der Formel VI sind bekannte Verbindungen oder Analoga der bekannten Verbindungen und können in ähnlicher Weise wie die bekannten Verbindungen hergestellt werden.

Die Verbindungen der Formel I und deren vorerwähnte Salze besitzen eine ausgeprägte den Kaliumkanal aktivierende Wirkung und können als Medikamente verwendet werden, insbesondere bei der Bekämpfung bzw. Verhütung von Bluthochdruck, dekompensierter Herzinsuffizienz, Angina pectoris, peripheren und cerebralen Gefässerkrankungen oder Störungen der glatten Muskulatur, z.B. des Gastroinestinal-, Atmungs- und Urogenital-trakts, wie bei Magenulcus, Bowel-syndrom, Diverticulitis, reversibler Atmungseinschränkung oder Störungen der Geburtswehen. Im weiteren können sie auch bei einer Behandlung von Haarausfall verwendet werden.

Die den Kaliumkanal aktivierende Wirkung der erfindungsgemässen Verbindungen kann im folgenden Test gezeigt werden:

Die Portalvenen der Leber werden männlichen Sprague-Dawley Ratten entfernt und in Organbäder verbracht, und zwar unter einer Anfangsspannung von 0,5 g für die isometrische Druckaufzeichnung. Die Venen werden in Krebslösung inkubiert, welche aus 118 mM Natriumchlorid, 25 mM Natriumbicarbonat, 10,5 mM D-Glucose, 4,7 mM Kaliumchlorid, 0,4 mM Magnesiumsulfat, 1,2 mM Kaliumdihydrogenphosphat und 2,5 mM Calciumchlorid besteht und welche mit 95% Sauerstoff und 5% Kohlendioxid begast und bei 37° C gehalten wird. Nach einem halb- oder einstündigen Inkubieren werden weitere 20 mM Kaliumchlorid dazugegeben und eine weitere viertel bis halbe Stunde später steigende Konzentrationen der Testverbindung. Die Aktivität der Testverbindung wird als $IC_{50}$-Wert ausgedrückt, welcher diejenige Konzentration der Testverbindung darstellt, welche eine halb maximale Reduktion der durch das Kaliumchlorid hervorgerufenen Kontraktion bewirkt.

Tabelle

| Verbindung | $IC_{50}$ (µmol) |
|---|---|
| A | 0.16 ± 0.02 |
| B | 0.038 ± 0.002 |
| C | 0.014 ± 0.001 |
| D | 0.56 ± 0.07 |
| E | 0.015 ± 0.0005 |
| F | 0.28 ± 0.03 |
| G | 0.019 ± 0.0012 |
| H | 3.1 ± 0.8 |

Verbindung A : 2-(6-Cyan-3,4-dihydro-2,2-dimethyl-2H-1-benzopyran-4-yl)pyridin-N-oxid.
Verbindung B : 2-(6-Cyan-2,2-dimethyl-2H-1-benzopyran-4-yl)pyridin-N-oxid.
Verbindung C : 2-(3,4-Dihydro-2,2-dimethyl-6-nitro-2H-1-benzopyran-4-yl)pyridin-N-oxid.
Verbindung D : 2-(6-Acetyl-3,4-dihydro-2,2-dimethyl-2H-1-benzopyran-4-yl)pyridin-N-oxid.
Verbindung E : 2-(6-Cyan-2,2-dimethyl-2H-1-benzopyran-4-yl)-5-phenylpyridin-N-oxid.
Verbindung F : rac-trans-2-(6-Cyan-3,4-dihydro-3-hydroxy-2,2-dimethyl-2H-1-benzopyran-4--yl)pyridin-N-oxid.
Verbindung G : 2-(6-Cyan-2-äthyl-2-methyl-2H-1-benzopyran-4-yl)pyridin-N-oxid.
Verbindung H : 4-(5-Cyan-2-hydroxyphenyl)-2,2-dimethyl-2H-1-benzopyran-6-carbonitril.

Die Verbindungen der Formel I und deren vorerwähnte Salze können als Arzneimittel verwendet werden, beispielsweise in Form pharmazeutischer Präparate. Die pharmazeutischen Präparate können oral verabreicht werden, z.B. in Form von Tabletten, Lacktabletten, Dragées, Hart-und Weichgelatinekapseln, Lösungen, Emulsionen oder Suspensionen. Sie können jedoch auch rektal, z.B. in Form von Suppositorien, oder parenteral, z.B. in Form von Injektionslösungen, verabreicht werden.

Zur Herstellung der pharmazeutischen Präparate können die Verbindungen der Formel I und deren vorerwähnte Salze mit pharmazeutisch inerten anorganischen oder organischen Trägerstoffen verarbeitet werden. Geeignete Trägerstoffe, welche für Tabletten, Lacktabletten, Dragées und Hartgelatinekapseln verwendet werden können, sind beispielsweise Lactose. Maisstärke oder Derivate davon, Talk, Stearinsäure oder deren Salze und dergleichen. Geeignete Trägerstoffe für Weichgelatinekapseln sind beispielsweise pflanzliche Oele, Wachse, Fette, halbfeste und flüssige Polyole und dergleichen. Wasser, Polyole, Saccharose, Invertzucker, Glucose und dergleichen sind Beispiele geeigneter Trägerstoffe für die Herstellung von Lösungen und Sirupen. Geeignete Trägerstoffe für Injektionslösungen sind beispielsweise Wasser, Alkohole, Polyole, Glycerin, pflanzliche Oele und dergleichen. Natürliche und gehärtete Oele, Wachse, Fette, halbflüssige oder flüssige Polyole und dergleichen sind Beispiele geeigneter Trägerstoffe für Suppositorien.

Die pharmazeutischen Präparate können auch noch Konservierungsmittel, Lösungsvermittler, Stabilisierungsmittel, Netzmittel, Emulgiermittel, Süssstoffe, Farbstoffe, Aromastoffe, Salze zum Variieren des osmotischen Druckes, Puffer, Ueberzugstoffe oder Antioxidantien enthalten. Die pharmazeutischen Präparate können auch noch andere therapeutisch wertvolle Verbindungen enthalten.

Die erfindungsgemässen Verbindungen der Formel I und deren vorerwähnte Salze können bei der Bekämpfung bzw. Verhütung von Krankheiten, insbesondere bei der Bekämpfung bzw. Verhütung von Bluthochdruck, dekompensierter Herzisuffizienz, Angina pectoris, peripheren und cerebralen Gefässerkrankungen und Störungen der glatten Muskulatur verwendet werden. Die Dosierung der Verbindungen der Formel I und deren vorerwähnten Salze können innerhalb weiter Grenzen variieren und werden selbstverständlich in Abhängigkeit des individuellen Bedürfnisses in jedem einzelnen Fall festgesetzt. Im allgemeinen dürfte bei oraler Verabreichung an Erwachsene eine tägliche Dosis von ungefähr 0,1 mg bis ungefähr 10 mg, vorzugsweise ungefär 0,2 mg bis ungefähr 5 mg,angezeigt sein, obwohl die erwähnte obere Grenze

überschritten werden kann, wenn dies angezeigt ist. Die tägliche Dosis kann in einer einzigen Dosis oder in Teildosen verabreicht werden.

Die folgenden Beispiele veranschaulichen die vorliegende Erfindung:

Beispiel 1

130 mg 3,4-Dihydro-2,2-dimethyl-4-(2-pyridyl)-2H-1-benzopyran werden in 10 ml Dichlormethan bei Raumtemperatur gelöst und mit 93 mg m-Chlorperbenzoesäure versetzt. Ein nach 2 Stunden durchgeführtes Dünnschichtchromatogramm zeigt an, dass noch einiges Ausgangsmaterial vorhanden ist, worauf weitere m-Chlorperbenzoesäure bis zur vollständigen Reaktion zugegeben wird. Das Reaktionsgemisch wird nacheinander mit Natriumbisulfitlösung, Natriumbicarbonatlösung und Wasser gewaschen, über Natriumsulfat getrocknet und eingedampft, wobei man 105 mg 2-(3,4-Dihydro-2,2-dimethyl-2H-1-benzopyran-4-yl)-pyridin-N-oxid in Form eines Oels erhält. NMR (300 MHz, $CDCl_3$): δ 8.36-8.30 (1H, m), 7,21-7.04 (4H, m), 6,89-6,76 (3H, m), 5,36-5,25 (1H, m), 2,46 (1H, dd, 14Hz, 6.5Hz), 1,75 (1H, breit, t, 14Hz), 1,44 (3H, s), 1,40 (3H, s). MS (EI): 255 $M^+$), 238 ($M^+$-OH).

Das als Ausgangsmaterial eingesetzte 3,4-Dihydro-2,2-dimethyl-4-(2-pyridyl)-2H-1-benzopyran wurde wie folgt hergestellt

(A) 43,84 g 1-Brom-2-methylprop-1-en in 150 ml Tetrahydrofuran werden unter Erhitzen zum Rückfluss tropfenweise zu 9,8 g Magnesium in 50 ml Tetrahydrofuran gegeben. Nach einer Stunde lässt man das Reaktionsgemisch auf Raumtemperatur abkühlen und gibt dann 47,7 g 2-Methoxyphenyl-2-pyridylketon in 200 ml Tetrahydrofuran langsam zu. Nach 2-stündigem Rühren bei Raumtemperatur werden 200 ml gesättigte Ammoniumchloridlösung zugegeben, und das Gemisch mit Aethylacetat extrahiert. Der organische Extrakt wird über Natriumsulfat getrocknet und eingedampft. Der Rückstand wird an Kieselgel chromatographiert, wobei zunächst mit einem 1:6- und danach mit einem 1:4- Gemisch von Aethylacetat und Petroläther eluiert wird. Dabei erhält man 36.6 g 1-(2-Methoxyphenyl)-3-methyl-1-(2-pyridyl)-2-buten-1-ol in Form eines Oels.

(B) 36.6 g 1-(2-Methoxyphenyl)-3-methyl-1-(2-pyridyl)-2-buten-1-ol werden in 200 ml Dimethylformamid mit 28.6 g Natriummethanthiolat auf 70 °C erhitzt. Nach 10 Stunden lässt man das Gemisch auf Raumtemperatur abkühlen, giesst es auf verdünnte Salzsäure und extrahiert mit Aethylacetat. Der organische Extrakt wird über Natriumsulfat getrocknet und eingedampft. Chromatographie des Rückstands an Kieselgel unter Verwendung von Aethylacetat als Eluierungsmittel liefert 28.5 g 1-(2-Hydroxyphenyl)-3-methyl-1-(2-pyridyl)-2-buten-1-ol in form eines Oels.

(C) 4.42 g 1-(2-Hydroxyphenyl)-3-methyl-1-(2-pyridyl)-2-buten-1-ol werden in 70 ml Diäthylenglycoldimethyläther gelöst und für 2 Stunden auf 150 °C erhitzt. Das Reaktionsgemisch wird danach auf Raumtemperatur abgekühlt, das Lösungsmittel unter vermindertem Druck entfernt, und der Rückstand zwischen Aethylacetat und Natriumchloridlösung verteilt. Die organische Phase wird über Natriumsulfat getrocknet und eingedampft. Chromatographie des Rückstands an Kieselgel mit Aethylacetat/Petroläther (1:4) als Eluierungsmittel liefert 2.4 g 2,2-Dimethyl-4-(2-pyridyl)-2H-1-benzopyran, Schmelzpunkt 80-82 °C.

(D) 6.95 g 2,2-Dimethyl-4-(2-pyridyl)-2H-1-benzopyran werden in 100 ml Aethanol gelöst und bei Raumtemperatur mit 10% Palladium auf Kohle unter einer Wasserstoffatmosphäre geschüttelt. Nachdem das erforderliche Volumen Wasserstoff aufgenommen worden ist, wird der Katalysator abfiltriert, das Filtrat eingedampft, und der Rückstand aus n-Hexan umkristallisiert, wobei man 5.07 g 3,4-Dihydro-2,2-dimethyl-4-(2-pyridyl)-2H-1-benzopyran erhält, Schmelzpunkt 99-101 °C.

Beispiel 2

245 mg 3,4-Dihydro-2,2-dimethyl-6-nitro-4-(2-pyridyl)-2H-1-benzopyran werden bei Raumtemperatur in 5 ml Dichloromethan gelöst, und 149 mg m-Chlorperbenzoesäure werden zugegeben. Nach 3-tägigem Rühren bei Raumtemperatur wird solange weiter m-Chlorperbenzoesäure zugegeben, bis kein Ausgangsmaterial mehr im Dünnschichtchromatogramm festgestellt werden kann. Das Reaktionsgemisch wird nacheinander mit Natriumbisulfitlösung, Natriumbicarbonatlösung und Natriumchloridlösung gewaschen, über Natrimsulfat getrocknet und eingedampft. Der Rückstand wird unter Verwendung von 4% (V/V) Aethanol/Dichloromethan als Eluierungsmittel an Kieselgel chromatographiert, und der erhaltene Schaum mit n-Hexan angerieben, wobei man 80 mg 2-(3,4-Dihydro-2,2-dimethyl-6-nitro-2H-1-benzopyran-4-yl)-pyridin-N-oxid erhält, Schmelzpunkt 116-119 °C.

Das als Ausgangsmaterial eingesetzte 3,4-Dihydro-2,2-dimethyl-6-nitro-4-(2-pyridyl)-2H-1-benzopyran wurde wie folgt hergestellt:

1.32 g 3,4-Dihydro-2,2-dimethyl-4-(2-pyridy)-2H-1-benzopyran werden in 20 ml Acetonitril gelöst, und 0.73 g Nitroniumtetrafluorborat werden bei Raumtemperatur zugegeben. Nach 1 Stunde wird das Gemisch auf Wasser gegossen und mit Aethylacetat extrahiert. Der organische Extrakt wird mit Natriumchloridlösung gewaschen, über Natriumsulfat getrocknet und zu einem Oel eingedampft. Chromatographie dieses Oels an Kieselgel mit Aethylacetat/Petroläther (1:4) als Eluierungsmittel liefert 245 mg 3,4-Dihydro-2,2-dimethyl-6-nitro -4-(2-pyridyl)-2H-1-benzopyran in Form eines Oels.

Beispiel 3

Man löst 261 mg 6-Acetyl-3,4-dihydro-2,2-dimethyl-4-(2-pyridyl)-2H-1-benzopyran in 20 ml Dichlormethan bei Raumtemperatur und gibt 250 mg m-Chlorperbenzoesäure zu. Nach 3-tägigem Rühren bei Raumtemperatur wird das Gemisch nacheinander mit Natriumbisulfitlösung, Natriumbicarbonatlösung und Wasser gewaschen über Natriumsulfat getrocknet und eingedampft. Der Rückstand wird an Kieselgel mit 2%-5% (V/V) Methanol/Chloroform als Eluierungsmittel chromatographiert. Der dabei erhaltene Schaum wird mit Methylcyclohexan angerieben, und der erhaltene Festkörper aus t-Butylmethyläther umkristallisiert, wobei man 20 mg 2-(6-Acetyl-3,4-dihydro-2,2-dimethyl-2H-1-benzopyran-4-yl)pyridin-N-oxid erhält, Schmelzpunkt 132-133 ° C.

Das als Ausgangsmaterial eingesetzte 6-Acetyl-3,4-dihydro-2,2-dimethyl-4-(2-pyridyl)-2H-1-benzopyranwurde wie folgt hergestellt:

1.56 g 3,4-dihydro-2,2-dimethyl-2H-1-benzopyran-4-yl)pyridin werden bei Raumtemperatur in 20 ml Nitromethan gelöst. 1.67 g Aluminiumchlorid und 1.11 g Acetylchlorid werden zugegeben, und das Gemisch 1 Stunde bei 50 ° C gerührt. Verdünnte Natriumhydroxidlösung wird zugegeben, und das Gemisch mit Aethylacetat extrahiet. Der organische Extrakt wird mit Natriumchloridlösung gewaschen, über Natriumsulfat getrocknet und eingedampft. Der Rückstand wird an Kieselgel chromatographiert, wobei man zunächst ein 1:2-Gemisch und nachher ein 1:1-Gemisch von Aethylacetat und Petroläther als Eluierungsmittel verwendet. Anreiben des Rückstandes mit n-Hexan liefert einen Festkörper, welcher nach Umkristallisation aus t-Butylmethyläther 261 mg 6-Acetyl-3,4-dihydro-2,2-dimethyl-4-(2-pyridyl)-2H-1-benzopyran liefert, Schmelzpunkt 102-105 ° C.

Beispiel 4

101 mg 6-Acetyl-3,4-dihydro-2,2-dimethyl-4-(3-methyl-2-pyridyl)-2H-1-benzopyran werden bei Raumtemperatur in 5 ml Dichlormethan gelöst, und 91 mg m-Chlorperbenzoesäure werden zugegeben. Nach 1 Stunde wird das Gemisch nacheinander mit Natriumbisulfitlösung, Natriumbicarbonatlösung und Wasser gewaschen. Der organische Extrakt wird über Natriumsulfat getrocknet und zu einem Oel eingedampft. Dieses Oel wird mit t-Butylmethyläther angerieben, wobei ein Festkörper anfällt, welcher nach Umkristallisation aus Aethylacetat/t-Butylmethyläther, 28 mg 2-(6-Acetyl-3,4-dihydro-2,2-dimethyl-2H-1-benzopyran-4-yl)-3-methylpyridin-N-oxid liefert, Schmelzpunkt 154-156 ° C.

Das als Ausgangsmaterial eingesetzte 6-Acetyl-3,4-dihydro-2,2-dimethyl-4-(3-methyl-2-pyridyl)-2H-1-benzopyran wurde wie folgt hergestellt:

(A) 35.5 g o-Bromanisol werden tropfenweise zu 5.71 g Magnesiumspänen, welche mit 50 ml Tetrahydrofuran zugedeckt sind, gegeben, während man so erwärmt, dass der Rückfluss des Lösungsmittels aufrechterhalten bleibt. 15 Minuten nach der Beendigung der Zugabe werden 14.93 g 2-Cyan-3-methylpyridin in 150 ml Tetrahydrofuran tropfenweise zugegeben, wobei man nicht mehr weiter erwärmt. Nach 1 Stunde bei Raumtemperatur gibt man verdünnte Salzsäure und Aethylacetat sowie danach verdünnte Natriumhydroxidlösung bis zum pH 14 zu. Das Reaktionsgemisch wird dann mit Aethylacetat extrahiert, der organische Extrakt über Natriumsulfat getrocknet und eingedampft. Der Rückstand wird an Kieselgel chromatographiert, wobei man zunächst ein 1:3- und dann ein 1:2-Gemisch von Aethylacetat und Petroläther als Eluierungsmittel verwendet. Auf diese Weise erhält man 9.81 g 2-Methoxyphenyl-3-methyl-2-pyridylketon in Form eines Festkörpers, Schmelzpunkt 98-100 ° C.

(B) 9.23 g 1-Brom-2-methylprop-1-en werden unter Erwärmen tropfenweise zu 2.5 g Magnesiumspänen gegeben, welche mit Tetrahydrofuran zugedeckt sind. 7.76 g 2-Methoxyphenyl-3-methyl-2-pyridylketon werden unter Erwärmen zum Rückfluss zugegeben. Nach 4 Stunden lässt man das Reaktionsgemisch auf Raumtemperatur abkühlen. Gesättigte Ammoniumchloridlösung wird zugegeben, und das erhaltene Reaktionsgemisch mit Diäthyläther extrahiert. Der organische Extrakt wird über Natriumsulfat getrocknet und eingedampft. Der Rückstand wird an Kieselgel mit einem 1:4-Gemisch von Aethylacetat und Petroläther als Eluierungsmittel chromatographiert. Dabei erhält man 5.75 g 1-(2-Methoxyphenyl)-3-methyl-1-(3-methyl-2-pyridyl)-2-buten-1-ol in Form eines Oels.

(C) 7.13 g 1-(2-Methoxyphenyl)-3-methyl-1-(3-methyl-2-pyridyl)-2-buten-1-ol werden bei Raumtemperatur in 100 ml Dimethylformamid gelöst, und 5.29 g Natriummethanthiolat werden zugegeben. Nach 2-stündigem Rühren bei 120°C lässt man das Reaktionsgemisch auf Raumtemperatur abkühlen und dampft danach ein. Dann gibt man Wasser und Aethylacetat zu, trennt die organische Phase ab, trocknet sie über Natriumsulfat und dampft sie ein. Chromatographie des Rückstands an Kieselgel mit einem 1:4-Gemisch von Aethylacetat und Petroläther als Eluierungsmittel liefert 4.53 g 1-(2-Hydroxyphenyl)-4-methyl-1-(3-methyl-2-pyridyl)-2-buten-1-ol in Form eines Oels.

(D) 4.5 g 1-(2-Hydroxyphenyl)-3-methyl-1-(3-methyl-2-pyridyl)-2-buten-1-ol werden in 50 ml Diäthylenglycoldimethyläther gelöst und 1 Stunde zum Rückfluss erhitzt. Das Reaktionsgemisch wird auf Raumtemperatur abgekühlt und danach eingedampft. Der Rückstand wird an Kieselgel chromatographiert, wobei man zunächst mit einem 1:4- und dann mit einem 1:2-Gemisch von Aethylacetat und Petrolether eluiert. Dabei erhält man 2.97 g 2.2-Dimethyl-4-(3-methyl-2-pyridyl)-2H-1-benzopyran als Oel.

(E) 2.97 g 2,2-Dimethyl-4-(3-methyl-2-pyridyl)-2H-1-benzopyran werden in 50 ml Aethanol gelöst und bei Raumtemperatur unter einer Wasserstoffatmosphäre mit 10% Palladium auf Kohle in Gengenwart von 0.5 ml Essigsäure geschüttelt. Nach 24 Stunden wird der Katalysator abfiltriert, und das Filtrat eingedampft. Der Rückstand wird an Kieselgel mit einem 1:4-Gemisch von Aethylacetat und Petroläther als Eluierungsmittel chromatogrphiert, Dabei erhält man 1.4 g 3,4-Dihydro-2,2-dimethyl-4-(3-methyl-2-pyridyl)-2H-1-benzopyran sowie gemischte Fraktionen, enthaltend die genannte Verbindung und Ausgangsmaterial. Das oben beschriebene Verfahren wird mit den gemischten Fraktionen durchgeführt, wobei man 675 mg 3,4-Dihydro-2,2-dimethyl-4-(3-methyl-2-pyridyl)-2H-1-benzopyran erhält, welches aus t-Butylmethyläther umkristallisiert wird und dann bei 100-102°C. schmilzt.

(F) 712 mg 3,4-Dihydro-2,2-dimethyl-4-(3-methyl-2-pyridyl)-2H-1-benzopyran werden bei Raumtemperatur in 10 ml Nitromethan suspendiert. 750 mg Aluminiumchlorid und danach 500 mg Acetylchlorid werden zugegeben. Nach 1 Stunde bei Raumtemperatur gibt man verdünnte Natriumhydroxidlösung zu und extrahiert das Reaktionsgemisch mit Aethylacetat. Der organische Extrakt wird über Natriumsulfat getrocknet und eingedampft. Chromatographie des Rückstands an Kieselgel mit zunächst einem 1:3- und dann einem 1:1-Gemisch von Aethylacetat und Petroläther als Eluierungsmittel liefert ein Oel, welches mit n-Hexan angerieben wird. Der dabei anfallende Festkörper liefert nach Umkristallisation aus Cyclohexan 165 mg 6-Acetyl-3,4-dihydro-2,2-dimethyl-4-(3-methyl-2-pyridyl)-2H-1-benzopyran, Schmelzpunkt 87-88°C.

Beispiel 5

248 mg 3,4-Dihydro-2,2-dimethyl-4-(3-methyl-2-pyridyl)-6-nitro-2H-1-benzopyran werden bei Raumtemperatur in 20 ml Dichlormethan gelöst und mit 222 mg m-Chlorperbenzoesäure versetzt. Nach dem Rühren bei Raumtemperatur über Nacht wird die Lösung nacheinander mit Natriumbisulfitlösung Natriumbicarbonatlösung und Wasser gewaschen. Die organische Phase wird über Natriumsulfat getrocknet und eingedampft. Anreiben des erhaltenen Festkörpers mit Diäthyläther, Abfiltrieren und Umkristallisieren aus Acetonitril liefert 133 mg 2-(3,4-Dihydro-2,2-dimethyl-6-nitro-2H-1-benzopyran-4-yl)-3-methylpyridin-N-oxid, Schmelzpunkt 218-220°C.

Das als Ausgangsmaterial eingesetzte 3,4-Dihydro-2,2-dimethyl-4-(3-methyl-2-pyridyl)-6-nitro-2H-1-benzopyran wurde wie folgt hergestellt:

685 mg 3,4-Dihydro-2,2-dimethyl-4-(3-methyl-2-pyridyl)-2H-1-benzopyran werden bei Raumtemperatur in 20 ml Acetonitril gelöst, und 360 mg Nitroniumtetrafluorborat werden zugegeben. Nach 30 Minuten wird das Lösungsmittel abgedampft und Aethylacetat und Wasser zugegeben. Die organische Phase wird über Natriumsulfat getrocknet und eingedampft. Der Rückstand wird an Kieselgel mit einem 1:4-Gemisch von Aethylacetat und Petroläther als Eluierungsmittel chromatographiert, wobei man 374 mg 3,4-Dihydro-2,2-dimethyl-4-(3-methyl-2-pyridyl)-6-nitro-2H-1-benzopyran in Form eines Festkörpers erhält, welcher nach Umkristallisation aus t-Butylmethyläther einen Schmelzpunkt von 164-165°C aufweist.

Beispiel 6

406 mg m-Chlorperbenzoesäure werden bei Raumtemperatur zu einer Lösung von 524 mg 2,2-Dimethyl-4-(2-pyridyl)-2H-1-benzopyran-6-carbonitril in 15 ml Dichlormethan gegeben. Nach 17 stündigem Rühren bei Raumtemperatur wird die Lösung mit Natriumbicarbonatlösung gewaschen. Die organische Phase wird über Natriumsulfat getrocknet und eingedampft. Der Rückstand wird an Kieselgel mit einem 1:4-Gemisch von Methanol und Aethylacetat als Eluierungsmittel chromatographiert. Dabei erhält man 240 mg 2-(6-Cyan-2,2-dimethyl-2H-1-benzopyran-4-yl)pyridin-N-oxid, welches nach Umkristallisation aus Aethylacetat einen Schmelzpunkt von 187-189 °C aufweist.

Das als Ausgangsmaterial eingesetzte 2,2-Dimethyl-4-(2-pyridyl)-2H-1-benzopyran-6-carbonitril wurde nach der nachstehenden Methode (A) oder (B) hergestellt.

(A) 53 mg Kupfer(I)jodid, 293 mg Triphenylphosphin und 99 mg Palladium(II)chlorid werden in 320 ml Diäthylamin gelöst. Dann gibt man 10.4 g 4-(1,1-Dimethyl-2-propinyloxy)benzonitril und 11.5 g 2-Jodpyridin zu und rührt das Reaktionsgemisch unter einer Stickstoffatmosphäre 3 Tage bei Raumtemperatur. Wasser und Aethylacetat werden zugegeben, und die organische Phase abgetrennt, über Natriumsulfat getrocknet und eingedampft. Der Rückstand wird an Kieselgel chromatographiert, wobei man zunächst ein 1:3 - und dann ein 1:2-Gemisch von Aethylacetat und Petroläther als Eluierungsmittel verwendet, wobei man 12.98 g 4-[1,1-Dimethyl-3-(2-pyridyl)-2-propinyloxy]benzonitril als Oel erhält.

12.98 g 4-[1,1-Dimethyl-3-(2-pyridyl)-2-propinyloxy]benzonitril werden in 50 ml 1,2-Dichlorbenzol gelöst und 5 Stunden zum Rückfluss erhitzt. Nach dem Abkühlen wird das Reaktionsgemisch eingedampft. Der Rückstand wird an Kieselgel chromatographiert, wobei Aethylacetat/Petroläther (1:2), Aethylacetat/Petroläther (1:1) und Aethylacetat als Eluierungsmittel verwendet werden. Auf diese Weise erhält man 6.5 g 2,2-Dimethyl-4-(2-pyridyl)-2H-1-benzopyran-6-carbonitril, welches nach Umkristallisation aus Cyclohexan einen Schmelzpunkt von 106-108 °C aufweist.

(B) 280 mg 2-(6-Cyan-3,4-dihydro-2,2-dimethyl-2H-1-benzopyran-4-yl)pyridin-N-oxid werden während 24 Stunden in 3 ml Essigsäureanhydrid auf 120 °C erhitzt. Nach dem Abkühlen wird eingedampft, und der Rückstand zwischen Aethylacetat und wässriger Natriumbicarbonatlösung verteilt. Die organische Phase wird über Natriumsulfat getrocknet und eingedampft. Kristallisation des Rückstands aus Cyclohexan liefert 181 mg 2,2-Dimethyl-4-(2-pyridyl)-2H-1-benzopyran-6-carbonitril, welches ohne weitere Reinigung für die nächste Stufe eingesetzt wird.

Beispiel 7

406 mg m-Chlorperbenzoesäure werden bei Raumtemperatur zu einer Lösung von 528 mg 3,4-Dihydro-2,2-dimethyl-4-(2-pyridyl)-2H-1-benzopyran-6-carbonitril in 15 ml Dichlormethan gegeben. Nach 2 Stunden bei Raumtemperatur wird das Reaktionsgemisch mit Natriumbicarbonatlösung gewaschen, und die organische Phase über Natriumsulfat getrocknet und eingedampft. Der Rückstand wird aus t-Butylmethyläther kristallisiert und danach aus Toluol umkristallisiert, wobei man 360 mg 2-(6-Cyan-3,4-dihydro-2,2-dimethyl-2H-1-benzopyran-4-yl)pyridin-N-oxid erhält, Schmelzpunkt 158-160 °C.

Das als Ausgangsmaterial eingesetzte 3,4-Dihydro-2,2-dimethyl-4-(2-pyridyl)-2H-1-benzopyran-6-carbonitril wurde wie folgt hergestellt:

2.96 g 2,2-Dimethyl-4-(2-pyridyl)-2H-1-benzopyran-6-carbonitril werden in 100 ml Aethanol gelöst und bei Raumtemperatur zu 100 mg 10% Palladium auf Kohle gegeben. Das Reaktionsgemisch wird während 2 Stunden unter einer Wasserstoffatmosphäre bei Raumtemperatur geschüttelt. Der Katalysator wird dann abfiltriert, und das Filtrat eingedampft. Der Rückstand wird an Kieselgel mit einem 1:2-Gemisch von Aethylacetat und Petroläther als Eluierungsmittel chromatographiert. Dabei werden 2.44 g 3,4-Dihydro-2,2-dimethyl-4-(2-pyridyl)-2H-1-benzopyran-6-carbonitril erhalten, Schmelzpunkt 114-115 °C.

Beispiel 8

0.47 g 2-[1-(2-Hydroxy-5-methylthiophenyl)-3-methyl-2-butenyl]pyri-din-N-oxid werden in 10 ml Dichlormethan aufgenommen und mit 5 Tropfen Schwefelsäure 1 Stunde bei 20 °C gerührt. Das Reaktionsgemisch wird mit 20 ml Dichlormethan verdünnt und mit wässriger Natriumcarbonatlösung gewaschen. Die organische Phase wird eingedampft, und der Rückstand an Kieselgel chromatographiert, wobei man ein 19:1-Gemisch von Diäthyläther und Methanol als Eluierungsmittel verwendet. Dabei werden 0,44 g 2-(3,4-Dihydro-2,2-dimethyl-6-methylthio-2H-1-benzopyran-4-yl)pyridin-N-oxid als leicht gelbes Oel erhalten.

| Analyse für $C_{17}H_{19}NO_2S$: | | | |
|---|---|---|---|
| Berechnet: | C:67.7; | H:6.35; | N:4.65% |
| Gefunden: | C:67.8; | H:6.4; | N:4.6%. |

Das als Ausgangsmaterial eingesetzte 2-[1-(2-Hydroxy-5-methylthiophenyl)-3-methyl-2-butenyl]pyrid in-N-oxid wurde wie folgt hergestellt:

(A) 6.54 g 2-Picolin-N-oxid werden unter Rühren tropfenweise zu einer Suspension von 1.8 g Natriumhydrid 80% (G/G) in 50 ml trockenem Tetrahydrofuran gegeben. Das Reaktionsgemisch wird eine halbe

Stunde bei 20 °C gerührt und danach eine halbe Stunde zum Rückfluss erhitzt. Dann werden 15.2 g Aethyl 2-methyl-2-(4-methylthiophenoxy)propionat zugegeben, und das Reaktionsgemisch weitere 18 Stunden zum Rückfluss erhitzt. Das Lösungsmittel wird danach abedampft, und der Rückstand in Wasser aufgenommen, der pH-Wert mit 2M Salzsäure auf 5 eingestellt, und die Lösung mit Dichlormethan extrahiert. Die organische Phase wird eingedampft, und der Rückstand an Kieselgel mit einem 85:15-Gemisch von Diäthyläther und Methanol als Eluierungsmittel chromatographiert, wobei man 10.1 g 2-[3-Methyl-3-[4-(methylthio)phenoxy]-2-oxobutyl]pyridin-N-oxidin Form eines gelben Sirups erhält.

| Analyse für $C_{17}H_{19}NO_3S$: | | | |
|---|---|---|---|
| Berechnet: | C:64.3; | H:6.0; | N:4.4% |
| Gefunden | C:64.1; | H:6.0; | N:4.3% |

(B) 0.76 g Natriumborhydrid werden unter Rühren zu einer Lösung von 6.34 g 2-[3-Methyl-3-[4-(methylthio)phenoxy]-2-oxobutyl]pyridin-N-oxid in 50 ml Aethanol gegeben. Dann rührt man das Reaktionsgemisch 1 Stunde bei 20 °C, gibt 50 ml Wasser zu und entfernt das Aethanol durch Abdampfen. Die wässrige Lösung wird mit Aethylacetat extrahiert, und der organische Extrakt eingedampft, wobei man 5.65 g 2-[2-Hydroxy-3-methyl-3-(4-methylthiophenoxy)butyl]pyridin-N-oxid als weissen Festkörper erhält, Schmelzpunkt 98-100 °C (aus Diäthyläther).

(C) 1.25 g Methansulfonylchlorid werden unter Rühren tropfenweise zu einer Lösung von 2.73 g 2-[2-Hydroxy-3-methyl-3-(4-methylthiophenoxy)butyl]pyridin-N-oxid in 10 ml Pyridin gegeben, und das Reaktionsgemsisch 16 Stunden bei 20 °C gerührt. Weitere 1.25 g Methansulfonylchlorid werden danach zugegeben, und das Rühren für weitere 3 Stunden fortgesetzt. Danach wird das Reaktionsgemisch in 2M Salzsäure gegossen und mit Aethylacetat extrahiert. Das Lösungsmittel wird abgedampft, und der Rückstand aus Aethylacetat kristallisiert, wobei man 0.95 g des Methansulfonsäuresalzes von 2-[2-Methansulfonyloxy-3-methyl-3-(4-methylthiophenoxy)butyl]pyridin-N-oxid als weissen Festkörper erhält, Schmelzpunkt 126-128 °C.

(D) Eine Lösung von 3.35 g des Methansulfonsäuresalzes von 2-[2-Methansulfonyloxy-3-methyl-3-(4-methylthiophenoxy)butyl]pyridin-N-oxid und 2.25 g Natriumjodid in 50 ml Aceton wird während 2 Stunden zum Rückfluss erhitzt. Das Gemisch wird filtriert, und das Filtrat eingedampft. Der Rückstand wird zwischen Wasser und Dichlormethan verteilt, und die organische Phase eingedampft. Der Rückstand wird in 50 ml Aceton aufgenommen und 2 Stunden mit 2.25 g Natriumjodid zum Rückfluss erhitzt. Das abgekühlte Reaktionsgemisch wird danach eingedampft, und der Rückstand zwischen Wasser und Dichlormethan verteilt. Die organische Phase wird abgedampft, und der Rückstand aus Dichlormethan/Aethylacetat kristallisiert, wobei man 1.8 g 2-[2-Jod-3-methyl-3-(4-methylthiophenoxy)-butyl]pyridin-N-oxid als leicht gelben Festkörper erhält, Schmelzpunkt 191-193 °C.

(E) Eine Lösung von 0.86 g 2-[2-Jod-3-methyl-3-(4-methylthiophenoxy)butyl]pyridin-N-oxid und 2 ml Diisopropyläthylamin in 10 ml Aethanol wird 24 Stunden zum Rückfluss erhitzt. Das Lösungsmittel wird dann abgedampft, und der Rückstand zwischen 2M Salzsäure und Dichlormethan verteilt. Die organische Phase wird eingedampft, und der Rückstand aus Aethylacetat/n-Hexan umkristallisiert, wobei man 0.47 g 2-[1-(2-Hydroxy-5-methylthiophenyl)-3-methyl-2-butenyl]pyridin-N-oxid als weisslichen Festkörper erhält.

Beispiel 9

Eine Lösung von 0.45 g 2-(3,4-Dihydro-2,2-dimethyl-6-methylthio-2H-1-benzopyran-4-yl)pyridin-N-oxid und 0.645 g m-Chlorperbenzoesäure in 10 ml Dichlormethan wird 16 Stunden bei 20 °C gerührt. Das Reaktionsgemisch wird dann mit 20 ml Dichlormethan verdünnt, die erhaltene Lösung mit wässriger Natriumbicarbonatlösung und Natriumchloridlösung gewaschen und eingedampft. Kristallisation des Rückstand aus Aethylacetat liefert 0.446 g 2-(3,4-Dihydro-2,2-dimethyl-6-methylsulfonyl-2H-1-benzopyran-4-yl)-pyridin-N-oxid als weisslichen Festkörper, Schmelzpunkt 213-214 °C.

Beispiel 10

1.61 g 2,2-Dimethyl-4-(2-pyridyl)-2H-1-benzopyran werden bei Raumtemperatur in 10 ml Dichlormethan gelöst und mit 2.0 g m-Chloroperbenzoesäure versetzt. Nach 1 Stunde wird das Reaktionsgemisch mit Natriumbisulfitlösung, Natriumbicarbonatlösung und Wasser gewaschen. Die organische Phase wird über Natriumsulfat getrocknet und eingedampft. Der Rückstand wird an Kieselgel chromatographiert, wobei man ein 40:4:1-Gemisch von Aethylacetat, Aethanol und Ameisensäure als Eluierungsmittel verwendet. Das

Produkt wird als Oel erhalten, welches nach Anreiben mit Diäthyläther fest wird. Umkristallisation aus Toluol liefert 0.034 g 2-(2,2-Dimethyl-2H-1-benzopyran-4-yl)pyridin-N-oxid, Schmelzpunkt 148-150 °C.

Beispiel 11

330 mg 6-Brom-3,4-dihydro-2,2-dimethyl-4-(2-pyridyl)-2H-1-benzopyran werden bei Raumtemperatur in 10 ml Dichlormethan gelöst und mit 280 mg m-Chlorperbenzoesäure versetzt. Nach 3-tägigem Rühren bei Raumtemperatur wird das Reaktionsgemisch nacheinander mit Natriumbisulfitlösung, Natriumbicarbonatlösung und Wasser gewaschen. Die organische Phase wird über Natriumsulfat getrocknet und eingedampft. Der Rückstand wird mit 4%(V/V) Methanol/Aethylacetat als Eluierungsmittel an Kieselgel chromatographiert, wobei man 250 mg 2-(6-Brom-3,4-dihydro-2,2-dimethyl-2H-1-benzopyran-4-yl)pyridin-N-oxid als Schaum erhält. NMR (300 MHz, $CDCl_3$): δ 8.35-8.30 (1H, m), 7.26-7.15 (3H, m), 7.13-7.05 (1H, m), 6.95 (1H, breit,s), 6.75 (1H, d, 9Hz), 5.28 (1H, breit,s), 2.42 (1H, dd, 13Hz, 6Hz), 1.7 (1H, breit,s), 1.42 (3H, s), 1.37 (3H, s). MS (EI): 335 ($M^+[Br^{81}]$), 333 ($M^+[Br^{79}]$), 318 ($M^+[Br^{81}]$-OH), 316 ($M^+[Br^{79}]$-OH).

Das als Ausgangsmaterial eingesetzte 6-Brom-3,4-dihydro-2,2-dimethyl-4-(2-pyridyl)-2H-1-benzopyran wurde wie folgt hergestellt:

0.5 g 3,4-Dihydro-2,2-dimethyl-4-(2-pyridyl)-2H-1-benzopyran werden bei Raumtemperatur in 10 ml Tetrachlorkohlenstoff gelöst und mit 0.25 ml Pyridin und 0.12 ml Brom versetzt. Das Reaktionsgemisch wird 1 Stunde bei Raumtemperatur gerührt, dann eine Stunde bei 35 °C und schliesslich 1 Stunde bei 65 °C. Nach dem Abkühlen des Reaktionsgemisches wird dieses mit Natriumbicarbonatlösung gewaschen, die wässrigen Waschlösungen mit Dichlormethan extrahiert, und die vereinigten organischen Phasen mit Wasser gewaschen, über Natriumsulfat getrocknet und eingedampft. Chromatogrphie des Rückstands an Kieselgel mit 2% (V/V) Methanol/Dichlormethan als Eluierungsmittel und Umkristallisation aus t-Butylmethyläther liefert 230 mg 6-Brom-3,4-dihydro-2,2-dimethyl)-4-(2-pyridyl)-2H-1-benzopyran, Schmelzpunkt 134-136 °C.

Beispiel 12

402 mg Methyl 2,2-dimethyl-4-(2-pyridyl)-2H-1-benzopyran-6-carboxylat werden bei Raumtemperatur in 15 ml Dichlormethan gelöst und mit 330 mg m-Chlorperbenzoesäure versetzt. Nach dem Rühren bei Raumtemperatur über Nacht wird das Reaktionsgemisch mit Natriumbisulfitlösung und Natriumbicarbonatlösung gewaschen, über Natriumsulfat getrocknet und eingedampft. Der Rückstand wird mit 10% (V/V) Methanol/ Aethylacetat als Eluierungsmittel chromatographiert. Umkristallisation des Produkts aus t-Butylmethyläther liefert 65 mg 2-[6-(Methoxycarbonyl)-2,2-dimethyl-2H-1-benzopyran-4-yl]pyridin-N-oxid, Schmelzpunkt 155-157 °C.

Das als Augangsmaterial eingesetzte Methyl 2,2-dimethyl-4-(2-pyridyl)-2H-1-benzopyran-6-carboxylat wurde wie folgt hergestellt:

(A) 2.2 g 2,2-Dimethyl-4-(2-pyridyl)-2H-1-benzopyran-6-carbonitril werden in 40 ml 0.5M Natriumhydroxidlösung suspendiert und 18 Stunden zum Rückfluss erhitzt. Das Reaktionsgemisch wird danach auf Raumtemperatur abgekühlt und mit Aethylacetat extrahiert. Die wässrige Phase wird mit Citronensäure angesäuert (pH 6), worauf ein Festkörper auskristallisiert. Dieser Festkörper wird abfiltriert, mit Diäthyläther gewaschen und aus Aethanol umkristallisiert, wobei 1.27 g 2,2-Dimethyl-4-(2-pyridyl)-2H-1-benzopyran-6-carbonsäure erhalten werden, Schmelzpunkt 238-240 °C.

(B) 0.65 g 2,2-Dimethyl-4-(2-pyridyl)-2H-1-benzopyran-6-carbonsäure werden in 10 ml Thionylchlorid gelöst und 1 Stunde bei Raumtemperatur gerührt. Man dampft das Reaktionsgemisch ein, gibt 5 ml Methanol zu und dampft das Reaktionsgemisch erneut ein. Der Rückstand wird zwischen Diäthyläther und wässriger Natriumhydroxidlösung verteilt. Die organische Phase wird abgetrennt, über Natriumsulfat getrocknet und eingedampft. Der Rückstand wird an Kieselgel mit einem 1:1-Gemisch von Aethylacetat/Petroläther als Eluierungsmittel chromatographiert. Umkristallisation des Produkts aus Cyclohexan liefert 345 mg Methyl 2,2-dimethyl-4-(2-pyridyl)-2H-1-benzopyran-6-carboxylat, Schmelzpunkt 94-95 °C.

Beispiel 13

313 mg Methyl 3,4-dihydro-2,2-dimethyl-4-(2-pyridyl)-2H-1-benzopyran-6-carboxylat werden bei Raumtemperatur in 10 ml Dichlormethan gelöst und mit 283 mg m-Chlorperbenzoesäure versetzt. Nach 2-stündigem Rühren bei Raumtemperatur wird das Reaktionsgemisch mit Natriumbisulfitlösung und Natrimbicarbonatlösung gewaschen. Die organische Phase wird über Natriumsulfat getrocknet und eingedampft. Der

Rückstand wird an Kieselgel chromatographiert, wobei zunächst 2% (V/V) Methanol/Aethylacetat und schliesslich 5% (V/V) Methanol/Aethylacetat als Eluierungsmittel verwendet werden. Dabei erhält man 250 mg 2-[3,4-Dihydro-6-(methoxycarbonyl)-2,2-dimethyl-2H-1-benzopyran-4-yl]pyridin-N-oxid als Oel, welches sich beim Anreiben mit Diäthyläther verfestigt. Nach Umkristallisation auf Toluol schmilzt das Produkt bei 128-130 °C.

Das als Ausgangsmaterial eingesetzte Methyl 3,4-dihydro-2,2-dimethyl-4-(2-pyridyl)-2H-1-benzopyran-6-carboxylat wurde wie folgt hergestellt:

(A) 2.44 g 3,4-Dihydro-2,2-dimethyl-4-(2-pyridyl)-2H-1-benzopyran-6-carbonitril werden in 100 ml 0.37M Natriumhydroxidlösung suspendiert und über Nacht zum Rückfluss erhitzt. Die erhaltene Lösung wird mit Aethylacetat extrahiert, und die wässrige Phase mit Citronensäure angesäuert. Der ausgefallene Festkörper wird abfiltriert und mit Wasser und Diäthyläther gewaschen. Auf diese Weise erhält man 1.6 g 3,4-Dihydro-2,2-dimethyl-4-(2-pyridyl)-2H-1-benzopyran-6-carbonsäure, Schmelzpunkt 207-208 °C.

(B) 0.8 g 3,4-Dihydro-2,2-dimethyl-4-(2-pyridyl)-2H-1-benzopyran-6-carbonsäure werden bei Raumtemperatur in 10 ml Thionylchlorid gerührt. Die Reaktion wird mit Dünnschichtchromatographie an Kieselgel unter Verwendung eines 40:4:1-Gemisches von Aethylacetat/Aethanol/Ameisensäure als Fliessmittel verfolgt. Nachdem die Reaktion vollständig ist, dampft man das Reaktionsgemisch ein, gibt Toluol zu, dampft das Gemisch erneut ein, gibt Methanol zu und dampft das Gemisch nocheinmal ein. Der Rückstand wird zwischen Diäthyläther und Natriumbicarbonatlösung verteilt. Die organische Phase wird über Natriumsulfat getrocknet und eingedampft. Der Rückstand wird mit einem 1:1-Gemisch von Aethylacetat/Petroläther als Eluierungsmittel chromatographiert, wobei man 430 mg Methyl 3,4-dihydro-2,2-dimethyl-4-(2-pyridyl)-2H-1-benzopyran-6-carboxylat erhält, welches nach Umkristallisation aus Cyclohexan bei 113-115 °C schmilzt.

## Beispiel 14

564 mg 3,4-Dihydro-2,2-dimethyl-4-(2-pyridyl)-2H-1-benzopyran-6-carboxamid werden bei Raumtemperatur in 20 ml Dichlormethan gelöst und mit 540 mg m-Chlorperbenzoesäure versetzt. Nach Rühren über Nacht bei Raumtemperatur wird das Reaktionsgemisch mit Natriumbisulfitlösung, Natriumbicarbonatlösung und Wasser gewaschen. Die organische Phase wird über Natriumsulfat getrocknet und eingedampft, wobei man einen Festkörper erhält, welcher nach Umkristallisation aus Isopropanol 164 mg 2-(6-Carbamoyl-3,4-dihydro-2,2-dimethyl-2H-1-benzopyran-4-yl)pyridin-N-oxid liefert, Schmelzpunkt 248-250 °C.

Das als Ausgangsmaterial eingesetzte 3,4-Dihydro-2,2-dimethyl-4-(2-pyridyl)-2H-1-benzopyran-6-carboxamid wurde wie folgt hergestellt:

803 mg 3,4-Dihydro-2,2-dimethyl-4-(2-pyridyl)-2H-1-benzopyran-6-carbonsäure werden in 10 ml Thionylchlorid gelöst und bei Raumtemperatur 1 Stunde gerührt. Das Gemisch wird eingedampft, der Rückstand in Toluol gelöst, und die Lösung erneut eingedampft. Der Rückstand wird mit 0,35% (G/V) Ammoniak versetzt, und das Reaktionsgemisch zwichen Aethylacetat und Natriumbicarbonatlösung verteilt. Die organische Phase wird mit Wasser und Natriumchloridlösung gewaschen, über Natriumsulfat getrocknet und eingedampft. Umkristallisation des Rückstands aus Aethanol liefert 400 mg 3,4-Dihydro-2,2-dimethyl-4-(2-pyridyl)-2H-1-benzopyran-6-carboxamid, Schmelzpunkt 225-227 °C.

## Beispiel 15

In analoger Weise wie im ersten Absatz von Beispiel 11 beschrieben wurde aus 6-Chlor-3,4-dihydro-2,2-dimethyl-4-(2-pyridyl)-2H-1-benzopyran das 2-(6-Chlor-3,4-dihydro-2,2-dimethyl-2H-1-benzopyran-4-yl)-pyridin-N-oxid als Schaum erhalten. NMR(300 MHz, $CDCl_3$): δ 8.36-8.30 (1H, m), 7.26-7.18 (2H, m), 7.14-7.05 (2H, m), 6.84-6.75 (2H, m), 5.29 (1H, breit,s), 2.42 (1H, dd, 12.5Hz, 6Hz), 1.76 (1H, breit,s), 1.44 (3H, s), 1.39 (3H, s). MS (EI): 289 ($M^+$ [$Cl^{35}$]), 272 ($M^+$ [$Cl^{35}$]-OH).

Das als Ausgangsmaterial eingesetzte 6-Chlor-3,4-dihydro-2,2-dimethyl-4-(2-pyridyl)-2H-1-benzopyran wurde wie folgt hergestellt:

1 g 3,4-Dihydro-2,2-dimethyl-4-(2-pyridyl)-2H-1-benzopyran wird bei Raumtemperatur in 20 ml Tetrachlorkohlenstoff gelöst und mit 0.25 ml Pyridin und 10 ml einer 0.42M Lösung von Chlor in Tetrachlorkohlenstoff versetzt. Nach 30 Minuten wird das Reaktionsgemisch mit Natriumbicarbonatlösung gewaschen, und die organische Phase abgetrennt, über Natriumsulfat getrocknet und eingedampft. Der Rückstand wird an Kieselgel mit 1% (V/V) Methanol/ Dichlormethan und danach mit 2% (V/V) Methanol/Dichlormethan als Eluierungsmittel chromatographiert. Umkristallisation der erhaltenen Produkts aus t-Butylmethyläther liefert 200 mg 6-Chlor-3,4-dihydro-2,2-dimethyl-4-(2-pyridyl)-2H-1-benzopyran, Schmelzpunkt 124-125 °C.

Beispiel 16

In analoger Weise wie im ersten Absatz von Beispiel 11 beschrieben wurde aus 4-(2-Pyridyl)-2,2,6-trimethyl-2H-1-benzopyran das 2-(2,2,6-Trimethyl-2H-1-benzopyran-4-yl)pyridin-N-oxid erhalten. Schmelzpunkt 189-191 °C nach Umkristallisation aus Aethylacetat.

Das als Ausgangsmaterial eingesetzte 4-(2-Pyridyl)-2,2,6-trimethyl-2H-1-benzopyran wurde in analoger Weise wie in Beispiel 4 (A)-(D) beschrieben aus 2-Brom-4-methylanisol und 2-Cyanpyridin hergestellt.

Beispiel 17

In analoger Weise wie im ersten Absatz von Beispiel 6 beschrieben wurde aus 2,2-Dimethyl-4-(2-pyridyl)-6-(trifluormethyl)-2H-1-benzopyran das 2-[6-(Trifluormethyl)-2,2-dimethyl-2H-1-benzopyran-4-yl]-pyridin-N-oxid erhalten, Schmelzpunkt 149-152 °C nach Umkristallisation aus Cyclohexan.

Das als Ausgangsmaterial eingesetzte 2,2-Dimethyl-4-(2-pyridyl)-6-trifluormethyl-2H-1-benzopyran wurde wie folgt hergestellt:

(A) 1.62 g 4-(Trifluormethyl)phenol, 1.53 g 2-Chlor-2-methyl-3-butin und 10 g Kaliumcarbonat werden in 50 ml Aceton zum Rückfluss erhitzt. Nach 18, 42 und 66 Stunden gibt man jeweils weitere 1.53 g 2-Chlor-2-methyl-3-butin zu. 72 Stunden nach der letzten Zugabe lässt man das Reaktionsgemisch auf Raumtemperatur abkühlen und verteilt es zwischen Diäthyläther und Wasser. Die organische Phase wird mit wässriger Natriumhydroxidlösung gewaschen, über Natriumsulfat getrocknet und eingedampft. Der Rückstand wird mit 5% (V/V) Aethylacetat/Petroläther als Eluierungsmittel an Kieselgel chromatographiert, wobei man 1.8 g 4-(1,1-Dimethyl-2-propinyloxy)trifluormethylbenzol als gelbes Oel erhält.

(B) Das 4-(1,1-Dimethyl-2-propinyloxy)trifluormethylbenzol wird in analoger Weise wie in Beispiel 6 (A) beschrieben in das 2,2-Dimethyl-4-(2-pyridyl)-6-trifluormethyl-2H-1-benzopyran übergeführt.

Beispiel 18

In analoger Weise wie im ersten Absatz von Beispiel 3 beschrieben wurde aus 6-(t-Butyl)-3,4-dihydro-2,2-dimethyl-4-(2-pyridyl)-2H-1-benzopyran das 2-[6-(t-Butyl)-3,4-dihydro-2,2-dimethyl-2H-1-benzopyran-4-yl]pyridin-N-oxid als weisser Festkörper erhalten, Schmelzpunkt 128-130 °C nach Umkristallisation aus Cyclohexan.

Das als Ausgangsmaterial eingesetzte 6-(t-Butyl)-3,4-dihydro-2,2-dimethyl-4-(2-pyridyl)-2H-1-benzopyran wurde in analoger Weise wie im letzten Absatz von Beispiel 3 beschrieben hergestellt, wobei jedoch Pivaloylchlorid anstelle von Acetylchlorid eingesetzt wurde.

Beispiel 19

94 mg 6-Benzoyl-3,4-dihydro-2,2-dimethyl-4-(2-pyridyl)-2H-1-benzopyran und 52 mg m-Chlorperbenzoesäure werden in 15 ml Dichlormethan bei Raumtemperatur so lange gerührt, bis das Dünnschichtchromatogramm die Vollständigkeit der Reaktion anzeigt. Das Reaktionsgemisch wird dann nacheinander mit Natriumbisulfitlösung und Natriumsulfatlösung gewaschen, über Natriumsulfat getrocknet und eingedampft. Der Rückstand wird an Kieselgel mit 10% (V/V) Methanol/Aethylacetat als Eluierungsmittel chromatographiert. Nach Anreiben mit Diäthyläther erhält man 20 mg 2-(6-Benzoyl-3,4-dihydro-2,2-dimethyl-2H-1-benzopyran-4-yl)-pyridin-N-oxid, Schmelzpunkt 134-136 °C.

Das als Ausgangsmaterial eingesetzte 6-Benzoyl-3,4-dihydro-2,2-dimethyl-4-(2-pyridyl)-2H-1-benzopyran wurde wie folgt hergestellt:

264 mg Aluminiumchlorid werden zu einer eisgekühlten Lösung von 237 mg 3,4-Dihydro-2,2-dimethyl-4-(2-pyridyl)-2H-1-benzopyran in 6 ml Nitromethan gegeben. Das Reaktionsgemisch wird 5 Minuten gerührt, mit 349 mg Benzoylchlorid versetzt und dann 30 Minuten bei 0 °C und 16 Stunden bei Raumtemperatur weitergerührt. Das Reaktionsgemisch wird mit Diäthyläther verdünnt und mit Natriumhydroxidlösung gewaschen. Die organische Phase wird über Natriumsulfat getrocknet und eingedampft. Chromatographie des Rückstands an Kieselgel mit einem 2:3-Gemisch von Aethylacetat und Petroläther als Eluierungsmittel liefert 94 mg 6-Benzoyl-3,4-dihydro-2,2-dimethyl-4-(2-pyridyl)-2H-1-benzopyran.

Beispiel 20

134 mg 3,4-Dihydro-2,2-dimethyl-6-(4-nitrobenzoyl)-4-(2-pyridyl)-2H-1-benzopyran werden in 15 ml Dichlormethan gelöst und mit 72 mg m-Chlorperbenzoesäure versetzt. Das Reaktionsgemisch wurde über

Nacht bei Raumtemperatur gerührt und dann nacheinander mit Natriumbisulfitlösung und Natriumbicarbonatlösung gewaschen. Die organische Phase wird über Natriumsulfat getrocknet und eingedampft. Der Rückstand wird an Kieselgel mit einem 4:1-Gemisch von Aethylacetat und Methanol als Eluierungsmittel chromatographiert. Umkristallisation des Rückstands aus Isopropanol liefert 50 mg 2-[3,4-Dihydro-2,2-dimethyl-6-(4-nitrobenzoyl)-2H-1-benzopyran-4-yl]pyridin-N-oxid, Schmelzpunkt 209-211 °C.

Das als Ausgangsmaterial eingesetzte 3,4-Dihydro-2,2-dimethyl-6-(4-nitrobenzoyl)-4-(2-pyridyl)-2H-1-benzopyran wurde wie folgt hergestellt:

200 mg 3,4-Dihydro-2,2-dimethyl-4-(2-pyridyl)-2H-1-benzopyranwerden in 10 ml Nitromethan gelöst, und die Lösung unter einer Stickstoffatmosphäre auf 0 °C abgekühlt. Man gibt zunächst 240 mg Aluminiumchlorid in Form eines feinen Puders und, nach 5 Minuten Rühren bei 0 °C, 388 mg 4-Nitrobenzoylchlorid zu. Nach 16 Stunden bei Raumtemperatur werden 120 mg Aluminiumchlorid zugegeben, und das Gemisch 45 Minuten bei 100 °C gerührt. Nach Verdünnen mit Diäthyläther und Waschen mit Natriumhydroxidlösung wird die organische Phase über Natriumsulfat getrocknet und eingedampft. Der Rückstand kristallisiert und wird mit Diäthyläther angerieben, wobei man 150 mg 3,4-Dihydro-2,2-dimethyl-6-(4-nitrobenzoyl)-4-(2-pyridyl)-2H-1-benzopyran erhält.

Beispiel 21

207 mg 3,4-Dihydro-2,2-dimethyl-6-(2-jodbenzoyl)-4-(2-pyridyl)-2H-1-benzopyran werden in 15 ml Dichlormethan mit 76 mg m-Chlorperbenzoesäure 3 Stunden gerührt. Das Reaktionsgemisch wird nacheinander mit Natriumbisulfitlösung und Natriumbicarbonatlösung gewaschen, die organische Phase über Natriumsulfat getrocknet und eingedampft. Der Rückstand wird an Kieselgel mit 13% (V/V) Methanol/Aethylacetat als Eluierungsmittel chromatographiert, wobei man nach Umkristallisation aus Diäthyläther 15 mg 2-[3,4-Dihydro-2,2-dimethyl-6-(2-jodbenzoyl)-2H-1-benzopyran-4-yl]pyridin-N-oxid erhält, Schmelzpunkt 114-120 °C.

Das als Ausgangsmaterial eingesetzte 3,4-Dihydro-2,2-dimethyl-6-(2-jodbenzoyl)-4-(2-pyridyl)-2H-1-benzopyran wurde wie folgt hergestellt:

250 mg 3,4-Dihydro-2,2-dimethyl-4-(2-pyridyl)-2H-1-benzopyran werden in 10 ml Nitromethan gelöst und in einem Eisbad unter einer Stickstoffatmosphäre gekühlt. 280 mg Aluminiumchlorid in Form eines feinen Puders und nach 5 Minuten 700 mg 2-Jodbenzoylchlorid werden augegeben. Nach 30-minütigem Rühren bei 0 °C und 1-stündigem Rühren bei Raumtemperatur wird das Reaktionsgemisch mit Diäthyläther verdünnt und mit Natriumhydroxidlösung gewaschen. Die organische Phase wird über Natriumsulfat getrocknet und eingedampft. Chromatographie des Rückstands an Kieselgel mit einem 1:2-Gemisch von Aethylacetat und Petroläther liefert 230 mg 3,4-Dihydro-2,2-dimethyl-6-(2-jodbenzoyl)-4-(2-pyridyl)-2H-1-benzopyran.

Beispiel 22

In analoger Weise wie im ersten Absatz von Beispiel 21 beschrieben wurde aus 3,4-Dihydro-2,2-dimethyl-6-(3-jodbenzoyl)-4-(2-pyridyl)-2H-1-benzopyran das 2-[3,4-Dihydro-2,2-dimethyl-6-(3-jodbenzoyl)-2H-1-benzopyran-4-yl]pyridin-N-oxid erhalten, Schmelzpunkt 128-130 °C (aus Cyclohexan/Aethylacetat).

Das als Ausgangsmaterial eingesetzte 3,4-Dihydro-2,2-dimethyl-6-(3-jodbenzoyl)-4-(2-pyridyl)-2H-1-benzopyran wurde in analoger Weise wie im letzten Absatz von Beispiel 21 beschrieben hergestellt, wobei 3-Jodbenzoylchlorid anstelle von 2-Jodbenzoylchlorid verwendet wurde.

Beispiel 23

In analoger Weise wie im ersten Absatz von Beispiel 21 beschrieben wurde aus 3,4-Dihydro-2,2-dimethyl-6-(4-jodbenzoyl)-4-(2-pyridyl)-2H-1-benzopyran das 2-[3,4-Dihydro-2,2-dimethyl-6-(4-jodbenzoyl)-2H-1-benzopyran-4-yl]pyridin-N-oxid erhalten, Schmelzpunkt 171-173 °C (aus Aethylacetat).

Das als Ausgangsmaterial eingesetzte 3,4-Dihydro-2,2-dimethyl-6-(4-jodbenzoyl)-4-(2-pyridyl)-2H-1-benzopyran wurde in analoger Weise wie im letzten Absatz von Beispiel 21 beschrieben hergestellt, wobei 4-Jodbenzoylchlorid anstelle von 2-Jodbenzoylchlorid verwendet wurde.

Beispiel 24

In analoger Weise wie im ersten Absatz von Beispiel 6 beschrieben wurde aus 2-Aethyl-2-methyl-4-(2-pyridyl)-2H-1-benzopyran-6-carbonitril das 2-(6-Cyan-2-äthyl-2-methyl-2H-1-benzopyran-4-yl)pyridin-N-oxid

erhalten, Schmelzpunkt 129-131 °C (aus Aethylacetat/Petroläther).

Das als Ausgangsmaterial eingesetzte 2-Aethyl-2-methyl-4-(2-pyridyl)-2H-1-benzopyran-6-carbonitril wurde wie folgt hergestellt:

(A) 17.47 g 2-Chlor-2-methylpent-1-in werden zu einem Gemisch von 11.9 g 4-Cyanphenol, 6.0 g Natriumhydroxid, 15 ml einer 40%igen methanolischen Lösung von Benzyltrimethylammoniumhydroxid in 85 ml Wasser und 85 ml Dichlormethan gegeben und 4 Tage gerührt. Die organische Phase wird abgetrennt, mit 2M Natriumhydroxidlösung gewaschen, über Natriumsulfat getrocknet und eingedampft. Der Rückstand wird mit einem 1:9-Gemisch von Aethylacetat und Petroläther als Eluierungsmittel chromatographiert, wobei man 10.1 g 4-(1-Aethyl-1-methyl-2-propinyloxy)benzonitril erhält.

(B) 4-(1-Aethyl-1-methyl-2-propinyloxy)benzonitril wird in analoger Weise wie im Beispiel 6(A) beschrieben in 2-Aethyl-2-methyl-4-(2-pyridyl)-2H-benzopyran-6-carbonitril übergeführt.

Beispiel 25

In analoger Weise wie im ersten Absatz von Beispiel 4 beschrieben wurde aus 6-Acetyl-2-methyl-4-(2-pyridyl)-2H-1-benzopyran das 2-(6-Acetyl-2-methyl-2H-1-benzopyran-4-yl)pyridin erhalten, Schmelzpunkt 170-172 °C (aus Toluol).

Das als Ausgangsmaterial eingesetzte 6-Acetyl-2-methyl-4-(2-pyridyl)-2H-1-benzopyran wurde aus 1-Brom-2-methylprop-1-en und 2-Methoxyphenyl-2-pyridylketon in analoger Weise wie in Beispiel 1 (A)-(D) beschrieben hergestellt.

Beispiel 26

152 mg 4-(6-Chlor-2-pyridyl)-3,4-dihydro-2,2-dimethyl-2H-1-benzopyran-6-carbonitril und 136 mg m-Chlorperbenzoesäure werden in 15 ml Chloroform zum Rückfluss erhitzt. Nach 10 Stunden und 20 Stunden werden weitere 80 mg bzw. 50 mg, m-Chlorperbenzoesäure zugegeben. Nach 30 Stunden wird das Reaktionsgemisch auf Raumtemperatur abgekühlt, nacheinander mit Natriumbisulfitlösung und Natriumbicarbonatlösung gewaschen, über Natriumsulfat getrocknet und eingedampft. Chromatographie des Rückstands an Kieselgel mit Aethylacetat als Eluierungsmittel liefert nach Anreiben mit n-Hexan einen Festkörper, welcher aus Acetonitril umkristallisiert wird. Auf diese Weise werden 33 mg 2-Chlor-6-(6-cyan-3,4-dihydro-2,2-dimethyl-2H-1-benzopyran-4-yl)pyridin-N-oxid erhalten, Schmelzpunkt 195-196 °C.

Das als Ausgangsmaterial eingesetzte 4-(6-Chlor-2-pyridyl)-3,4-dihydro-2,2-dimethyl-2H-1-benzopyran-6-carbonitril wurde wie folgt hergestellt:

1 g 2-(6-Cyan-3,4-dihydro-2,2-dimethyl-2H-1-benzopyran-4-yl)pyridin-N-oxid wird in 15 ml Phosphoroxychlorid gelöst, 2 Stunden auf 80 °C erhitzt und danach über Nacht auf Raumtemperatur abgekühlt. Das Phosphoroxychlorid wird unter vermindertem Druck entfernt, und der Rückstand in Natriumbicarbonatlösung und Aethylacetat aufgenommen. Die wässrige Phase wird mehrere Male mit Aethylacetat extrahiert, und die vereinigten Aethylacetatextrakte über Natriumsulfat getrocknet und eingedampft. Der Rückstand wird an Kieselgel chromatographiert. Eluieren mit einem 1:4-Gemisch von Aethylacetat und Petroläther liefert 271 mg 4-(6-Chlor-2-pyridyl)-3,4-dihydro-2,2-dimethyl-2H-1-benzopyran-6-carbonitril und Eluieren mit einem 1:2-Gemisch von Aethylacetat und Petroläther liefert 170 mg 4-(4-Chlor-2-pyridyl)-3,4-dihydro-2,2-dimethyl-2H-1-benzopyran-6-carbonitril.

Beispiel 27

79 mg 4-(4-Chlor-2-pyridyl)-3,4-dihydro-2,2-dimethyl-2H-1-benzopyran-6-carbonitril werden in 10 ml Dichlormethan gelöst und mit 70 mg m-Chlorperbenzoesäure versetzt. Das Reaktionsgemisch wird 3 Tage bei Raumtemperatur gerührt, dann nacheinander mit Natriumbisulfitlösung, Natriumbicarbonatlösung und Wasser gewaschen, über Natriumsulfat getrocknet und eingedampft. Der Rückstand wird an Kieselgel mit 1% (V/V) Methanol/Aethylacetat als Eluierungsmittel chromatographiert und liefert nach Anreiben mit Diäthyläther einen Festkörper, welcher aus Aethylacetat/Petroläther umkristallisiert wird. Auf diese weise erhält man 20 mg 4-Chlor-2-(6-cyan-3,4-dihydro-2,2-dimethyl-2H-1-benzopyran-4-yl)pyridin-N-oxid, Schmelzpunkt 173-174 °C.

Beispiel 28

250 mg 4-Chlor-2-(6-cyan-3,4-dihydro-2,2-dimethyl-2H-1-benzopyran-4-yl)pyridin-N-oxid werden in 6 ml Methanol gelöst und zu einer Lösung von Natriummäthoxid (hergestellt aus 0.5 g metallischem Natrium in

50 ml Methanol) gegeben. Das Reaktionsgemisch wird 2 Stunden unter einer Stickstoffatmosphäre zum Rückfluss erhitzt und danach auf Raumtemperatur abgekühlt. Die Lösung wird eingedampft, und der Rückstand in Aethylacetat und Wasser aufgenommen, die organische Phase abgetrennt, über Natriumsulfat getrocknet und eingedampft. Der Rückstand wird an Kieselgel mit 20% (V/V) Methanol/Aethylacetat als Eluierungsmittel chromatographiert, wobei man einen Festkörper erhält, welcher aus Aethylacetat umkristallisiert wird. Auf diese Weise erhält man 65 mg 2-(6-Cyan-3,4-dihydro-2,2-dimethyl-2H-1-benzopyran-4-yl)-4-methoxypyridin-N-oxid, Schmelzpunkt 196-198 ° C.

Beispiel 29

406 mg m-Chlorperbenzoesäure werden zu einer Lösung von 554 mg 4-(6-Amino-2-pyridyl)-2,2-dimethyl-2H-1-benzopyran-6-carbonitril in 10 ml Dichlormethan gegeben, und das Reaktionsgemisch 3 Tage gerührt. Das Gemisch wird dann mit Natriumbicarbonatlösung gewaschen, über Natriumsulfat getrocknet und eingedampft. Der Rückstand wird an Kieselgel mit einen 1:4-Gemisch von Methanol und Aethylacetat als Eluierungsmittel chromatographiert, wobei man einem Festkörper erhält, welcher nach Anreiben mit Dichlormethan und Umkristallisation aus Isopropanol 130 mg 2-Amino-6-(6-cyan-2,2-dimethyl-2H-1-benzopyran-4-yl)pyridin-N-oxid liefert, Schmelzpunkt 241-242 ° C.

Das als Ausgangsmaterial eingesetzte 4-(6-Amino-2-pyridyl)-2,2-dimethyl-2H-1-benzopyran-6-carbonitril wurde in analoger Weise wie in Beispiel 6(A) beschrieben aus 4-(1,1-Dimethyl-2-propinyloxy)benzonitril und 2-Amino-6-jodpyridin hergestellt.

Beispiel 30

In analoger Weise wie im ersten Absatz von Beispiel 7 beschrieben wurde aus 4-(6-Amino-2-pyridyl-)3,4-dihydro-2,2-dimethyl-2H-1-benzopyran-6-carbonitril das 2-Amino-6-(6-cyan-3,4-dihydro-2,2-dimethyl-2H-1-benzopyran-4-yl)pyridin-N-oxid erhalten, Schmelzpunkt 210-212 ° C (aus Isopropanol).

Das als Ausgangsmaterial eingesetzte 4-(6-Amino-2-pyridyl)-3,4-dihydro-2,2-dimethyl-2H-1-benzopyran-6-carbonitril wurde in analoger Weise wie im letzten Absatz von Beispiel 7 beschrieben aus 4-(6-Amino-2-pyridyl)-2,2-dimethyl-2H-1-benzopyran-6-carbonitril hergestellt.

Beisiel 31

350 mg 2-[1-(5-Cyan-2-hydroxyphenyl)-3-methyl-2-butenyl]-6-methylpyridin-N-oxid in 15 ml Dichlormethan werden mit 100 µl konzentierter Schwefelsäure eine Stunde gerührt. Weitere 100 µl konzentrierter Schwefelsäure werden zugegeben, und das Rühren wird 30 Minuten fortgesetzt. Das Reaktionsgemisch wird mit Wasser gewaschen, über Natriumsulfat getrocknet und eingedampft. Der Rückstand wird an Kieselgel mit einem 1:9-Gemisch von Methanol und Aethylacetat als Eluierungsmittel chromatographiert und liefert ein Oel, welches aus Diäthyläther kristallisiert. Auf diese Weise erhält man 0.26 g 2-(6-Cyan-3,4-dihydro-2,2-dimethyl-2H-1-benzopyran-4-yl)-6-methylpyridin-N-oxid, Schmelzpunkt 165-167 ° C.

Das als Ausgangsmaterial eingesetzte 2-[1-(5-Cyan-2-hydroxyphenyl)-3-methyl-2-butenyl]-6-methylpyridin-N-oxid wurde wie folgt hergestellt:

(A) 3.69 g 2,6-Lutidin-N-oxid in 15 ml trockenem Tetrahydrofuran werden bei -78 ° C unter einer Stickstoffatmosphäre unter Rühren zu einer Lösung von Lithiumdiisopropylamid (hergestellt aus 3.03 g Diisopropylamin und 18.75 ml einer 1,6M Lösung von n-Butyllithium in n-Hexan) in 75 ml Tetrahydrofuran gegeben. Das Reaktionsgemisch wird 30 Minuten gerührt und dann mit 6.99 g Aethyl 2-methyl-2-(4-cyanphenoxy)propionat in 15 ml Tetrahydrofuran versetzt. Man lässt das Reaktionsgemisch bis auf Raumtemperatur erwärmen, rührt es 1 Stunde und nimmt es danach in Aethylacetat und Wasser auf. Die organische Phase wird abgetrennt, über Natriumsulfat getrocknet und eingedampft. Der Rückstand wird an Kieselgel mit einem 1:4-Gemisch von Methanol und Aethylacetat als Eluierungsmittel chromatographiert, wobei man nach Umkristallisation aus Isopropanol 7.9 g 2-[3-(4-Cyanphenoxy)-3-methyl-2-oxobutyl]-6-methylpyridin-N-oxid erhält, Schmelzpunkt 127-129 ° C.

(B) 7.3 g 2-[3-(4-Cyanphenoxy)-3-methyl-2-oxobutyl]-6-methylpyridin-N-oxid in 160 ml Aethanol werden mit 0.96 g Natriumborhydrid versetzt, und das Gemisch 30 Minuten gerührt. Das Lösungsmittel wird abgedampft, und der Rückstand in Wasser und Dichlormethan aufgenommen. Die organische Phase wird abgetrennt, über Natriumsulfat getrocknet und eingedampft. Der Rückstand wird aus Diäthyläther kristallisiert und liefert 7.1 g 2-[3-(4-Cyanphenoxy)-2-hydroxy-3-methylbutyl]-6-methylpyridin-N-oxid, Schmelzpunkt 89-92 ° C.

(C) 573 mg Methansulfonylchlorid werden unter Rühren zu einer Lösung von 1.56 g 2-[3-(4-Cyanphenoxy)-2-hydroxy-3-methylbutyl]-6-methylpyridin-N-oxid in 3 ml Triäthylamin und 25 ml Dichlormethan bei Raumtemperatur gegeben. Nach 1 Stunde werden weitere 573 mg Methansulfonylchlorid zugegeben, und das Reaktionsgemisch so lange gerührt, bis die Reaktion nach dem Dünnschichtchromatogramm vollständig ist. Das Gemisch wird mit verdünnter Salzsäure und 2M Natriumhydroxidlösung gewaschen, über Natriumsulfat getrocknet und eingedampft, wobei man 1,8 g, 2-[3-(4-Cyanphenoxy)-2-methansulfonyloxy-3-methylbutyl]-6-methylpyridin-N-oxid erhält.

(D) 1.7 g 2-[3-(4-Cyanphenoxy)-2-methansulfonyloxy-3-methylbutyl]-6-methylpyridin-N-oxid werden zu einer Lösung von 200 mg 80%igem Natriumhydrid in 25 ml Isopropanol gegeben, und das Reaktionsgemisch 30 Minuten gerührt. Das Lösungsmittel wird abgedampft, und der Rückstand zwischen Wasser und Dichlormethan verteilt. Die organische Phase wird über Natriumsulfat getrocknet und eingedampft. Der Rückstand wird mit 10% (V/V) Methanol/Aethylacetat als Eluierungsmittel an Kieselgel chromatographiert, wobei man 960 mg 2-[3-(4-Cyanphenoxy)-3-methyl-1-butenyl]-6-methylpyridin-N-oxidals farblosen Gummi erhält

(E) 850 mg 2-[3-(4-Cyanphenoxy)-3-methyl-1-butenyl]-6-methylpyridin-N-oxid in 10 ml 1,2-Dichlorbenzol werden eine Stunde auf 150 °C erhitzt. Das Lösungsmittel wird abgedampft, wobei man einen Festkörper erhält, welcher mit Diäthyläther angerieben und abfiltriert wird. Auf diese Weise erhält man 600 mg 2-[1-(5-Cyan-2-hydroxyphenyl)-3-methyl-2-butenyl]-6-methylpyridin-N-oxid, das ohne weitere Reinigung in die nächste Stufe eingesetzt wird. Eine Probe wird aus Isopropanol umkristallisiert und schmiltzt dann bei 214-215 °C.

Beispiel 32

406 mg m-Chlorperbenzoesäure werden zu einer Lösung von 552 mg 2,2-Dimethyl-4-(4-methyl-2-pyridyl)-2H-1-benzopyran-6-carbonitril in 20 ml Dichlormethan gegeben, und das Gemisch 1 Stunde gerührt. Danach wird das Gemisch mit Natriumbicarbonatlösung gewaschen, über Natriumsulfat getrocknet und eingedampft. Der Rückstand wird mit einem 1:4-Gemisch von Methanol und Aethylacetat als Eluierungsmittel an Kieselgel chromatographiert. Nach Umkristallisation aus Aethylacetat werden 140 mg 2-(6-Cyan-2,2-dimethyl-2H-1-benzopyran-4-yl)-4-methylpyridin-N-oxid erhalten, Schmelzpunkt 199-210 °C.

Das als Ausgangsmaterial eingesetzte 2,2-Dimethyl-4-(4-methyl-2-pyridyl)-2H-1-benzopyran-6-carbonitril wurde in analoger Weise wie in Beispiel 6(A) beschrieben aus 2-Jod-4-methylpyridin und 4-(1,1-Dimethyl-2-propinyloxy)benzonitril hergestellt.

Beispiel 33

In analoger Weise wie im ersten Absatz von Beispiel 7 beschrieben wurde aus 3,4-Dihydro-2,2-dimethyl-4-(4-methyl-2-pyridyl)-2H-1-benzopyran-6-carbonitril das 2-(6-Cyan-3,4-dihydro-2,2-dimethyl-2H-1-benzopyran-4-yl)-4-methylpyridin-N-oxid erhalten, Schmelzpunkt 156-159 °C (aus Diäthyläther).

Das als Ausgangsmaterial eingesetzte 3,4-Dihydro-2,2-dimethyl-4-(4-methyl-2-pyridyl)-2H-1-benzopyran-6-carbonitril wurde in analoger Weise wie im letzen Absatz von Beispiel 7 beschrieben aus 2,2-Dimethyl-4-(4-methyl-2-pyridyl)-2H-1-benzopyran-6-carbonitril hergestellt.

Beispiel 34

1 g 2,2-Dimethyl-4-(5-methyl-2-pyridyl)-2H-1-benzopyran-6-carbonitril und 0.62 g m-Chlorperbenzoesäure werden in 30 ml Dichlormethan gelöst, und das Reaktionsgemisch 3 Stunden bei Raumtemperatur gerührt. Das Gemisch wird mit Natriumbicarbonatlösung gewaschen, über Natriumsulfat getrocknet und eingedampft. Der Rückstand wird an Kieselgel mit 20% (V/V) Methanol/Aethylacetat als Eluierungsmittel chromatographiert, wobei man nach Umkristallisation auch Aethylacetat 280 mg 2-(6-Cyan-2,2-dimethyl-2H-1-benzopyran-4-yl)-5-methylpyridin-N-oxid erhält, Schmelzpunkt 151-154 °C.

Das als Ausgangsmaterial eingesetzte 2,2-Dimethyl-4-(5-methyl-2-pyridyl)-2H-1-benzopyran-6-carbonitril wurde in analoger Weise wie im Beispiel 6(A) beschrieben aus 2-Jod-5-methylpyridin und 4-(1,1-Dimethyl-2-propinyloxy)benzonitril hergestellt.

Beispiel 35

In analoger Weise wie im ersten Absatz von Beispiel 7 beschrieben wurde aus 3,4-Dihydro-2,2-dimethyl-4-(5-methyl-2-pyridyl)-2H-1-benzopyran-6-carbonitril das 2-(6-Cyan-3,4-dihydro-2,2-dimethyl-2H-1-benzopyran-4-yl)-5-methylpyridin-N-oxid erhalten, Schmelzpunkt 151-153°C (aus Aethylacetat und Cyclohexan).

Das als Ausgangsmaterial eingesetzte 3,4-Dihydro-2,2-dimethyl-4-(5-methyl-2-pyridyl)-2H-1-benzopyran-6-carbonitril wurde in analoger Weise wie im letzten Absatz von Beispiel 7 beschrieben aus 2,2-Dimethyl-4-(5-methyl-2-pyridyl)-2H-1-benzopyran-6-carbonitril hergestellt.

Beispiel 36

237 mg Methyl 6-(6-cyan-3,4-dihydro-2,2-dimethyl-2H-1-benzopyran-4-yl)-3-pyridincarboxylat werden in 30 ml Dichlormethan gelöst und mit 180 mg m-Chlorperbenzoesäure versetzt. Das Gemisch wird über Nacht bei Raumtemperatur gerührt und danach nacheinander mit Natriumbisulfitlösung, Natriumbicarbonatlösung und Wasser gewaschen, über Natriumsulfat getrocknet und eingedampft. Das erhaltene Oel wird mit Diäthyläther angerieben, wobei man einen Festkörper erhält, welcher aus t-Butylmethyläther umkristallisiert wird. Auf diese Weise werden 60 mg 2-(6-Cyan-3,4-dihydro-2,2-dimethyl-2H-1-benzopyran-4-yl)-5-(methoxycarbonyl)pyridin-N-oxid erhalten, Schmelzpunkt 135-137°C.

Das als Ausgangsmaterial eingesetzte Methyl 6-(6-cyan-3,4-dihydro-2,2-dimethyl-2H-1-benzopyran-4-yl)-3-pyridincarboxylat wurde wie folgt hergestellt:

(A) 11 g 2-Jodpyridin-5-carbonsäure werden in 300 ml Dichlormethan suspendiert und mit 4.8 g Benzylalkohol, 10 g Dicyclohexylcarbodiimid und 100 mg 4-Dimethylaminopyridin versetzt. Das Reaktionsgemisch wird bei Raumtemperatur gerührt und nach 2 Stunden abfiltriert. Das Filtrat wird eingedampft, und der Rückstand an Kieselgel chromatographiert, wobei zunächst ein 1:9- und danach ein 1:6-Gemisch von Aethylacetat und Petroläther als Eluierungsmittel verwendet wird. Auf diese Weise erhält man 12.2 g Benzyl 2-jodpyridin-5-carboxylat, welches ohne weitere Reinigung in die nächste Stufe eingesetzt wird.

(B) 12.2 g Benzyl 2-jodpyridin-5-carboxylat und 5.64 g 4-(1,1-Dimethyl-2-propinyloxy)benzonitril werden 7 Tage bei Raumtemperatur mit 32 mg Kupfer(I)jodid, 162 mg Triphenylphosphin und 180 mg Palladium-(II)chlorid in 280 ml Diäthylamin unter einer Stickstoffatmosphäre gerührt. Das Reaktionsgemisch wird eingedampft, und der Rückstand in Aethylacetat und Wasser aufgenommen. Die organische Phase wird abgetrennt, über Natriumsulfat getrocknet und eingedampft. Der Rückstand wird mit einem 1:4-Gemisch von Aethylacetat und Petroläther als Eluierungsmittel chromatographiert, wobei man 9.04 g Benzyl 2-[3-(4-cyanphenoxy)-3-methyl-1-butin-1-yl]pyridin-5-carboxylat erhält, welches ohne weitere Reinigung in die nächste Stufe eingesetzt wird.

(C) 9 g Benzyl 2-[3-(4-cyanphenoxy)-3-methyl-1-butin-1-yl]pyridin-5-carboxylat werden in 300 ml 1,2-Dichlorbenzol gelöst, und die Lösung wird tropfenweise innerhalb von 5 Stunden unter Erhitzen zum Rückfluss zu 100 ml 1,2-Dichlorbenzol gegeben. Nach weiteren 2 Stunden lässt man das Reaktionsgemisch auf Raumtemperatur abkühlen und dampft ein. Der Rückstand wird an Kieselgel chromatographiert, wobei 1:9-, 1:6- und 1:4-Gemische von Aethylacetat und Petroläther verwendet werden. Auf diese Weise erhält man 5,5 g Benzyl 6-(6-cyan-2,2-dimethyl-2H-1-benzopyran-4-yl)-3-pyridincarboxylat, welches ohne weitere Reinigung in die nächste Stufe eingesetzt wird.

(D) 5.06 g Benzyl 6-(6-cyan-2,2-dimethyl-2H-1-benzopyran-4-yl)-3-pyridincarboxylat werden mit 7.8 ml Tributylamin, 244 mg 10% Palladium auf Kohle und 0.9 ml Ameisensäure auf 100°C erhitzt. Nach 12 Stunden werden weitere 7.8 ml Tributylamin und 10 ml Ameisensäure zugegeben. Nach 24 Stunden werden weitere 2 ml Tributylamin und 3 ml Ameisensäure zugegeben. Nach 26 Stunden werden weitere 170 mg 10% Palladium auf Kohle 2 ml Tributylamin und 3 ml Ameisensäure zugegeben. Nach 28 Stunden wird das Reaktionsgemisch eingedampft und abfiltriert. Das Filtrat wird eingedampft, und der Rückstand an Kieselgel mit einem 1:2-Gemisch von Aethylacetat und Petroläther und Aethylacetat als Eluierungsmittel chromatographiert. Auf diese Weise erhält man 1,4 g 6-(6-cyan-2,2-dimethyl-2H-1-benzopyran-4-yl)-3-pyridincarbonsäure, welche ohne weitere Reinigung in die nächste Stufe eingesetzt wird.

(E) 1.4 g 6-(6-Cyan-2,2-dimethyl-2H-1-benzopyran-4-yl)-3-pyridincarbonäure werden in 200 ml Aethylacetat gelöst und über Nacht unter einer Wasserstoffatmosphäre mit 103 mg 10% Palladium auf Kohle geschüttelt. Das Reaktionsgemisch wird filtriert, und das Filtrat eingedampft. Auf diese Weise werden 680 mg 6-(6-Cyan-2,4-dihydro-2,2-dimethyl-2H-1-benzopyran-4-yl)-3-pyridincarbonsäure erhalten, welche ohne weitere Reinigung in die nächste Stufe eingesetzt wird.

(F) 300 mg 6-(6-Cyan-3,4-dihydro-2,2-dimethyl-2H-1-benzopyran-4-yl)-3-pyridincarbonsäure in 10 ml Methanol werden so lange mit einer ätherischen Lösung von Diazomethan versetzt, bis die gelbe Farbe bestehen bleibt. Die gelbe Farbe wird durch die tropfenweise Zugabe von Essigsäure zum Verschwinden gebracht, und das Gemisch dann eingedampft. Der Rückstand wird in Aethylacetat gelöst, und die

Lösung mit Natriumbicarbonatlösung gewaschen, über Natriumsulfat getrocknet und eingedampft, wobei man nach Umkristallisation aus t-Butylmethyläther 270 mg Methyl 6-(6-Cyan-3,4-dihydro-2,2-dimethyl-2H-1-benzopyran-4-yl)-3-pyridincarboxylat erhält. Schmelzpunkt 131-133 °C.

Beispiel 37

1 g 4-(5-Amino-2-pyridyl)-2,2-dimethyl-2H-1-benzopyran-6-carbonitril wird in 15 ml Dichlormethan gelöst und mit 1 g m-Chlorperbenzoesäure versetzt. Nach 1-stündigem Rühren bei Raumtemperatur werden weitere 0.5 g m-Chlorperbenzoesäure zugegeben. Nach Rühren über Nacht bei Raumtemperatur wird das Reaktionsgemisch nacheinender iumbisulfitlösung. Natriumbicarbonatlösung und Wasser gewaschen, über Natriumsulfat getrocknet und eingedampft. Der Rückstand wird an Kieselgel mit zunächst 10% und dann 20% (V/V) Methanol/Aethylacetat als Eluierungsmittel chromatographiert. Das Produkt 5-Amino-2-(6-cyan-2,2-dimethyl-2H-1-benzopyran-4-yl)-pyridin wird in das Hydrochlorid übergeführt, welches aus Isopropanol umkristallisiert wird. Auf diese Weise werden 238 mg 5-Amino-2-(6-cyan-2,2-dimethyl-2H-1-benzopyran-4-yl)pyridin-N-oxid-hydrochlorid erhalten, Schmelzpunkt 235-237 °C.

Das als Ausgangsmaterial eingesetzte 4-(5-Amino-2-pyridyl)-2,2-dimethyl-2H-1-benzopyran-6-carbonitril wurde wie folgt hergestellt:

(A) 2,2-Dimethyl-4-(5-nitro-2-pyridyl)-2H-1-benzopyran-6-carbonitril wurde in analoger Weise wie in Beipiel 6(A) beschrieben aus 4-(1,1-Dimethyl-2-propinyloxy)benzonitril und 2-Jod-5-nitropyridin hergestellt.

(B) 1.91 g 2,2-Dimethyl-4-(5-nitro-2-pyridyl)-2H-1-benzopyran-6-carbonitril werden in 25 ml Essigsäure gelöst und mit 25 ml Wasser und 1.3 g Eisenpulver versetzt. Nach 1-stündigem Rühren bei 100 °C wird das Reaktionsgemisch in 2M Natriumhydroxidlösung gegossen, und das erhaltene Gemisch mit Aethylacetat extrahiert. Die organische Phase wird filtriert, und das Filtrat über Natriumsulfat getrocknet und eingedampft, wobei man nach Anreiben mit Diätyläther 600 mg 4-(5-Amino-2-pyridyl)-2,2-dimethyl-2H-1-benzopyran-6-carbonitril erhält, welches ohne weitere Reinigung weiter verwendet wird.

Beispiel 38

In analoger Weise wie im ersten Absatz von Beispiel 7 beschrieben wurde aus 4-(5-Amino-2-pyridyl)-3,4-dihydro-2,2-dimethyl-2H-1-benzopyran-6-carbonitril das 5-Amino-2-(6-cyan-3,4-dihydro-2,2-dimethyl-2H-1-benzopyran-4-yl)pyridin-N-oxid erhalten, Schmelzunkt 218-220 °C (aus Acetonitril).

Das als Ausgangsmaterial eingesetzte 4-(5-Amino-2-pyridyl)-3,4-dihydro-2,2-dimethyl-2H-1-benzopyran-6-carbonitril wurde in analoger Weise wie im letzten Absatz von Beispiel 7 beschrieben aus 2,2-Dimethyl-4-(5-nitro-2-pyridyl)-2H-1-benzopyran-6-carbonitril hergestellt.

Beispiel 39

400 mg 4-(5-Hydroxy-2-pyridyl)-2,2-dimethyl-2H-1-benzopyran-6-carbonitril und 350 mg m-Chlorperbenzoesäure werden über Nacht in 100 ml Dichlormethan gerührt. Das Reaktionsgemisch wird nacheinander mit Natriumbisulfitlösung, Natriumbicarbonatlösung und Wasser gewaschen, über Natriumsulfat getrocknet und eingedampft. Der Rückstand wird mit 10% (V/V) Methanol/ Aethylacetat als Eluierungsmittel chromatographiert, wobei man 30 mg 2-(6-Cyan-2,2-dimethyl-2H-1-benzopyran-4-yl)-5-hydroxypyridin-N-oxid erhält, Schmelzpunkt 260-262 °C (aus Aethanol).

Das als Ausgangsmaterial eingesetzte 4-(5-Hydroxy-2-pyridyl)-2,2-dimethyl-2H-1-benzopyran-6-carbonitril wurde wie folgt hergestellt:

1.62 g 4-(5-Amino-2-pyridyl)-2,2-dimethyl-2H-1-benzopyran-6-carbonitril werden in einem Gemisch von 12 ml konzentrierter Salzsäure, 30 g Eis und 18 ml N-Methylpyrrolidon suspendiert. Das Reaktionsgemisch wird auf -5 °C abgekühlt und mit 0.44 g Natriumnitrit in 5 ml Wasser tropfenweise versetzt, während man die Temperatur unterhalb 0 °C hält. Nach beendeter Zugabe wird das Reaktionsgemisch auf Raumtemperatur aufgewärmt und danach 1 Stunde auf 40 °C erhitzt. Das Gemisch wird mit Aethylacetat extrahiert, und der Extrakt eingedampft. Der Rückstand wird in Diäthyläther und Wasser aufgenommen, und die organische Phase abgetrennt, mit Wasser gewaschen, über Natriumsulfat getrocknet und eingedampft. Der Rückstand wird an Kieselgel mit einem 1:1-Gemisch von Aethylacetat und Petroläther und Aethylacetat als Eluierungsmittel chromatographiert, wobei man 270 mg 4-(5-Chlor-2-pyridyl)-2,2-dimethyl-2H-1-benzopyran-6-carbonitril und 254 mg 4-(5-Hydroxy-2-pyridyl)-2,2-dimethyl-2H-1-benzopyran-6-carbonitril erhält.

Beispiel 40

In analoger Weise wie im ersten Absatz von Beispiel 39 beschrieben wurde aus 4-(5-Chlor-2-pyridyl)-2,2-dimethyl-2H-1-benzopyran-6-carbonitril das 5-Chlor-2-(6-cyan-2,2-dimethyl-2H-1-benzopyran-4-yl)-pyridin-N-oxid als Oel erhalten. NMR (300 MHz, $CDCl_3$): δ 8.30 (1H, breit,s), 7.35 (1H, dd, 10Hz, 2Hz), 7.29 (1H, dd, 9Hz, 2Hz), 7.22 (1H, d, 10Hz), 6.87 (1H, d, 2Hz), 6.83 (1H, d, 10Hz), 5.82 (1H, s), 1.47 (6H, s). MS (EI): 3.12 ($H^+[Cl^{35}]$).

Beispiel 41

In analoger Weise wie im ersten Absatz von Beispiel 39 beschrieben wurde aus 4-(5-Chlor-2-pyridyl)-3,4-dihydro-2,2-dimethyl-2H-1-benzopyran-6-carbonitril das 5-Chlor-2-(6-cyan-3,4-dihydro-2,2-dimethyl-2H-1-benzopyran-4-yl)pyridin-N-oxid erhalten, Schmelzpunkt 159-161 °C (aus Aethanol).

Das als Ausgangsmaterial eingesetzte 4-(5-Chlor-2-pyridyl)-3,4-dihydro-2,2-dimethyl-2H-1-benzopyran-6-carbonitril wurde hergestellt, indem man in analoger Weise wie im letzten Absatz von Beispiel 7 beschrieben 2,2-Dimethyl-4-(5-nitro-2-pyridyl)-2H-1-benzopyran-6-carbonitril hydrierte und danach das erhaltene 4-(5-Amino-2-pyridyl)-3,4-dihydro-2,2-dimethyl-2H-1-benzopyran-6-carbonitril dem im letzten Absatz von Beispiel 39 beschriebenen Verfahren unterwarf.

Besipiel 42

In analoger Weise wie im ersten Absatz von Beispiel 6 beschrieben wurde aus 2,2-Dimethyl-4-(5-phenyl-2-pyridyl)-2H-1-benzopyran-6-carbonitril das 2-(6-Cyan-2,2-dimethyl-2H-1-benzopyran-4-yl)-5-phenylpyridin-N-oxid erhalten, Schmelzpunkt 173-175 °C (aus Aethylacetat).

Das als Ausgangsmaterial eingesetzte 2,2-Dimethyl-4-(5-phenyl-2-pyridyl)-2H-1-benzopyran-6-carbonitril wurde wie folgt hergestellt:

(A) 15.98 g 2-Chlor-5-phenylpyridin werden in 150 ml 55%iger wässriger Jodwasserstoffsäure gelöst, und die Lösung 2 Stunden zum Rückfluss erhitzt. Nach Abkühlen fällt ein Festkörper aus, der abfiltriert und mit Wasser gewaschen wird. Der Festkörper wird zwischen Diäthyläther und 2M Natriumhydroxidlösung verteilt, und die organische Phase wird über Natriumsulfat getrocknet und eingedampft, wobei man 15.87 g 2-Jod-5-phenylpyridin erhält, welches ohne weitere Reinigung weiter verwendet wird.

(B) In analoger Weise wie in Beispiel 6 (A) und (B) beschrieben wurde aus 2-Jod-5-phenylpyridin und 4-(1,1-Dimethyl-2-propinyloxy)benzonitril das 2,2-Dimethyl-4-(5-phenyl-2-pyridyl)-2H-1-benzopyran-6-carbonitril erhalten.

Beispiel 43

In analoger Weise wie im ersten Absatz von Beispiel 7 beschrieben wurde aus 3,4-Dihydro-2,2-dimethyl-4-(5-phenyl-2-pyridyl)-2H-1-benzopyran-6-carbonitril das 2-(6-Cyan-3,4-dihydro-2,2-dimethyl-2H-1-benzopyran-4-yl)-5-phenylpyridin-N-oxid erhalten, Schmelzpunkt 174-176 °C (aus Aethylacetat).

Das als Ausgangsmaterial eingesetzte 3,4-Dihydro-2,2-dimethyl-4-(5-phenyl-2-pyridyl)-2H-1-benzopyran-6-carbonitril wurde in analoger Weise wie im letzten Absatz von Beispiel 7 beschrieben durch katalytische Hydrierung von 2,2-Dimethyl-4-(5-phenyl-2-pyridyl)-2H-1-benzopyran-6-carbonitril erhalten.

Beispiel 44

In analoger Weise wie im ersten Absatz von Beispiel 3 beschrieben wurde aus 6-Acetyl-2,2-dimethyl-4-(2-pyridyl)-2H-1-benzopyran das 2-(6-Acetyl-2,2-dimethyl-2H-1-benzopyran-4-yl)pyridin-N-oxid erhalten. Schmelzpunkt 165-167 °C (aus Diäthyläther).

Das als Ausgangsmaterial eingesetzte 6-Acetyl-2,2-dimethyl-4-(2-pyridyl)-2H-1-benzopyran wurde in analoger Weise wie im letzten Absatz von Beispiel 3 beschrieben aus 2,2-Dimethyl-4-(2-pyridyl)-2H-1-benzopyran erhalten.

Beispiel 45

In analoger Weise wie im ersten Absatz von Beispiel 6 beschrieben wurde aus 6-Brom-2,2-dimethyl-4-(2-pyridyl)-2H-1-benzopyran das 2-(6-Brom-2,2-dimethyl-2H-1-benzopyran-4-yl)pyridin-N-oxid erhalten, Schmelzpunkt 146-148 °C (aus Aethylacetat).

Das als Ausgangsmaterial eingesetzte 6-Brom-2,2-dimethyl-4-(2-pyridyl)-2H-1-benzopyran wurde wie folgt hergestellt:

(A) 69.2 g p-Bromphenol und 33.6 g 2-Methylbut-3-in-2-ol werden in 600 ml Dichlormethan gelöst und mit 75 ml Diäthylazodicarboxylat versetzt. 126 g Triphenylphosphin werden portionsweise zugegeben, und das Reaktionsgemisch über Nacht gerührt. Das Gemisch wird mit verdünnter Salzsäure und 2M Natriumhydroxidlösung gewaschen, über Natriumsulfat getrocknet und eingedampft. Der Rückstand wird mit einem 1:10 Gemisch von Aethylacetat und Petroläther als Eluierungsmittel chromatographiert. Das so erhaltene Oel wird destilliert, wobei 14.4 g 4-(1,1-Dimethyl-2-propinyloxy)brombenzol erhalten werden, Siedepunkt 96-106 °C/1 mmHg.

(B) In analoger Weise wie im Beispiel 6 (A) beschrieben wurde aus 4-(1,1-Dimethyl-2-propinyloxy)-brombenzol und 2-Jodpyridin das 6-Brom-2,2-dimethyl-4-(2-pyridyl)-2H-1-benzopyran erhalten.

Beispiel 46

In analoger Weise wie im ersten Abstatz von Beispiel 6 beschrieben wurde aus 4-[5-(4-Methylphenyl)-2-pyridyl]-2,2-dimethyl-2H-1-benzopyran-6-carbonitril das 2-(6-Cyan-2,2-dimethyl-2H-1-benzopyran-4-yl)-5-(4-methylphenyl)pyridin-N-oxid erhalten, Schmelzpunkt 173-175 °C (Zers.; aus Aethylacetat).

Das als Ausgangsmaterial eingesetzte 4-[5-(4-Methylphenyl)-2-pyridyl]-2,2-dimethyl-2H-1-benzopyran-6-carbonitril wurde wie folgt hergestellt:

(A) 9.5 g 4-(5-Amino-2-pyridyl)-2,2-dimethyl-2H-1-benzopyran-6-carbonitril werden in 150 ml Essigsäure und 100 ml Wasser gelöst. 3.16 g Natriumnitrit in 10 ml Wasser werden in solchen Portionen zugegeben, dass die Temperatur unterhalb 5 °C gehalten werden kann. Nach 15 Minuten werden 23 g Kaliumjodid in 20 ml Wasser zugegeben, und das Gemisch 1 Stunde bei Raumtemperatur gerührt. Das Gemisch wird dann in 1 l 2M Natriumhydroxidlösung gegossen und mit Aethylacetat extrahiert. Der organische Extrakt wird über Natriumsulfat getrocknet und eingedampft. Der Rückstand wird mit einem 1:4-Gemisch von Aethyacetat und Petroläther als Eluierungsmittel chromatographiert, wobei man 4.78 g 4-(5-Jod-2-pyridyl)-2,2-dimethyl-2H-1-benzopyran -6-carbonitril erhält, welches ohne weitere Reinigung verwendet wird.

(B) 440 mg 4-(5-Jod-2-pyridyl)-2,2-dimethyl-2H-1-benzopyran-6-carbonitril, 386 mg p-Tolyltrimethylzinn, 144 mg Lithiumchlorid und 16 mg Bis(triphenylphosphin)palladiumdichlorid in 4 ml Dimethylformamid werden 2 Stunden auf 100 °C erhitzt. Nach Abkühlen auf Raumtemperatur werden 10%iges wässriges Ammoniak und Aethylacetat zugegeben, die beiden Phasen getrennt, und die wässrige Phase mit Aethylacetat rückextrahiert. Die vereinigten organischen Phasen werden über Natriumsulfat getrocknet und eingedampft. Der Rückstand wird an Kieselgel mit einem 1:4-Gemisch von Aethylacetat und Petroläther als Eluierungsmittel chromatographiert, wobei 268 mg 4-[5-(4-Methylphenyl)-2-pyridyl]-2,2-dimethyl-2H-1-benzopyran-6-carbonitril erhalten werden, welche ohne weitere Reinigung verwendet werden.

Beispiel 47

In analoger Weise wie im ersten Absatz von Beispiel 4 beschrieben wurde aus 6-Acetyl-2-methyl-2-phenyl-4-(2-pyridyl)-2H-1-benzopyran das 2-(6-Acetyl-2-methyl-2-phenyl-2H-1-benzopyran-4-yl)pyridin-N-oxid erhalten, Schmelzpunkt 192-194 °C (aus Acetonitril).

Das als Ausgangsmaterial eingesetzte 6-Acetyl-2-methyl-2-phenyl-4-(2-pyridyl)-2H-1-benzopyran wurde in analoger Weise wie in Beispiel 4 (B) bis (D) und (F) beschrieben aus 2-Methoxyphenyl-2-pyridylketon und 1-Brom-2-phenyl-1-propen hergestellt.

Beispiel 48

In analoger Weise wie im ersten Absatz von Beispiel 7 beschrieben wurde aus (-)-3,4-Dihydro-2,2-dimethyl-4-(2-pyridyl)-2H-1-benzopyran-6-carbonitril das (-)-2-(6-Cyan-3,4-dihydro-2,2-dimethyl-2H-1-benzopyran-4-yl)pyridin-N-oxid erhalten, Schmelzpunkt 142-144 °C (aus Diathyläther);

$$[\alpha]^{20}_{589} = -76.8°$$

(c = 0.997 in Aethanol).

Das als Ausgangsmaterial eingesetzte (-)-3,4-Dihydro-2,2-dimethyl-4-(2-pyridyl)-2H-1-benzopyran-6-carbonitril wurde wie folgt hergestellt:

(A) 36.15 g 3,4-Dihydro-2,2-dimethyl-4-(2-pyridyl)-2H-1-benzopyran-6-carbonsäure und 41.39 g Chinin werden in 650 ml Aethylacetat gelöst und zur Kristallisation stehen gelassen. 16.5 g Festkörper werden abfiltriert und in 250 ml Aethylacetat und 150 ml 2M Essigsäure gelöst. Die organische Phase wird mit 2M Essigsäure gewaschen. Die vereinigten wässrigen Phasen werden mit Aethylacetat gewaschen, und die organische Phase wird mit 25 ml 2% (G/V) Citronensäurelösung gewaschen. Die organischen Phasen werden vereinigt, mit Wasser gewaschen, über Natriumsulfat getrocknet und eingedampft, wobei 7.2 g (-)-3,4-Dihydro-2,2-dimethyl-4-(2-pyridyl)-2H-1-benzopyran-6-carbonsäure erhalten werden.

(B) 7.2 (-)-3,4-Dihydro-2,2-dimethyl-4-(2-pyridyl)-2H-1-benzopyran-6-carbonsäure werden in 30 ml Thionylchlorid 1 Stunde auf 70°C erhitzt, und das Reaktionsgemisch danach eingedampft. Der Rückstand wird in Toluol gelöst, und die Lösung eingedampft, um damit Spuren von Thionylchlorid zu entfernen. Der Rückstand wird in 100 ml Dichlormethan gelöst und unter Rühren mit 50 ml konzentriertem wässrigem Ammoniak versetzt. Danach wird 15 Minuten weitergerührt, die organische Phase danach abgetrennet, mit Wasser gewaschen, über Natriumsulfat getrocknet und eingedampft, wobei man 7.1 g (-)-3,4-Dihydro-2,2-dimethyl-4-(2-pyridyl)-2H-1-benzopyran-6-carboxamid erhält, welches ohne weitere Reinigung verwendet wird.

(C) 7.19 g (-)-3,4-Dihydro-2,2-dimethyl-4-(2-pyridyl)-2H-1-benzopyran-6-carboxamid werden in 25 ml Phosphoroxychlorid 30 Minuten auf 80°C erhitzt. Das Reaktionsgemisch wird dann abgekühlt und eingedampft. Der Rückstand wird in Dichlormethan und 2M Natriumcarbonatlösung gelöst. Die organische Phase wird abgetrennt, über Natriumsulfat getrocknet und eingedampft, wobei 6.65 g (-)-3,4-Dihydro-2,2-dimethyl-4-(2-pyridyl)-2H-1-benzopyran-6-carbonitril erhalten werden, welche ohne weitere Reinigung verwendet werden.

Beispiel 49

In analoger Weise wie im ersten Absatz von Beispiel 7 beschrieben wurde aus (+)-3,4-Dihydro-2,2-dimethyl-4-(2-pyridyl)-2H-1-benzopyran-6-carbonitril das (+)-2-(6-Cyan-3,4-dihydro-2,2-dimethyl-2H-1-benzopyran-4-yl)pyridin-N-oxid erhalten, Schmelzpunkt 143-144°C (aus Diäthyläther);

$$[\alpha]^{20}_{589} = +78.8°$$

(c = 1.001 in Aethanol).

Das als Ausgangsmaterial eingesetzte (+)-3,4-Dihydro-2,2-dimethyl-4-(2-pyridyl)-2H-1-benzopyran-6-carbonitril wurde aus den Mutterlaugen der in Beispiel 48 (A) beschriebenen Aufspaltung mit Chinin hergestellt. Die freie Säure wird in analoger Weise hergestellt. 4.01 g dieser Säure werden in 90 ml Aceton gelöst und mit 2.42 g (S)-(-)-1-(1-Naphthyläthylamin versetzt. Das ausgefallene Kristallisat wird abfiltriert und aus Dioxan unkristallisiert. Die Kristalle werden in Aethylacetat und 2% (G/V) Citronensäure gelöst. Die abgetrennte wässrige Phase wird mit Aethylacetat rückextrahiert, die organischen Phasen werden vereinigt, mit Wasser gewaschen, über Natriumsulfat getrocknet und eingedampft, wobei 1,41 g (+)-3,4-Dihydro-2,2-dimethyl-4-(2-pyridyl)-2H-1-benzopyran-6-carbonsäure erhalten werden. Diese Säure wird in analoger Weise wie in Beispiel 48 (B) und (C) beschrieben in das (+)-3,4-Dihydro-2,2-dimethyl-4-(2-pyridyl)-2H-1-benzopyran-6-carbonitril übergeführt.

Beispiel 50

2.24 g 5-Benzyloxy-2-[3-(4-cyanphenoxy)-3-methyl-1-butenyl]pyridin-N-oxid in 45 ml Toluol werden 12 Stunden auf 80°C erhitzt. Das Lösungsmittel wird abgedampft, und der Rückstand mit einem 97,5:2,5-Gemisch von Diäthyläther und Methanol als Eluierungsmittel an Kieselgel chromatographiert, wobei 0,45 g 5-Benzyloxy-2-(6-Cyan-3,4-dihydro-2,2-dimethyl-2H-1-benzopyran-4-yl)pyridin-N-oxid in Form eines weissen Festkörpers erhalten werden, Schmelzpunkt 224°C.

Das als Ausgangsmaterial eingesetzte 5-Benzyloxy-2-[3-(4-cyanphenoxy)-3-methyl-1-butenyl]pyridin-N-oxid wurde wie folgt hergestellt:

(A) 23.8 g 4-Cyanphenol in 150 ml Dimethylformamid werden tropfenweise unter Rühren zu einer Suspension von 6 g 80%igem Natriumhydrid in 100 ml Dimethylformamid gegeben, und das Gemisch danach 1 weitere Stunde gerührt. 39 g Aethyl bromisobutyrat werden tropfenweise zugegeben, und das Gemisch 76 Stunden auf 100°C erhitzt. Die Lösungsmittel werden abgedampft, und der Rückstand

zwischen Diäthyläther und Wasser verteilt. Die organische Phase wird nacheinander mit 2M Natriumhydroxidlösung und Natriumchloridlösung gewaschen und danach eingedampft, wobei 9.4 g Aethyl 2-(4-cyanphenoxy)-2-methylpropionat als farblose, visköse Flüssigkeit erhalten werden, Siedepunkt 115-117 °C/ 0.05 mmHg.

(B) 5 ml einer 1.2M Lösung von Butyllithium in n-Hexan werden unter Rühren bei -78 °C und unter einer Stickstoffatmosphäre zu einer Lösung von 0.6 g Diisopropylamin in 10 ml Tetrahydrofuran gegeben. Die Lösung wird weitere 15 Minuten gerührt, und dann werden 1.07 g 5-Benzyloxy-2-methylpyridin-N-oxid in 10 ml Tetrahydrofuran zugegeben. Das Reaktionsgemisch wird auf 20 °C erwärmt, 30 Minuten gerührt und dann auf -78 °C gekühlt. 1.16 g Aethyl 2-(4-cyanphenoxy)-2-methylpropionat werden zugegeben, und das Gemisch dann auf 20 °C erwärmt und 16 Stunden gerührt. Das Gemisch wird mit 50 ml Aethylacetat verdünnt und nacheinander mit Wasser und Natriumchloridlösung gewaschen. Die organische Phase wird abgedampft, und der Rückstand an Kieselgel mit einem 94:4-Gemisch von Dichlormethan und Methanol als Eluierungsmittel chromatographiert, wobei 0,46 g 5-Benzyloxy-2-[3-(4-cyanphenoxy)-3-methyl-2-oxobutyl]pyridin-N-oxid in Form eines schwach gelben Festkörpers erhalten werden, Schmelzpunkt 134-136 °C (aus Diäthyläther).

(C) 0.46 g Natriumborhydrid werden unter Rühren zu einer Lösung von 4.53 g 5-Benzyloxy-2-[3-(4-cyanphenoxy)-3-methyl-2-oxybutyl]pyridin-N-oxid in 60 ml Aethanol gegeben. Nach 2 Stunden wird die Lösung mit 200 ml Wasser verdünnt und mit Diäthyläther extrahiert. Die organische Phase wird eingedampft, wobei 3.67 g 5-Benzyloxy-2-[3-(4-cyanphenoxy)-2-hydroxy-3-methylbutyl]pyridin-N-oxid erhalten werden, welche nach Umkristallisation aus Aethanol einen Schmelzpunkt von 125 °C aufweisen.

(D) 0.7 g Methansulfonylchlorid werden unter Rühren zu einer Lösung von 2.3 g 5-Benzyloxy-2-[3-(4-cyanphenoxy)-2-hydroxy-3-methylbutyl]pyridin-N-oxid und 1 ml 2,6-Lutidin in 10 ml Dichlormethan gegeben. Das Reaktionsgemisch wird 4,5 Stunden gerührt, und dann werden 1 ml 2,6-Lutidin und 0.7 g Methansulfonylchlorid zugegeben. Nach 2.5 Stunden werden 1 ml 2,6-Lutidin und 0.7 g Methansulfonylchlorid zugegeben, und das Reaktionsgemisch 16 Stunden gerührt. Das Gemisch wird dann mit 50 ml Dichlormethan verdünnt und nacheinander mit 2M Salzsäure, Wasser und 10%iger Natriumbicarbonatlösung gewaschen. Die organische Lösung wird eingedampft, und der Rückstand an Kieselgel mit einem 9:1-Gemisch von Diäthyläther und Methanol als Eluierungsmittel chromatographiert, wobei 0.89 g 5-Benzyloxy-2-[3-(4-cyanphenoxy)-3-methyl-2-(methysulfonyloxy)butyl]pyridin-N-oxid als crèmefarbener Festkörper erhalten werden, welcher nach Umkristallisation aus Aethylacetat bei 42 °C schmilzt.

(E) 1.69 g 5-Benzyloxy-2-[3-(4-cyanphenoxy)-3-methyl-2-(methysulfonyloxy)butyl]pyridin-N-oxid werden zu einer Lösung von 0.14 g 80%igem Natriumhydrid in 15 ml Isopropanol gegeben, und die Lösung 16 Stunden bei 20 °C gerührt. Das Lösungsmittel wird dann abgedampft, und der Rückstand zwischen Aethylacetat und Natriumchloridlösung verteilt. Die organische Phase wird mit Natriumchloridlösung gewaschen und eingedampft, wobei 1.64 g 5-Benzyloxy-2-[3-(4-cyanphenoxy)-3-methyl-1-butenyl]-pyridin-N-oxid als crémefarbener Festkörper erhalten werden, welcher nach Umkristallisation aus Aethylacetat bei 125 °C schmilzt.

## Beispiel 51

0.21 g 5-Benzyloxy-2-(6-cyan-3,4-dihydro-2,2-dimethyl-2H-1-benzopyran-4-yl)pyridin-N-oxid in 90 ml Methanol werden bei Raumtemperatur und unter Atmosphärendruck 30 Minuten über 10% Palladium auf Kohle hydriert. Der Katalysator wird abfiltriert, und das Filtrat eingedampft. Der Rückstand wird mit einem 19:1-Gemisch von Dichlormethan und Methanol als Eluierungsmittel an Kieselgel chromatographiert, wobei 0.05 g 2-(6-Cyan-3,4-dihydro-2,2-dimethyl-2H-1-benzopyran-4-yl)-5-hydroxypyridin-N-oxid als weisslicher Festkörper erhalten werden, Schmelzpunkt 224 °C (aus Aethylacetat).

## Beispiel 52

In analoger Weise wie im ersten Absatz von Beispiel 7 beschrieben wurde aus rac-trans-3,4-Dihydro-3-hydroxy-2,2-dimethyl-4-(2-pyridyl)-2H-1-benzopyran-6-carbonitril das rac-trans-2-(6-Cyan-3,4-dihydro-3-hydroxy-2,2-dimethyl-2H-1-benzopyran-4-yl)pyridin-N-oxid erhalten, Schmelzpunkt 222-224 °C (aus Acetonitril).

Das als Ausgangsmaterial eingesetzte rac-trans-3,4-Dihydro-3-hydroxy-2,2-dimethyl-4-(2-pyridyl)-2H-1-benzopyran-6-carbonitril wurde wie folgt hergestellt:

(A) 3 g 2,2-Dimethyl-4-(2-pyridyl)-2H-1-benzpyran-6-carbonitril und 420 mg Natriumwolframat werden in 30 ml Methanol und 30 ml Acetonitril auf 50 °C erhitzt und mit 12 ml einer 30%igen (G/V) Wasserstoffperoxidlösung versetzt. Nach Erhitzen über Nacht wird das Gemisch eingedampft, und der Rückstand in

Dichlormethan und Wasser aufgenommen. Die organische Phase wird abgetrennt, über Natriumsulfat getrocknet und eingedampft. Der Rückstand wird an Kieselgel mit einem 1:3- und 1:2-Gemisch von Aethylacetat und Petroläther als Eluierungsmittel chromatographiert, wobei 440 mg 1a,7b-Dihydro-2,2-dimethyl-7b-(2-pyridyl)-2H-oxireno[c][1]benzopyran-6-carbonitril erhalten werden, welche ohne weitere Reinigung verwendet werden.

(B) 646 mg 1a,7b-Dihydro-2,2-dimethyl-7b-(2-pyridyl)-2H-oxireno[c][1]benzopyran-6-carbonitril werden in 100 ml Aethanol gelöst und mit 100 mg 10% Palladium auf Kohle versetzt. Das Gemisch wird über Nacht in einer Wasserstoffatmosphäre geschüttelt und dann filtriert. Das Filtrat wird eingedampft, und das verbleibende Oel zweimal an Kieselgel chromatographiert, wobei das Eluieren zunächst mit einem 1:3-Gemisch von Aethylacetat und Petroläther und dann mit 1% bis 2% (V/V) Methanol/Dichlormethan erfolgt. Auf diese Weise erhält man 66 mg rac-cis-3,4-Dihydro-3-hydroxy-2,2-dimethyl-4-(2-pyridyl)-2H-1-benzopyran-6-carbonitril, Schmelzpunkt 186-188 °C (aus Aetonitril) und 340 mg rac-trans-3,4-Dihydro-3-hydroxy-2,2-dimethyl-4-(2-pyridyl)-2H-1-benzopyran-6-carbonitril, Schmelzpunkt 175-176 °C (aus t-Butylmethyläther).

Beispiel 53

In analoger Weise wie im ersten Absatz von Beispiel 7 beschrieben wurde aus rac-cis-3,4-Dihydro-3-hydroxy-2,2-dimethyl-4-(2-pyridyl)-2H-1-benzopyran-6-carbonitril das rac-cis-2-(6-Cyan-3,4-dihydro-3-hydroxy-2,2-dimethyl-2H-1-benzopyran-4-yl)pyridin-N-oxid erhalten, Schmelzpunkt 215-216 °C (aus Acetonitril).

Beispiel 54

242 mg 3,4-Dihydro-2,2-dimethyl-4-(2-pyrimidinyl)-2H-1-benzopyran-6-carbonitril werden bei Raumtemperatur in 5 ml Dichlormethan gelöst und mit 450 mg m-Chlorperbenzoesäure versetzt. Nach Rühren über Nacht wird das Reaktionsgemisch mit Natriumbisulfitlösung und Natriumbicarbonatlösung gewaschen. Die organische Phase wird über Natriumsulfat getrocknet und eingedampft. Der Rückstand wird mit einem 40:4:1-Gemisch von Aethylacetat/Aethanol/Ameisensäure als Eluierungsmittel an Kieselgel chromatographiert. Das Produkt wird in Form eines Oels erhalten, welches in Diäthyläther gelöst und mit Natriumbicarbonatlösung gewaschen wird. Die organische Phase wird über Natriumsulfat getrocknet und eingedampft. Das verbleibende Oel wird aus t-Butylmethyläther kristallisiert, wobei man 25 mg 2-(6-Cyan-3,4-dihydro-2,2-dimethyl-2H-1-benzopyran-4-yl)pyrimidin-1-oxid erhält, Schmelzpunkt 98-100 °C.

Das als Ausgangsmaterial eingesetzte 3,4-Dihydro-2,2-dimethyl-4-(2-pyrimidinyl)-2H-1-benzopyran-6-carbonitril wurde wie folgt hergestellt:

(A) 0.63 g 4-(1,1-Dimethyl-2-propinyloxy)benzonitril, 0.75 g 2-Jodpyrimidin, 18 mg Triphenylphosphin, 12 mg Palladium(II)chlorid und 3.5 mg Kupfer(I)jodid werden in 20 ml Triäthylamin unter Stickstoff über Nacht gerührt. Das Gemisch wird zur Trockene eingedampft, und dann werden Aethylacetat und Wasser zugegeben. Die organische Phase wird über Natriumsulfat getrocknet und eingedampft. Der Rückstand wird an Kielgel mit einem 1:3- und danach mit einem 1:1-Gemisch von Aethylacetat und Petroläther als Eluierungsmittel chromatographiert, wobei 580 mg 4-[1,1-Dimethyl-3-(2-pyrimidinyl)-2-propinyloxy]-benzonitril als Oel erhalten werden.

(B) 580 mg 4-[1,1-Dimethyl-3-(2-pyrimidinyl)-2-propinyloxy]benzonitril werden in 20 ml Dichlorbenzol 3 Stunden zum Rückfluss erhitzt. Die Lösung wird dann abgekühlt und zur Trockene eingedampft. Der Rückstand wird zunächst mit einem 1:2 - und danach mit einem 1:1-Gemisch von Aethylacetat und Petroläther als Eluierungsmittel an Kieselgel chromatographiert. Dabei werden 361 mg 2,2-Dimethyl-4-(2-pyrimidinyl)-2H-1-benzopyran-6-carbonitril erhalten, welche nach Umkristallisation aus t-Butylmethyläther bei 108-109.5 °C schmelzen.

(C) 1.0 g 2,2-Dimethyl-4-(2-pyrimidinyl)-2H-1-benzopyran-6-carbonitril werden in 100 ml Aethanol gelöst, mit 10% Palladium auf Kohle versetzt und unter einer Wasserstoffatmosphäre bei Raumtemperatur geschüttelt. Nach 2 Stunden wird der Katalysator abfiltriert, und das Filtrat eingedampft. Der Rückstand wird an Kieselgel mit einem 1:1-Gemisch von Aethylacetat und Petroläther als Eluierungsmittel chromatographiert. Das Produkt wird aus Cyclohexan umkristallisiert, wobei 0.5 g 3,4-Dihydro-2,2-dimethyl-4-(2-pyrimidinyl)-2H-1-benzopyran-6-carbonitril erhalten werden, Schmelzpunkt 109-111 °C.

Beispiel 55

In analoger Weise wie im ersten Absatz von Beispiel 8 beschrieben wurde aus 0.22 g 6-[5-Cyan-2-hydroxy-α-(2-methylpropenyl)benzyl]pyrimidin-1-oxid 0.01 g 6-(6-Cyan-3,4-dihydro-2,2-dimethyl-2H-1-benzopyran-4-yl)pyrimidin-1-oxid als leicht weisslicher Festkörper erhalten (aus Aethylacetat/n-Hexan).

| Analyse für $C_{16}H_{15}N_3O_2$: | | | |
|---|---|---|---|
| Berechnet: | C: 68.31; | H: 5.37; | N: 14.94% |
| Gefunden : | C: 68.01; | H: 5.35; | N: 14.71%. |

Das als Ausgangsmaterial eingesetzte 6-[5-Cyan-2-hydroxy-α-(2-methylpropenyl)benzyl]pyrimidin-1-oxid wurde wie folgt hergestellt:

(A) 10 ml einer 1.2M Lösung von Butyllithium in n-Hexan werden unter Rühren bei -78°C und einer Stickstoffatmosphäre zu einer Lösung von 1.68 ml Diisopropylamin in 50 ml Tetrahydrofuran gegeben. Die Lösung wird 15 Minuten weitergerührt, und eine Lösung von 4-Methylpyrimidin in 20 ml Tetrahydrofuran wird zugegeben. Die Lösung wird auf 20°C erwärmt und 2 Stunden gerührt. Die Lösung wird dann auf -78°C gekühlt und mit 2.33 g Aethyl 2-(4-cyanphenoxy)-2-methylpropionat in 30 ml Tetrahydrofuran versetzt. Dann wird das Reaktionsgemisch auf 20°C erwärmt und 16 Stunden gerührt. Das Gemisch wird dann zwischen Aethylacetat und Wasser verteilt. Die organische Phase wird mit Natriumchloridlösung gewaschen und eingedampft. Der Rückstand wird mit Aethylacetat als Eluierungsmittel an Kieselgel chromatographiert, wobei 0.69 g 4-[2-Hydroxy-1,1-dimethyl-3-(4-pyrimidinyl)-2-propenyloxy]benzonitril als gelber Festkörper erhalten werden, Schmelzpunkt 115-117°C (aus Aethylacetat/n-Hexan).

(B) 0.013 mg Natriumborhydrid werden zu einer Lösung von 0.098 mg 4-[2-Hydroxy-1,1-dimethyl-3-(4-pyrimidinyl)-2-propenyloxy]benzonitril in 3 ml Aethanol gegeben, und die Lösung 16 Stunden bei 20°C gerührt. Das Lösungsmittel wird abgedampft, und der Rückstand zwischen Aethylacetat und Wasser verteilt. Die organische Phase wird eingedampft, und der Rückstand an Kieselgel mit Aethylacetat als Eluierungsmittel chromatographiert. Dabei erhält man 0.073 g 4-[2-Hydroxy-1,1-dimethyl-3-(4-pyrimidinyl)propoxy]benzonitril in Form eines weissen Festkörpers, Schmelzpunkt 96-98°C.

(C) 3.77 g m-Chloroperbenzoesäure werden zu einer Lösung von 4.78 g 4-[2-Hydroxy-1,1-dimethyl-3-(4-pyrimidinyl)propoxy]benzonitril in 75 ml Dichlormethan gegeben, und das Gemisch 16 Stunden bei 20°C gerührt. Weitere 0.38 g m-Chlorperbenzoesäure werden zugegeben, und das Gemisch weitere 24 Stunden bei 20°C gerührt. Das Gemisch wird dann nacheinander mit Wasser und Natriumchloridlösung gewaschen. Die organische Lösung wird eingedampft, und der Rückstand mit einem 9:1-Gemisch von Diäthyläther und Methanol an Kieselgel chromatographiert, wobei man 0.98 g 6-[3-(4-Cyanphenoxy)-2-hydroxy-3-methylbutyl]pyrimidin-1-oxid in Form eines weissen Festkörpers erhält, Schmelzpunkt 105-107.5°C (aus Aethylacetat).

(D) 0.23 g Methansulfonylchlorid werden zu einer Lösung von 0.59 g 6-[3-(4-Cyanphenoxy)-2-hydroxy-3-methylbutyl]pyrimidin-1-oxid und 0.2 g Triäthylamin in 10 ml Dichlormethan gegeben. Das Gemisch wird 2 Stunden bei 20°C gerührt, und dann werden weitere 0.46 g Methansulfonylchlorid und 0.4 g Triäthylamin zugegeben. Nach 2 Stunden werden weitere 0.6 g Triäthylamin zugegeben, und das Gemisch 1,5 Stunden gerührt. Das Gemisch wird dann mit Wasser gewaschen und eingedampft. Der Rückstand wird an Kieselgel mit Aethylacetat als Eluierungsmittel chromatographiert, wobei 0.25 g (E)-6-[3-(4-Cyanphenoxy)-3-methyl-1-butenyl]pyrimidin-1-oxid in Form eines leicht gelblichen Festkörpers erhalten werden, Schmelzpunkt 101-102°C.

(E) 0.24 g (E)-6-[3-(4-Cyanphenoxy)-3-methyl-1-butenyl]pyrimidin-1-oxid in 30 ml Toluol werden 16 Stunden zum Rückfluss erhitzt. Das Lösungsmittel wird dann abgedampft, und der Rückstand mit Aethylacetat als Eluierungsmittel an Kieselgel chromatographiert. Dabei werden 0.08 g 6-[5-Cyan-2-hydroxy-α-(2-methylpropenyl)benzyl]pyrimidin-1-oxid in Form eines weissen Festkörpers erhalten, Schmelzpunkt 190-192°C (aus Aethylacetat).

Beispiel 56

In analoger Weise wie im ersten Abatz von Beispiel 8 beschrieben wurden aus 0.35 g 6-[5-Cyan-2-hydroxy-α-(2-methylpropenyl)benzyl]pyrazin-1-oxid 0.02 g 2-(6-Cyan-3,4-dihydro-2,2-dimethyl-2H-1-benzopyran-4-yl)pyrazin-1-oxid in Form eines weissen Festkörpers erhalten, Schmelzpunkt 166-168°C (aus Diäthyläther).

Das als Ausgangsmaterial eingesetzte 6-[5-Cyan-2-hydroxy-α-(2-methylpropenyl)benzyl]pyrazin-1-oxid wurde wie folgt hergestellt:

(A) 10 ml einer 1.2M Lösung von Butyllithium in n-Hexan werden unter Rühren bei -78°C und unter einer Stickstoffatmosphäre zu einer Lösung von 1,68 ml Diisopropylamin in 10 ml Tetrahydrofuran gegeben. Die Lösung wird weitere 15 Minuten gerührt und dann mit 0.94 g 2-Methylpyrazin in 20 ml Tetrahydrofuran versetzt. Dann lässt man die Lösung auf 20°C erwärmen und rührt 1 Stunde. Man kühlt die Lösung dann auf -78°C, versetzt sie mit 2.33 g Aethyl 2-(4-cyanphenoxy)-2-methylpropionat in 30 ml Tetrahydrofuran und lässt das Reaktionsgemisch auf 20°C erwärmen. Das Gemisch wird mit 20 ml Wasser behandelt, und das Lösungsmittel abgedampft. Der Rückstand wird mit Aethylacetat als Eluierungsmittel an Kieselgel chromatographiert, wobei man 1.37 g 4-[1,1-Dimethyl-2-oxo-3-(2-pyrazinyl)-propoxy]benzonitril erhält.

(B) 0.107 g Natriumborhydrid werden unter Rühren zu einer Suspension von 0.79 g 4-[1,1-Dimethyl-2-oxo-3-(2-pyrazinyl)propoxy]benzonitril in 20 ml Aethanol gegeben. Das Reaktionsgemisch wird 1,5 Stunden bei 20°C gerührt und danach mit Wasser verdünnt. Die Lösung wird mit Aethylacetat extrahiert, und die organische Phase danach mit Natriumchloridlösung gewaschen und eingedampft, wobei man 0.5 g 4-[2-Hydroxy-1,1-dimethyl-3-(2-pyrazinyl)propoxy]benzonitril als weissen Festkörper erhält, Schmelzpunkt 94-95°C (aus Diäthyläther).

(C) 0.18 g m-Chlorperbenzoesäure werden unter Rühren zu einer Lösung von 0.28 g 4-[2-Hydroxy-1,1-dimethyl-3-(2-pyrazinyl)propoxy]benzonitril in 10 ml Dichlormethan gegeben. Nach 16 Stunden werden weitere 36 mg m-Chlorperbenzoesäure zugegeben, und das Gemisch 24 Stunden gerührt. Die Lösungsmittel werden dann abgedampft, und der Rückstand mit einem 95:5-Gemisch von Diäthyläther und Methanol als Eluierungsmittel an Kieselgel chromatographiert, wobei 0,09 g 2-[3-(4-Cyanphenoxy)-2-hydroxy-3-methylbutyl]pyrazin-1-oxid als weisser Festkörper erhalten werden, Schmelzpunkt 103-104°C (aus Diäthyläther).

(D) 0.32 g Methansulfonylchlorid werden unter Rühren zu einer Lösung von 0.84 g 2-[3-(4-Cyanphenoxy)-2-hydroxy-3-methylbutyl]pyrazin-1-oxid in 5 ml Pyridin gegeben. Das Reaktionsgemisch wird 5 Stunden bei 20°C gerührt, und danach werden weitere 0.32 g Methansulfonylchlorid zugegeben. Das Gemisch wird dann 70 Stunden bei 20°C gerührt und dann in 2M Salzsäure gegossen. Die Lösung wird mit Dichlormethan extrahiert, und die organische Phase mit Natriumchloridlösung gewaschen und eingedampft. Der Rückstand wird mit n-Hexan angerieben, wobei 0.95 g 2-[2-(4-Cyanphenoxy)-2-methyl-3-(methylsulfonyl)propyl]pyrazin-1-oxid als weisser Festkörper erhalten werden, Schmelzpunkt 146-147°C.

(E) 0.89 g 2-[2-(4-Cyanphenoxy)-2-methyl-3-(methylsulfonyl)propyl]pyrazin-1-oxid werden zu einer Lösung von 71 mg 80%igem Natriumhydrid in 5 ml Isopropanol gegeben, und das Gemisch 16 Stunden bei 20°C gerührt. Das Lösungsmittel wird abgedampft, und der Rückstand zwischen Wasser und Aethylacetat verteilt. Die organische Phase wird mit Natriumchloridlösung gewaschen und eingedampft. Der Rückstand wird an Kieselgel mit einem 4:1-Gemisch von Aethylacetat und n-Hexan als Eluierungsmittel chromatographiert, wobei man 0.5 g (E)-2-[2-(4-Cyanphenoxy)-2-methyl-1-butenyl]pyrazin-1-oxid als weissen Festkörper erhält, Schmelzpunkt 107-109°C.

(F) 0.47 g (E)-2-[2-(4-Cyanphenoxy)-2-methyl-1-butenyl]pyrazin-1-oxid in 10 ml Toluol werden 16 Stunden zum Rückfluss erhitzt. Das abgekühlte Reaktionsgemisch wird dann filtriert, wobei man 0.4 g 6-[5-Cyan-2-hydroxy-α-(2-methylpropenyl)benzyl]pyrazin-1-oxid als schwach bräunlichen Festkörper erhält, Schmelzpunkt 212-213°C.

Beispiel 57

450 mg m-Chlorperbenzoesäure werden bei Raumtemperatur zu einer Lösung von 624 mg 2,2-Dimethyl-4-(2-chinolyl)-2H-1-benzopyran -6-carbonitril in 20 ml Dichlormethan gegeben. Nach 6-stündigem Rühren bei Raumtemperatur wird das Reaktionsgemisch mit Natriumbicarbonatlösung gewaschen, und die organische Phase über Natriumsulfat getrocknet und eingedampft. Der Rückstand wird ein erstes Mal mit einem 4:1-Gemisch von Aethylacetat und Petroläther als Eluierungsmittel und dann ein zweites Mal mit einem 1:9-Gemisch von Methanol und Aethylacetat als Eluierungsmittel an Kieselgel chromatographiert. Kristallisation des Produkts aus Toluol liefert 45 mg 2-(6-Cyan-2,2-dimethyl-2H-1-benzopyran-4-yl)chinolin-1-oxid, Schmelzpunkt 177°C.

Das als Ausgangsmaterial eingesetzte 2,2-Dimethyl-4-(2-chinolyl)-2H-1-benzopyran -6-carbonitril wurde wie folgt hergestellt:

(A) 5,1 g 2-Jodchinolin werden bei Raumtemperatur zu einer Lösung von 18 mg Palladium(II)chlorid, 52 mg Triphenylphosphin und 38 mg Kupfer(I)jodid in 100 ml Diäthylamin gegeben. 3,7 g 4-(1,1-Dimethyl-2-propinyloxy)benzonitril werden zugegeben. Nach 18-stündigem Rühren bei Raumtemperatur wird das Reaktionsgemisch eingedampft, und der Rückstand zwischen Aethylacetat und Wasser verteilt. Die organische Phase wird über Natriumsulfat getrocknet und eingedampft. Der Rückstand wird an Kieselgel mit einem 1:2-Gemisch von Aethylacetat und Petroläther als Eluierungsmittel chromatographiert und liefert 5,8 g 4-[1,1-Dimethyl-3-(2-chinolyl)-2-propinyloxy]benzonitril als gelben Gummi.

(B) 5,8 g 4-[1,1-Dimethyl-3-(2-chinolyl)-2-propinyloxy]benzonitril werden in 50 ml Dichlorbenzol 2 Stunden zum Rückfluss erhitzt. Dann lässt man das Reaktionsgemisch auf Raumtemperatur abkühlen und dampft es zur Trockene ein. Der Rückstand wird an Kieselgel mit einem 1:3-Gemisch von Aethylacetat und Petroläther als Eluierungsmittel chromatographiert. Umkristallisation des Produkts aus Isopropanol liefert 2,1 g 2,2-Dimethyl-4-(2-chinolyl)-2H-1-benzopyran -6-carbonitril, Schmelzpunkt 102-104 °C.

Beispiel 58

203 mg m-Chlorperbenzoesäure werden bei Raumtemperatur zu einer Lösung von 314 mg 3,4-Dihydro-2,2-dimethyl-4-(2-chinolyl)-2H-1-benzopyran-6-carbonitril in 10 ml Dichlormethan gegeben. Nach 2-stündigem Rühren wird das Reaktionsgemisch mit Natriumbisulfitlösung und Natriumbicarbonatlösung gewaschen. Die organische Phase wird über Natriumsulfat getrocknet und eingedampft. Umkristallisation des Rückstands aus Diäthyläther liefert 230 mg 2-(6-Cyan-3,4-dihydro-2,2-dimethyl-2H-1-benzopyran-4-yl)-chino lin-1-oxid, Schmelzpunkt 183-185 °C.

Das als Ausgangsmaterial eingesetzte 3,4-Dihydro-2,2-dimethyl-4-(2-chinolyl)-2H-1-benzopyran-6-carbonitril wurde wie folgt hergestellt:

624 mg 2,2-Dimethyl-4-(2-chinolyl)-2H-1-benzopyran-6-carbonitril werden bei Raumtemperatur in 25 ml Aethanol mit 50 mg 10% Palladium auf Kohle unter einer Wasserstoffatmosphäre geschüttelt. Nach 4 Stunden wird das Reaktionsgemisch abfiltriert, und das Filtrat eingedampft. Der Rückstand wird mit einem 1:2-Gemisch von Aethylacetat und Petroläther als Eluierungsmittel an Kieselgel chromatographiert. Umkristallisation des Produkts aus Isopropanol liefert 410 mg 3,4-Dihydro-2,2-dimethyl-4-(2-chinolyl)-2H-1-benzopyran-6-carbonitril, Schmelzpunkt 174-176 °C.

Beispiel 59

1,02 g 4-(2-Methoxyphenyl)-2,2-dimethyl-2H-1-benzopyran-6-carbonitril und 736 mg Natriummethanthiolat werden in 10 ml Dimethylformamid unter einer Stickstoffatmosphäre 1,5 Stunden zum Rückfluss erhitzt und danach in ein Gemisch von Diäthyläther und Wasser gegossen. Die organische Phase wird abgetrennt, über Natriumsulfat getrocknet und eingedampft. Der Rückstand wird mit einem 3:1-Gemisch von Petroläther und Aethylacetat als Eluierungsmittel an Kieselgel chromatographiert. Umkristallisation des erhaltenen Festkörpers aus Diäthyläther/Petroläther liefert 210 mg 4-(2-Hydroxyphenyl)-2,2-dimethyl-2H-1-benzopyran-6-carbonitril, Schmelzpunkt 139-140 °C.

Das als Ausgangsmaterial eingesetzte 4-(2-Methoxyphenyl)-2,2-dimethyl-2H-1-benzopyran-6-carbonitril wurde wie folgt hergestellt:

(A) 2,34 g 2-Jodanisol werden bei Raumtemperatur zu einer Lösung von 9 mg Palladium(II)chlorid, 26 mg Triphenylphosphin und 19 ml Kupfer(I)jodid in 25 ml Diäthylamin gegeben. Dieses Gemisch wird mit 1,85 g 4-(1,1-Dimethyl-2-propinyloxy)benzonitril versetzt, und das erhaltene Gemisch 48 Stunden gerührt. Das Reaktionsgemisch wird eingedampft, und der Rückstand in einem Gemisch von Aethylacetat und Wasser verteilt. Die organische Phase wird abgetrennt, über Natriumsulfat getrocknet und eingedampft. Chromatographie des Rückstands an Kieselgel mit einem 1:4-Gemisch von Aethylacetat und Petroläther als Eluierungsmittel liefert 0,99 g 4-[1,1-Dimethyl-3-(2-methoxyphenyl)-2-propinyloxy]-benzonitril als Oel.

(B) 0,99 g 4-[1,1-Dimethyl-4-(2-methoxyphenyl)-2-propinyloxy]benzonitril werden in 10 ml 1,2-Dichlorbenzol gelöst und 1,5 Stunden zum Rückfluss erhitzt. Nach dem Abkühlen wird die Lösung eingedampft, und der Rückstand an Kieselgel mit einem 1:4-Gemisch von Aethylacetat und Petroläther als Eluierungsmittel chromatographiert. Umkristallisation aus Petroläther (Siedepunkt 60-80 °C), liefert 480 mg 4-(2-Methoxyphenyl)-2,2-dimethyl-2H-1-benzopyran-6-carbonitril, Schmelzpunkt 109-111 °C.

Beispiel 60

1,58 g 4-(4-Cyan-2-methoxyphenyl)-2,2-dimethyl-2H-1-benzopyran-6-carbonitril und 1,05 g Natriummethanthiolat werden 20 Minuten in 15 ml Dimethylformamid auf 100 °C erhitzt. Man lässt danach das Reaktionsgemisch auf Raumtemperatur abkühlen und giesst es in Wasser und Diäthyläther. Die wässrige

Phase wird mit 2M wässriger Salzsäure angesäuert und mit Aethylacetat extrahiert. Der organische Extrakt wird dreimal mit Wasser gewaschen, über Natriumsulfat getrocknet und eingedampft. Der Rückstand wird mit einem 2:3-Gemisch von Aethylacetat und Petroläther als Eluierungsmittel an Kieselgel chromatographiert. Umkristallisation des Produkts aus Toluol liefert 1,1g 14-(5-Cyan-2-hydroxyphenyl)-2,2-dimethyl-2H-1-benzopyran-6-carbonitril, Schmelzpunkt 213-215 ° C.

Das als Ausgangsmaterial eingesetzte 4-(4-Cyan-2-methoxyphenyl)-2,2-dimethyl-2H-1-benzopyran-6-carbonitril wurde wie folgt hergestellt:

(A) 5,18 g 3-Jod-4-methoxybenzonitril werden bei Raumtemperatur zu einer Lösung von 18 mg Palladium(II)chlorid, 52 mg Triphenylphosphin und 38 mg Kupfer(I)jodid in 100 ml Diäthylamin gegeben. 3,7 g 4-(1,1-Dimethyl-2-propinyloxy)benzonitril werden zugegeben, und das Reaktionsgemisch unter einer Stickstoffatmosphäre 2 Tage gerührt. Das Gemisch wird dann eingedampft, und der Rückstand zwischen Aethylacetat und Wasser verteilt. Die organische Phase wird über Natriumsulfat getrocknet und eingedampft. Chromatographie des Rückstands an Kieselgel mit einem 1:2-Gemisch von Aethylacetat und Petroläther als Eluierungsmittel liefert 4,8 g 3-[3-(4-Cyanphenoxy)-3-methyl-1-butin-1-yloxy]-4-methoxybenzonitril als Festkörper, welches ohne weitere Reinigung verwendet wird.

(B) 4,8 g 3-[3-[(4-Cyanphenoxy)-3-methyl-1-butin-1-yloxy]-4-methoxybenzonitril werden in 50 ml 1,2-Dichlorbenzol 2,5 Stunden zum Rückfluss erhitzt. Dann lässt man das Reaktionsgemisch auf Raumtemperatur abkühlen, dampft es ein, und chromatographiert den Rückstand mit einem 1:2-Gemisch von Aethylacetat und Petroläther als Eluierungsmittel an Kieselgel. Umkristallisation des Produkts aus Isopropanol liefert 3,8 g 4-(4-Cyan-2-methoxyphenyl)-2,2-dimethyl-2H-1-benzopyran-6-carbonitril, Schmelzpunkt 137-139 ° C.

Beispiel 61

400 mg 3-(6-Cyan-3,4-dihydro-2,2-dimethyl-2H-1-benzopyran-4-yl)pyridin-N-oxid werden in 10 ml Essigsäureanhydrid gelöst und 8 Stunden zum Rückfluss erhitzt. Nach Abdampfen des Lösungsmittels wird der Rückstand in Aethanol gelöst und mit 15 mg 80%-igem (G/G) Natriumhydrid versetzt. Nach 30-minütigem Rühren wird das Reaktionsgemisch eingedampft, und der Rückstand in einem Gemisch von Aethylacetat und Wasser gelöst. Die wässrige Phase wird mit verdünnter Salzsäure angesäuert, mit Aethylacetat extrahiert, dann mit Nariumbicarbonatlösung basisch gestellt und mit Aethylacetat rückextrahiert. Die vereinigten organischen Extrakte werden über Natriumsulfat getrocknet und eingedampft. Chromatographie des Rückstands an Kieslgel mit 8% (V/V) Methanol/Aethylacetat als Eluierungsmittel liefert 310 mg 3-(6-Cyan-3,4-dihydro-2,2-dimethyl-2H-1-benzopyran-4-yl)-2(1H)-pyridon, Schmelzpunkt 196-197 ° C nach Umkristallisation aus t-Butylmethyläther.

Das als Ausgangsmaterial eingesetzte 3-(6-Cyan-3,4-dihydro-2,2-dimethyl-2H-1-benzopyran-4-yl)pyridin-N-oxid wurde wie folgt hergestellt:

(A) 10,25 g 3-Jodpyridin werden bei Raumtemperatur zu einer Lösung von 44 mg Palladium(II)chlorid, 131 mg Triphenylphosphin und 95 mg Kupfer(I)jodid in 200 mg Diäthylamin gegeben. 9,25 g 4-(1,1-Dimethyl-2-propinyloxy)benzonitril werden zugegeben, und das Reaktionsgemisch 3 Tage gerührt. Das Gemisch wird dann eingedampft, und der Rückstand zwischen Aethylacetat und Wasser verteilt. Die organische Phase wird über Natriumsulfat getrocknet und eingedampft. Chromatographie des Rückstands an Kieselgel mit einem 11:9-Gemisch von Aethylacetat und Petroläther als Eluierungsmittel liefert 11,8 g 4-[1,1-Dimethyl-3-(3-pyridyl)-2-propinyloxy]benzonitril als Oel.

(B) 11,8 g 4-[1,1-Dimethyl-3-(3-pyridyl)-2-propinyloxy]benzonitril werden in 75 ml 1,2-Dichlorbenzol 3,5 Stunden zum Rückfluss erhitzt. Dann lässt man das Reaktionsgemisch auf Raumtemperatur abkühlen und dampft es ein. Der Rückstand wird mit Aethylacetat als Eluierungsmittel an Kieselgel chromatographiert. Umkristallisation aus Cyclohexan liefert 6,7 g 2,2-Dimethyl-4-(3-pyridyl)-2H-1-benzopyran-6-carbonitril, Schmelzpunkt 98-99 ° C.

(C) 2,1 g 2,2-Dimethyl-4-(3-pyridyl)-2H-1-benzopyran-6-carbonitril werden in 50 ml Aethylacetat gelöst und unter einer Wasserstoffatmosphäre bei Raumtemperatur mit 50 mg 10% Palladium auf Kohle für insgesamt 30 Stunden geschüttelt, wobei der Katalysator mehrmals gewechselt wird. Das Gemisch wird dann abfiltriert, und das Filtrat eingedampft. Chromatographie des Rückstands an Kieselgel mit Aethylacetat als Eluierungsmittel liefert 910 mg 3,4-Dihydro-2,2-dimethyl-4-(3-pyridyl)-2H-1-benzopyran-6-carbonitril als Oel.

(D) 610 mg m-Chlorperbenzosäure werden zu einer Lösung von 792 mg 3,4-Dihydro-2,2-dimethyl-4-(3-pyridyl)-2H-1-benzopyran-6-carbonitril in 15 ml Dichlormethan gegeben. Nach 1-stündigem Rühren bei Raumtemperatur wird das Reaktionsgemisch mit Natriumbisulfitlösung und Natriumbicarbonatlösung gewaschen. Die organische Phase wird über Natriumsulfat getrocknet und eingedampft. Kristallisation

des Rückstands aus t-Butylmethyläther liefert 710 mg 3-(6-Cyan-3,4-dihydro-2,2-dimethyl-2H-1-benzopyran-4-yl)pyridin-N-oxid als Festkörper, Schmelzpunkt 134-137 °C.

Beispiel 62

0,95 g Aluminiumchlorid werden bei 0 °C zu 0,6 g 4-(2,2-Dimethyl-2H-1-benzopyran-4-yl)-3-pyridinol in 50 ml Nitromethan gegeben. Das Reaktionsgemisch wird 5 Minuten gerührt und danach bei 0 °C mit 0,51 ml Acetylchlorid versetzt. Dann lässt man 15 Minuten bei 0 °C und 15 Minuten bei 20 °C weiterrühren, dampft das Lösungsmittel ab und verteilt den Rückstand zwischen Aethylacetat und verdünnter wässriger Natriumhydroxidlösung. Die organische Phase wird über Natriumsulfat getrocknet und eingedampft. Der Rückstand wird in Aethanol gelöst und mit 0,1 g einer 60%-igen Suspension von Natriumhydrid in Mineralöl versetzt. Nach 20-minütigem Rühren wird das Lösungsmittel abgedampft, und der Rückstand zwischen Wasser und Aethylacetat verteilt. Die wässrige Phase wird mit Essigsäure angesäuert und mit Aethylacetat extrahiert. Der organische Extrakt wird getrocknet und zu 270 mg eines Oels eingedampft. Dieses Oel wird in äthanolischem Chlorwasserstoff gelöst, und die Lösung eingedampft. Umkristallisation des Rückstands aus Isopropanol liefert 4-(6-Acetyl-2,2-dimethyl-2H-1-benzopyran-4-yl)-3-pyridinol-hydrochlorid, Schmelzpunkt 229-331 °C (Zers.).

Beispiel 63

0,66 g N,N,N',N'-Tetramethyläthylendiamin werden zu 30 ml Tetrahydrofuran gegeben, und das Gemisch auf -78 °C gekühlt. 2,3 ml einer 2,5M Lösung von n-Butyllithium in n-Hexan werden zugegeben, das Reaktionsgemisch 10 Minuten bei -78 °C gerührt und dann mit 1 g 3-(N,N-Diäthylcarbamoyloxy)pyridin in 5 ml Tetrahydrofuran versetzt. Nach weiteren 50 Minuten bei -78 °C wird 1 g 2,2-Dimethylchromanon in 5 ml Tetrahydrofuran unter Rühren tropfenweise zugegeben. Man hält das Reaktionsgemisch 1 Stunde bei -78 °C und lässt es dann auf Raumtemperatur erwärmen. Das Lösungsmittel wird abgedampft, und der Rückstand mit Wasser und Aethylacetat geschüttelt. Die organische Phase wird über Natriumsulfat getrocknet und eingedampft. Chromatographie des Rückstands an Kieselgel mit einem 1:1-Gemisch von Aceton und Petroläther als Eluierungsmittel liefert 60 mg 4-(2,2-Dimethyl-2H-1-benzopyran-4-yl) -3-pyridinol, Schmelzpunkt 228-230 °C nach Umkristallisation aus Isopropanol.

Die folgenden Beispiele illustrieren typische pharmazeutische Präparate, enthaltend eine erfindungsgemässe Verbindung:

Beispiel A

Tabletten, enthaltend die folgenden Bestandteile, können in herkömmlicher Weise hergestellt werden:

| Bestandteile | pro Tablette |
|---|---|
| Verbindung der Formel I | 5 mg |
| Lactose | 125 mg |
| Maisstärke | 65 mg |
| Talk | 4 mg |
| Magnesiumstearat | 1 mg |
| Tablettengewicht | 200 mg |

Beispiel B

Kapseln, enthaltend die folgenden Bestandteile, können in herkömmlicher Weise hergestellt werden:

| Bestandteile | pro Kapsel |
|---|---|
| Verbindung der Formel I | 10 mg |
| Lactose | 165 mg |
| Maisstärke | 20 mg |
| Talk | 5 mg |
| Kapselfüllgewicht | 200 mg |

**Patentansprüche**
**Patentansprüche für folgende Vertragsstaaten : AT, BE, CH, DE, FR, GB, IT, LI, LU, NL, SE**

**1.** Verbindungen der allgemeinen Formel

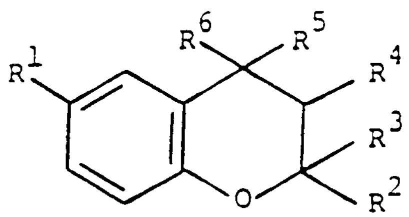

I

worin $R^1$ Wasserstoff, Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl, $R^4$ Wasserstoff oder Hydroxy und $R^5$ Wasserstoff oder $R^4$ und $R^5$ zusammen eine Kohlenstoff-Kohlenstoff-Bindung und $R^6$ eine Aryl gruppe, welche eine Hydroxygruppe in 2-Stellung trägt, eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Hydroxy-3-pyridyl, 2-Hydroxy-4-methyl-3-pyridyl, 3-Hydroxy-4-pyridyl, 2-Pyrazinyl-1-oxid, 2-Pyrimidinyl-1-oxid, 6-Pyrimidinyl-1-oxid oder 2-Chinolyl-1-oxid bedeuten, wobei die als "nieder" bezeichneten Reste 1-7 Kohlenstoffatome aufweisen und "Aroyl" gegebenenfalls durch Halogen oder Nitro substituiertes Benzoyl und "Aryl" gegebenenfalls durch Halogen, Cyan oder nieder Alkyl ein- oder mehrfach substituiertes Phenyl oder Naphthyl bedeuten,
und pharmazeutisch verwendbare Säureadditionssalze der Verbindungen der Formel I, welche basisch sind.

**2.** Verbindungen gemäss Anspruch 1, worin $R^1$ Wasserstoff, Halogen, Nitro, Cyan, nieder Alkyl, nieder Alkoxycarbonyl, nieder Alkylthio, nieder Alkylsulfonyl, $C_{2-7}$-Alkanoyl, Carbamoyl, Mono(nieder alkyl)-carbamoyl oder Di(nieder alkyl)carbamoyl, $R^2$ und $R^3$ je Wasserstoff oder nieder Alkyl und $R^4$ und $R^5$ je Wasserstoff oder zusammen eine Kohlenstoff-Kohlenstoff-Bindung bedeuten.

**3.** Verbindungen gemäss Anspruch 1 oder 2, worin $R^1$ Nitro, Cyan oder $C_{2-7}$-Alkanoyl bedeutet.

**4.** Verbindungen gemäss Anspruch 3, worin $R^1$ Nitro, Cyan oder Acetyl bedeutet.

**5.** Verbindungen gemäss einem der Ansprüche 1-4, worin $R^2$ und $R^3$ je nieder Alkyl bedeuten.

**6.** Verbindungen gemäss Anspruch 5, worin $R^2$ und $R^3$ je Methyl bedeuten.

**7.** Verbindungen gemäss einem der Ansprüche 1-6, worin $R^4$ und $R^5$ je Wasserstoff oder $R^4$ und $R^5$ zusammen eine Kohlenstoff-Kohlenstoff-Bindung bedeuten.

**8.** Verbindungen gemäss einem der Ansprüche 1-7, worin $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet.

**9.** Verbindungen gemäss Anspruch 8, worin $R^6$ eine 2-Pyridyl-1-oxidgruppe bedeutet, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist.

**10.** Verbindungen gemäss einem der Ansprüche 1-9, worin $R^1$ Nitro, Cyan oder Acetyl, $R^2$ und $R^3$ je Methyl, $R^4$ und $R^5$ Wasserstoff oder zusammen eine Kohlenstoff-Kohlenstoff-Bindung und $R^6$ eine 2-Pyridyl-1-oxidgruppe bedeutet, welche gegebenenfalls durch Halogen, Amino, Hydroxy, Benzyloxy, Phenyl, nieder Alkylphenyl, nieder Alkyl oder nieder Alkoxycarbonyl substituiert ist.

**11.** 2-(3,4-Dihydro-2,2-dimethyl-6-nitro-2H-1-benzopyran-4-yl)pyridin-N-oxid.

**12.** 2-(6-Acetyl-3,4-dihydro-2,2-dimethyl-2H-1-benzopyran-4-yl)pyridin-N-oxid.

**13.** 2-(6-Cyan-3,4-dihydro-2,2-dimethyl-2H-1-benzopyran-4-yl)pyridin-N-oxid.

**14.** 2-(6-Cyan-3,4-dihydro-2,2-dimethyl-2H-1-benzopyran-4-yl)-5-phenylpyridin-N-oxid.

**15.** 2-(6-Acetyl-3,4-dihydro-2,2-dimethyl-2H-1-benzopyran-4-yl)-3-methylpyridin-N-oxid,
2-(3,4-Dihydro-2,2-dimethyl-6-nitro-2H-1-benzopyran-4-yl)-3-methylpyridin-N-oxid,
2-(6-Cyan-2,2-dimethyl-2H-1-benzopyran-4-yl)pyridin-N-oxid,
2-(3,4-Dihydro-2,2-dimethyl-2H-1-benzopyran-4-yl)pyridine-N-oxid,
2-(3,4-Dihydro-2,2-dimethyl-6-methylthio-2H-1-benzopyran-4-yl)pyridin-N-oxid,
2-(3,4-Dihydro-2,2-dimethyl-6-methylsulfonyl-2H-1-benzopyran-4-yl)pyridin-N-oxid,
2-(2,2-Dimethyl-2H-1-benzopyran-4-yl)pyridin-N-oxid,
2-(6-Brom-3,4-dihydro-2,2-dimethyl-2H-1-benzopyran-4-yl)pyridin-N-oxid,
2-[6-(Methoxycarbonyl)-2,2-dimethyl-2H-1-benzopyran-4-yl]pyridin-N-oxid,
2-[3,4-Dihydro-6-(methoxycarbonyl)-2,2-dimethyl-2H-1-benzopyran-4-yl]pyridin-N-oxid,
2-(6-Carbamoyl-3,4-dihydro-2,2-dimethyl-2H-1-benzopyran-4-yl)pyridin-N-oxid,
2-(6-Cyan-3,4-dihydro-2,2-dimethyl-2H-1-benzopyran-4-yl)pyrimidin-1-oxid,
2-(6-Cyan-2,2-dimethyl-2H-1-benzopyran-4-yl)chinolin-1-oxid,
2-(6-Cyan-3,4-dihydro-2,2-dimethyl-2H-1-benzopyran-4-yl)chinolin-1-oxid,
4-(2-Hydroxyphenyl)-2,2-dimethyl-2H-1-benzopyran-6-carbonitril,
4-(5-Cyan-2-hydroxyphenyl)-2,2-dimethyl-2H-1-benzopyran-6-carbonitril oder
3-(6-Cyan-3,4-dihydro-2,2-dimethyl-2H-1-benzopyran-4-yl)-2(1H)-pyridon.

**16.** 2-(6-Chlor-3,4-dihydro-2,2-dimethyl-2H-1-benzopyran-4-yl)pyridin-N-oxid,
2-(2,2,6-Trimethyl-2H-1-benzopyran-4-yl)pyridin-N-oxid,
2-[6-(Trifluormethyl)-2,2-dimethyl-2H-1-benzopyran-4-yl]pyridin-N-oxid,
2-[6-(t-Butyl)-3,4-dihydro-2,2-dimethyl-2H-1-benzopyran-4-yl]pyridin-N-oxid,
2-(6-Benzoyl-3,4-dihydro-2,2-dimethyl-2H-1-benzopyran-4-yl)pyridin-N-oxid,
2-[3,4-Dihydro-2,2-dimethyl-6-(4-nitrobenzoyl)-2H-1-benzopyran-4-yl]pyridin-N-oxid,
2-[3,4-Dihydro-2,2-dimethyl-6-(2-jodbenzoyl)-2H-1-benzopyran-4-yl]pyridin-N-oxid,
2-[3,4-Dihydro-2,2-dimethyl-6-(3-jodbenzoyl)-2H-1-benzopyran-4-yl]pyridin-N-oxid,
2-[3,4-Dihydro-2,2-dimethyl-6-(4-jodbenzoyl)-2H-1-benzopyran-4-yl]pyridin-N-oxid,
2-(6-Cyan-2-äthyl-2-methyl-2H-1-benzopyran-4-yl)pyridin-N-oxid,
2-(6-Acetyl-2-methyl-2H-1-benzopyran-4-yl)pyridin-N-oxid,
2-Chlor-6-(6-cyan-3,4-dihydro-2,2-dimethyl-2H-1-benzopyran-4-yl)pyridin-N-oxid,
4-Chlor-2-(6-cyan-3,4-dihydro-2,2-dimethyl-2H-1-benzopyran-4-yl)pyridin-N-oxid,
2-(6-Cyan-3,4-dihydro-2,2-dimethyl-2H-1-benzopyran-4-yl)-4-methoxypyridin-N-oxid,
2-Amino-6-(6-cyan-2,2-dimethyl-2H-1-benzopyran-4-yl)pyridin-N-oxid,
2-Amino-6-(6-cyan-3,4-dihydro-2,2-dimethyl-2H-1-benzopyran-4-yl)pyridin-N-oxid,
2-(6-Cyan-3,4-dihydro-2,2-dimethyl-2H-1-benzopyran-4-yl)-6-methylpyridin-N-oxid,
2-(6-Cyan-2,2-dimethyl-2H-1-benzopyran-4-yl)-4-methylpyridin-N-oxid,

2-(6-Cyan-3,4-dihydro-2,2-dimethyl-2H-1-benzopyran-4-yl)-4-methylpyridin-N-oxid,
2-(6-Cyan-3,4-dihydro-2,2-dimethyl-2H-1-benzopyran-4-yl)-5-(methoxycarbonyl)pyridin-N-oxid,
5-Amino-2-(6-cyan-2,2-dimethyl-2H-1-benzopyran-4-yl)pyridin-N-oxid,
5-Amino-2-(6-cyan-3,4-dihydro-2,2-dimethyl-2H-1-benzopyran-4-yl)pyridin-N-oxid,
2-(6-Cyan-2,2-dimethyl-2H-1-benzopyran-4-yl)-5-hydroxypyridin-N-oxid,
5-Chlor-2-(6-cyan-2,2-dimethyl-2H-1-benzopyran-4-yl)pyridin-N-oxid,
5-Chlor-2-(6-cyan-3,4-dihydro-2,2-dimethyl-2H-1-benzopyran-4-yl)pyridin-N-oxid,
2-(6-Cyan-2,2-dimethyl-2H-1-benzopyran-4-yl)-5-phenylpyridin-N-oxid,
2-(6-Acetyl-2,2-dimethyl-2H-1-benzopyran-4-yl)pyridin-N-oxid,
2-(6-Cyan-2,2-dimethyl-2H-1-benzopyran-4-yl)-5-methylpyridin-N-oxid,
2-(6-Cyan-3,4-dihydro-2,2-dimethyl-2H-1-benzopyran-4-yl)-5-methylpyridin-N-oxid,
2-(6-Brom-2,2-dimethyl-2H-1-benzopyran-4-yl)pyridin-N-oxid,
2-(6-Cyan-2,2-dimethyl-2H-1-benzopyran-4-yl)-5-(4-methylphenyl)pyridin-N-oxid,
2-(6-Acetyl-2-methyl-2-phenyl-2H-1-benzopyran-4-yl)pyridin-N-oxid,
5-Benzyloxy-2-(6-cyan-3,4-dihydro-2,2-dimethyl-2H-1-benzopyran-4-yl)pyridin-N-oxid,
2-(6-Cyan-3,4-dihydro-2,2-dimethyl-2H-1-benzopyran-4-yl)-5-hydroxypyridin-N-oxid,
rac-trans-2-(6-Cyan-3,4-dihydro-3-hydroxy-2,2-dimethyl-2H-1-benzopyran-4-yl)pyridin-N-oxid,
rac-cis-2-(6-Cyan-3,4-dihydro-3-hydroxy-2,2-dimethyl-2H-1-benzopyran-4-yl)pyridin-N-oxid,
6-(6-Cyan-3,4-dihydro-2,2-dimethyl-2H-1-benzopyran-4-yl)pyrimidin-1-oxid,
2-(6-Cyan-3,4-dihydro-2,2-dimethyl-2H-1-benzopyran-4-yl)pyrazin-1-oxid,
4-(6-Acetyl-2,2-dimethyl-2H-1-benzopyran-4-yl)-3-pyridinol,
4-(2,2-Dimethyl-2H-1-benzopyran-4-yl)-3-pyridinol,
(-)-2-(6-Cyan-3,4-dihydro-2,2-dimethyl-2H-1-benzopyran-4-yl)pyridin-N-oxid oder
(+)-2-(6-Cyan-3,4-dihydro-2,2-dimethyl-2H-1-benzopyran-4-yl)pyridin-N-oxid.

**17.** Eine Verbindung der allgemeinen Formel

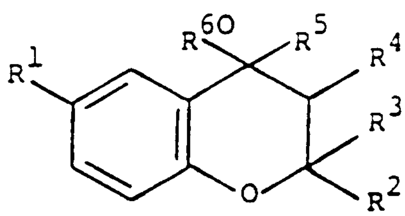

II

worin $R^1$ Wasserstoff, Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl, $R^4$ Wasserstoff oder Hydroxy und $R^5$ Wasserstoff oder $R^4$ und $R^5$ zusammen eine Kohlenstoff-Kohlenstoff-Bindung und $R^{60}$ eine Arylgruppe mit einer nieder Alkoxygruppe in 2-Stellung, 2-nieder Alkoxy-3-pyridyl, 2-nieder Alkoxy-4-methyl-3-pyridyl oder 3-nieder Alkoxy-4-pyridyl bedeuten, wobei die als "nieder" bezeichneten Reste 1-7 Kohlenstoffatome aufweisen und "Aroyl" gegebenenfalls durch Halogen oder Nitro substituiertes Benzoyl und "Aryl" gegebenenfalls durch Halogen, Cyan oder nieder Alkyl ein- oder mehrfach substituiertes Phenyl oder Naphthyl bedeuten.

**18.** Eine Verbindung der allgemeinen Formel

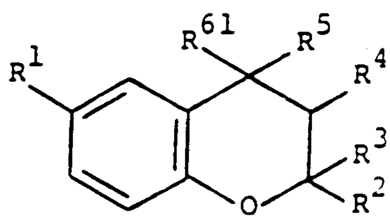

III

worin $R^1$ Wasserstoff, Halogen, Trifluormethyl, Nitro, Cyan, nieder Alkyl, nieder Alkoxycarbonyl, nieder

Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl, $R^4$ Wasserstoff oder Hydroxy und $R^5$ Wasserstoff oder $R^4$ und $R^5$ zusammen eine Kohlenstoff-Kohlenstoff-Bindung und $R^{61}$ eine 2-Pyridylgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl, 2-Pyrimidinyl, 6-Pyrimidinyl oder
2-Chinolyl bedeuten, wobei die als "nieder" bezeichneten Reste 1-7 Kohlenstoff-atome aufweisen und "Aroyl" gegebenenfalls durch Halogen oder Nitro substituiertes Benzoyl bedeutet.

**19.** Eine Verbindung der allgemeinen Formel

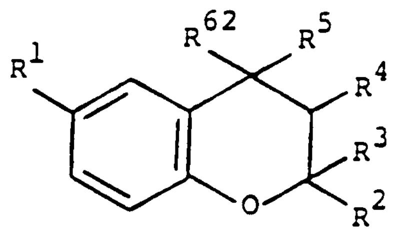

IV

worin $R^1$ Wasserstoff, Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl, $R^4$ Wasserstoff oder Hydroxy und $R^5$ Wasserstoff oder $R^4$ und $R^5$ zusammen eine Kohlenstoff-Kohlenstoff-Bindung und $R^{62}$ 3-Pyridyl-1-oxid oder 4-Methyl-3-pyridyl-1-oxid bedeuten, wobei die als "nieder" bezeichneten Reste 1-7 Kohlenstoffatome aufweisen und "Aroyl" gegebenenfalls durch Halogen oder Nitro substituiertes Benzoyl bedeutet.

**20.** Eine Verbinung der allgemeinen Formel

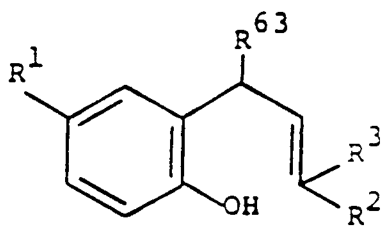

V

worin $R^1$ Wasserstoff, Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl und $R^{63}$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeuten, wobei die als "nieder" bezeichneten Reste 1-7 Kohlenstoffatome aufweisen und "Aroyl" gegebenenfalls durch Halogen oder Nitro substituiertes Benzoyl bedeutet.

**21.** Eine Verbindung gemäss einem der Ansprüche 1-16 oder ein pharmazeutisch verwendbares Säureadditionssalz einer solchen Verbindung, welche basisch ist, zur Anwendung als therapeutischer Wirkstoff.

**22.** Eine Verbindung gemäss einem der Ansprüche 1-16 oder ein pharmazeutisch verwendbares Säureadditionssalz einer solchen Verbindung, welche basisch ist, zur Anwendung bei der Bekämpfung bzw. Verhütung von Bluthochdruck, dekompensierter Herzinsuffizienz, Angina pectoris, peripheren und cerebralen Gefässerkrankungen oder Störungen der glatten Muskulatur.

**23.** Verfahren zur Herstellung einer Verbindung der in Anspruch 1 angegebenen Formel I oder eines pharmazeutisch verwendbaren Säureadditionssalz einer solchen Verbindung, welche basisch ist, dadurch gekennzeichnet, dass man

a) zur Herstellung einer Verbindung der Formel I, worin $R^6$ eine Arylgruppe mit einer Hydroxygruppe in 2-Stellung, 2-Hydroxy-3-pyridyl, 2-Hydroxy-4-methyl-3-pyridyl oder 3-Hydroxy-4-pyridyl bedeutet, die nieder Alkoxygruppe in einer Verbindung der allgemeinen Formel

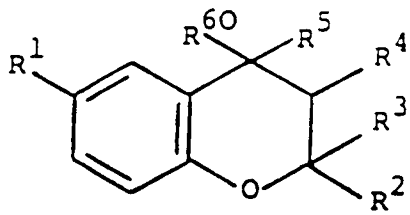

II

worin $R^1$, $R^2$, $R^3$, $R^4$ und $R^5$ die in Anspruch 1 angegebene Bedeutung besitzen und $R^{60}$ eine Arylgruppe mit einer nieder Alkoxygruppe in 2-Stellung, 2-nieder Alkoxy-3-pyridyl, 2-nieder Alkoxy-4-methyl-3-pyridyl oder 3-nieder Alkoxy-4-pyridyl bedeutet,

in eine Hydroxygruppe überführt, oder

b) zur Herstellung einer Verbindung der Formel I, worin $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet, eine Verbindung der allgemeinen Formel

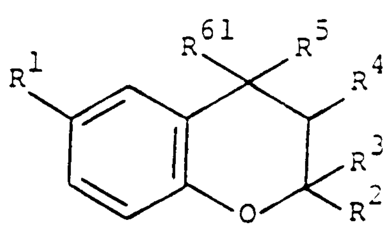

III

worin $R^1$, $R^2$, $R^3$, $R^4$ und $R^5$ die in Anspruch 1 angegebene Bedeutung besitzen und $R^{61}$ eine 2-Pyridylgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl, 2-Pyrimidinyl, 6-Pyrimidinyl oder 2-Chinolyl bedeutet,

oxidiert, oder

c) zur Herstellung einer Verbindung der Formel I, worin $R^6$ 2-Hydroxy-3-pyridyl oder 2-Hydroxy-4-methyl-3-pyridyl bedeutet, eine Verbindung der allgemeinen Formel

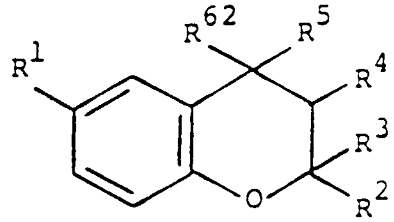

IV

worin $R^1$, $R^2$, $R^3$, $R^4$ und $R^5$ die in Anspruch 1 angegebene Bedeutung besitzen und $R^{62}$ 3-Pyridyl-1-oxid oder 4-Methyl-3-pyridyl-1-oxid bedeutet,

mit einem nieder Alkansäureanhydrid umsetzt und das erhaltene Produkt hydrolysiert, oder

d) zur Herstellung einer Verbindung der Formel I, worin $R^4$ und $R^5$ je Wasserstoff und $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeuten, eine

Verbindung der allgemeinen Formel

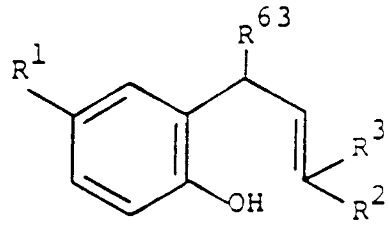

V

worin $R^1$, $R^2$, und $R^3$ die oben angegebene Bedeutung besitzen und $R^{63}$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet,
cyclisiert, oder

e) zur Herstellung einer Verbindung der Formel I, worin $R^1$ nieder Alkylsulfonyl bedeutet, eine Verbindung der Formel I, worin $R^1$ nieder Alkylthio bedeutet, oxidiert, oder

f) zur Herstellung einer Verbindung der Formel I, worin $R^1$ $C_{2-7}$-Alkanoyl oder Aroyl bedeutet, eine Verbindung der Formel I, worin $R^1$ Wasserstoff bedeutet, $C_{2-7}$-alkanoyliert oder aroyliert, oder

g) zur Herstellung einer Verbindung der Formel I, worin $R^6$ eine 2-Pyridyl-1-oxidgruppe mit einer nieder Alkoxygruppe in o- oder p-Stellung zur 1-Oxidgruppe bedeutet, eine Verbindung der Formel I, worin $R^6$ eine 2-Pyridyl-1-oxidgruppe mit einem Halogenatom in o- oder p-Stellung zur 1-Oxidgruppe bedeutet, mit einem Alkalimetall-nieder-alkoxid bei erhöhter Temperatur umsetzt, oder

h) zur Herstellung einer Verbindung der Formel I, worin $R^4$ und $R^5$ je Wasserstoff und $R^6$ eine Hydroxy-substituierte 2-Pyridyl-1-oxidgruppe bedeuten, eine Verbindung der Formel I, worin $R^4$ und $R^5$ je Wasserstoff und $R^6$ eine Benzyloxy-substituierte 2-Pyridyl-1-oxidgruppe bedeuten, katalytisch hydriert, oder

i) zur Herstellung einer Verbindung der Formel I, worin $R^4$ und $R^5$ zusammen eine Kohlenstoff-Kohlenstoff-Bindung und $R^6$ eine 3-Hydroxy-4-pyridylgruppe bedeuten, ein 3-[N,N-Di(nieder alkyl)-carbamoyloxy]pyridin mit einer Verbindung der allgemeinen Formel

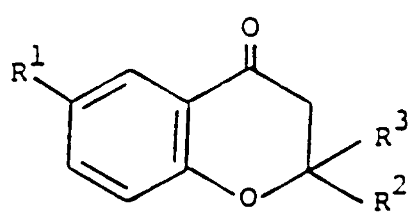

VI

worin $R^1$, $R^2$ und $R^3$ die oben angegebene Bedeutung besitzen,
in Gegenwart einer Alkalimetallalkylverbindung umsetzt, und/oder

j) erwünschtenfalls, ein erhaltenes Gemisch von diastereomeren Racematen in die entsprechenden diastereomeren Racemate oder optisch reinen Diastereomeren auf trennt, und/oder

k) erwünschtenfalls, ein erhaltenes Racemat in die beiden optischen Antipoden auftrennt, und/oder

l) erwünschtenfalls, ein erhaltenes cis/trans-Gemisch in die cis- und trans-Isomeren auftrennt, und

m) erwünschtenfalls, eine erhaltene basische Verbindung der Formel I in ein pharmazeutisch verwendbares Säureadditionssalz überführt.

**24.** Arzneimittel, enthaltend eine Verbindung gemäss einem der Ansprüche 1-16 oder ein pharmazeutisch verwendbares Säureadditionssalz einer solchen Verbindung, welche basisch ist, und ein therapeutisch inertes Excipiens.

**25.** Mittel zur Bekämpfung bzw. Verhütung von Bluthochdruck, dekompensierter Herzinsuffizienz, Angina pectoris, peripheren und cerebralen Gefässerkrankungen oder Störungen der glatten Muskulatur, enthaltend eine Verbindung gemäss einem der Ansprüche 1-16 oder ein pharmazeutisch verwendbares

Säureadditionssalz einer solchen Verbindung, welche basisch ist, und ein therapeutisch inertes Excipiens.

**26.** Verwendung einer Verbindung gemäss einem der Ansprüche 1-16 oder eines pharmazeutisch verwendbaren Säureadditionssalzes einer solchen Verbindung, welche basisch ist, zur Herstellung eines Mittels zur Bekämpfung bzw. Verhütung von Bluthochdruck, dekompensierter Herzinsuffizienz, Angina pectoris, peripheren und cerebralen Gefässerkrankungen oder Störungen der glatten Muskulatur.

**Patentansprüche für folgenden Vertragsstaat : ES**

**1.** Verfahren zur Herstellung von Verbindungen der allgemeinen Formel

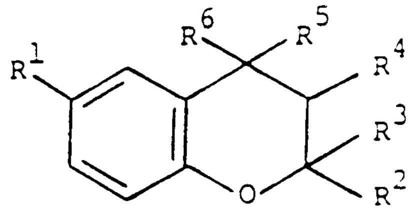

I

worin $R^1$ Wasserstoff, Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl, $R^4$ Wasserstoff oder Hydroxy und $R^5$ Wasserstoff oder $R^4$ und $R^5$ zusammen eine Kohlenstoff-Kohlenstoff-Bindung und $R^6$ eine Arylgruppe, welche eine Hydroxygruppe in 2-Stellung trägt, eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Hydroxy-3-pyridyl, 2-Hydroxy-4-methyl-3-pyridyl, 3-Hydroxy-4-pyridyl, 2-Pyrazinyl-1-oxid, 2-Pyrimidinyl-1-oxid, 6-Pyrimidinyl-1-oxid oder 2-Chinolyl-1-oxid bedeuten, wobei die als "nieder" bezeichneten Reste 1-7 Kohlenstoffatome aufweisen und "Aroyl" gegebenenfalls durch Halogen oder Nitro substituiertes Benzoyl und "Aryl" gegebenenfalls durch Halogen, Cyan oder nieder Alkyl ein- oder mehrfach substituiertes Phenyl oder Naphthyl bedeuten,
und pharmazeutisch verwendbaren Säureadditionssalzen solcher Verbindungen der Formel I, welche basisch sind, dadurch gekennzeichnet, dass man

a) zur Herstellung einer Verbindung der Formel I, worin $R^6$ eine Arylgruppe mit einer Hydroxygruppe in 2-Stellung, 2-Hydroxy-3-pyridyl, 2-Hydroxy-4-methyl-3-pyridyl oder 3-Hydroxy-4-pyridyl bedeutet, die nieder Alkoxygruppe in einer Verbindung der allgemeinen Formel

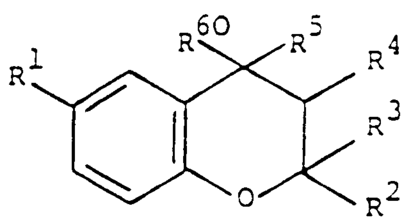

II

worin $R^1$, $R^2$, $R^3$, $R^4$ und $R^5$ die oben angegebene Bedeutung besitzen und $R^{60}$ eine Arylgruppe mit einer nieder Alkoxygruppe in 2-Stellung, 2-nieder Alkoxy-3-pyridyl, 2-nieder Alkoxy-4-methyl-3-pyridyl oder 3-nieder-Alkoxy-4-pyridyl bedeutet,
in eine Hydroxygruppe überführt, oder

b) zur Herstellung einer Verbindung der Formel I, worin $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet, eine Verbindung der allgemeinen Formel

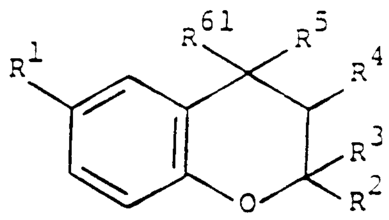

III

worin $R^1$, $R^2$, $R^3$, $R^4$ und $R^5$ die oben angegebene Bedeutung besitzen und $R^{61}$ eine 2-Pyridylgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl, 2-Pyrimidinyl, 6-Pyrimidinyl oder 2-Chinolyl bedeutet,
oxidiert, oder
c) zur Herstellung einer Verbindung der Formel I, worin $R^6$ 2-Hydroxy-3-pyridyl oder 2-Hydroxy-4-methyl-3-pyridyl bedeutet, eine Verbindung der allgemeinen Formel

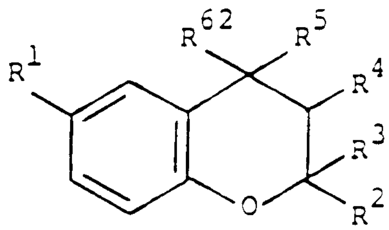

IV

worin $R^1$, $R^2$, $R^3$, $R^4$ und $R^5$ die oben angegebene Bedeutung besitzen und $R^{62}$ 3-Pyridyl-1-oxid oder 4-Methyl-3-pyridyl-1-oxid bedeutet,
mit einem nieder Alkansäureanhydrid umsetzt und das erhaltene Produkt hydrolysiert, oder
d) zur Herstellung einer Verbindung der Formel I, worin $R^4$ und $R^5$ je Wasserstoff und $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeuten, eine Verbindung der allgemeinen Formel

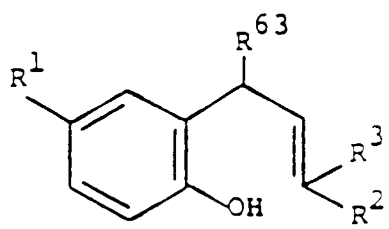

V

worin $R^1$, $R^2$, und $R^3$ die oben angegebene Bedeutung besitzen und $R^{63}$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet,
cyclisiert, oder
e) zur Herstellung einer Verbindung der Formel I, worin $R^1$ nieder Alkylsulfonyl bedeutet, eine Verbindung der Formel I, worin $R^1$ nieder Alkylthio bedeutet, oxidiert, oder
f) zur Herstellung einer Verbindung der Formel I, worin $R^1$ $C_{2-7}$-Alkanoyl oder Aroyl bedeutet, eine Verbindung der Formel I, worin $R^1$ Wasserstoff bedeutet, $C_{2-7}$-alkanoyliert oder aroyliert, oder
g) zur Herstellung einer Verbindung der Formel I, worin $R^6$ eine 2-Pyridyl-1-oxidgruppe mit einer nieder Alkoxygruppe in o- oder p-Stellung zur 1-Oxidgruppe bedeutet, eine Verbindung der Formel I, worin $R^6$ eine 2-Pyridyl-1-oxidgruppe mit einem Halogenatom in o- oder p-Stellung zur 1-Oxidgruppe bedeutet, mit einem Alkalimetall-nieder-alkoxid bei erhöhter Temperatur umsetzt, oder
h) zur Herstellung einer Verbindung der Formel I, worin $R^4$ und $R^5$ je Wasserstoff und $R^6$ eine Hydroxy-substituierte 2-Pyridyl-1-oxidgruppe bedeuten, eine Verbindung der Formel I, worin $R^4$ und $R^5$ je Wasserstoff und $R^6$ eine Benzyloxy-substituierte 2-Pyridyl-1-oxidguppe bedeuten, katalytisch hydriert, oder
i) zur Herstellung einer Verbindung der Formel I, worin $R^4$ und $R^5$ zusammen eine Kohlenstoff-Kohlenstoff-Bindung und $R^6$ eine 3-Hydroxy-4-pyridylgruppe bedeuten, ein 3-[N,N-Di(nieder alkyl)-carbamoyloxy]pyridin mit einer Verbindung der allgemeinen Formel

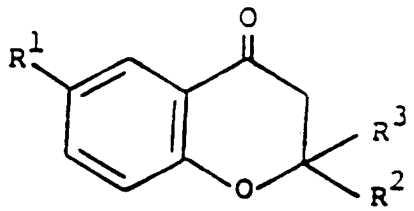

VI

worin $R^1$, $R^2$ und $R^3$ die oben angegebene Bedeutung besitzen,
in Gegenwart einer Alkalimetallalkylverbindung umsetzt, und/oder
j) erwünschtenfalls, ein erhaltenes Gemisch von diastereomeren Racematen in die entsprechenden diastereomeren Racemate oder optisch reinen Diastereomeren auftrennt, und/oder
k) erwünschtenfalls, ein erhaltenes Racemat in die beiden optischen Antipoden auftrennt, und/oder
l) erwünschtenfalls, ein erhaltenes cis/trans-Gemisch in die cis- und trans-Isomeren auftrennt, und
m) erwünschtenfalls, eine erhaltene basische Verbindung der Formel I in ein pharmazeutisch verwendbares Säureadditionssalz überführt.

**2.** Verfahren gemäss Anpruch 1, worin $R^1$ Wasserstoff, Halogen, Nitro, Cyan, nieder Alkyl, nieder Alkoxycarbonyl, nieder Alkylthio, nieder Alkylsulfonyl, $C_{2-7}$-Alkanoyl, Carbamoyl, Mono(nieder alkyl)-carbamoyl oder Di(nieder alkyl)carbamoyl, $R^2$ und $R^3$ je Wasserstoff oder nieder Alkyl und $R^4$ und $R^5$ je Wasserstoff oder zusammen eine Kohlenstoff-Kohlenstoff-Bindung bedeuten.

**3.** Verfahren gemäss Anspruch 1 oder 2, worin $R^1$ Nitro, Cyan oder $C_{2-7}$-Alkanoyl bedeutet.

**4.** Verfahren gemäss Anspruch 3, worin $R^1$ Nitro, Cyan oder Acetyl bedeutet.

**5.** Verfahren gemäss einem der Ansprüche 1-4, worin $R^2$ und $R^3$ je nieder Alkyl bedeuten.

**6.** Verfahren gemäss Anspruch 5, worin $R^2$ und $R^3$ je Methyl bedeuten.

**7.** Verfahren gemäss einem der Ansprüche 1-6, worin $R^4$ und $R^5$ je Wasserstoff oder zusammen eine Kohlenstoff-Kohlenstoff-Bindung bedeuten.

**8.** Verfahren gemäss einem der Ansprüche 1-7, worin $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet.

**9.** Verfahren gemäss Anspruch 8, worin $R^6$ eine 2-Pyridyl-1-oxidgruppe bedeutet, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist.

**10.** Verfahren gemäss einem der Ansprüche 1-9, worin $R^1$ Nitro, Cyan oder Acetyl, $R^2$ und $R^3$ je Methyl, $R^4$ und $R^5$ je Wasserstoff oder zusammen eine Kohlenstoff-Kohlenstoff-Bindung und $R^6$ eine 2-Pyridyl-1-oxidgruppe bedeuten, welche gegebenenfalls durch Halogen, Amino, Hydroxy, Benzyloxy, Phenyl, nieder Alkylphenyl, nieder Alkyl oder nieder Alkoxycarbonyl substituiert ist.

**11.** Verfahren gemäss Anspruch 2, dadurch gekennzeichnet, dass man 2-(3,4-Dihydro-2,2-dimethyl-6-nitro-2H-1-benzopyran-4-yl)pyridin-N-oxid herstellt.

**12.** Verfahren gemäss Anspruch 2, dadurch gekennzeichnet, dass man 2-(6-Acetyl-3,4-dihydro-2,2-dimethyl-2H-1-benzopyran-4-yl)pyridin-N-oxid herstellt.

**13.** Verfahren gemäss Anspruch 2, dadurch gekennzeichnet, dass man 2-(6-Cyan-3,4-dihydro-2,2-dimethyl-2H-1-benzopyran-4-yl)pyridin-N-oxid herstellt.

**14.** Verfahren gemäss Anspruch 2, dadurch gekennzeichnet, dass man 2-(6-Cyan-3,4-dihydro-2,2-dimethyl-2H-1-benzopyran-4-yl)pyridin-N-oxid herstellt.

**15.** Verwendung einer Verbindung der Formel I in Anspruch 1 oder eines pharmazeutisch verwendbaren Säureadditionssalzes einer solchen Verbindung, welche basisch ist, zur Herstellung eines Mittels zur Bekämpfung bzw. Verhütung von Bluthochdruck, dekompensierter Herzinsuffizienz, Angina pectoris, peripheren und cerebralen Gefässerkrankungen oder Störungen der glatten Muskulatur.

**Patentansprüche für folgenden Vertragsstaat : GR**

**1.** Verfahren zur Herstellung von Verbindungen der allgemeinen Formel

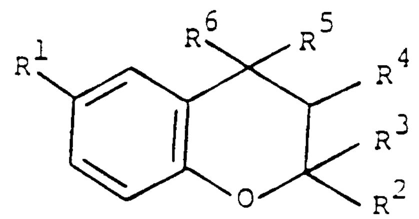

I

worin $R^1$ Wasserstoff, Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl, $R^4$ Wasserstoff oder Hydroxy, $R^5$ Wasserstoff oder $R^4$ und $R^5$ zusammen eine Kohlenstoff-Kohlenstoff-Bindung und $R^6$ eine Arylgruppe, welche eine Hydroxygruppe in 2-Stellung trägt, eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Hydroxy-3-pyridyl, 2-Hydroxy-4-methyl-3-pyridyl, 3-Hydroxy-4-pyridyl, 2-Pyrazinyl-1-oxid, 2-Pyrimidinyl-1-oxid, 6-Pyrimidinyl-1-oxid oder 2-Chinolyl-1-oxid bedeuten, wobei die als "nieder" bezeichneten Reste 1-7 Kohlenstoffatome aufweisen und "Aroyl" gegebenenfalls durch Halogen oder Nitro substituiertes Benzoyl und "Aryl" gegebenenfalls durch Halogen, Cyan oder nieder Alkyl ein- oder mehrfach substituiertes Phenyl oder Naphthyl bedeuten,
und pharmazeutisch verwendbaren Säureadditionssalzen solcher Verbindungen der Formel I, welche basisch sind, dadurch gekennzeichnet, dass man

a) zur Herstellung einer Verbindung der Formel I, worin $R^6$ eine Arylgruppe mit einer Hydroxygruppe in 2-Stellung, 2-Hydroxy-3-pyridyl, 2-Hydroxy-4-methyl-3-pyridyl oder 3-Hydroxy-4-pyridyl bedeutet, die nieder Alkoxygruppe in einer Verbindung der allgemeinen Formel

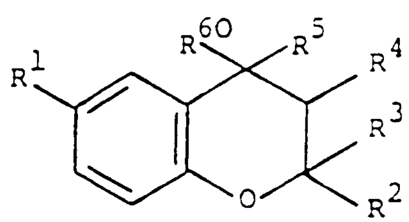

II

worin $R^1$, $R^2$, $R^3$, $R^4$ und $R^5$ die oben angegebene Bedeutung besitzen und $R^{60}$ eine Arylgruppe mit einer nieder Alkoxygruppe in 2-Stellung, 2-nieder Alkoxy-3-pyridyl, 2-nieder Alkoxy-4-methyl-3-pyridyl oder 3-nieder-Alkoxy-4-pyridyl bedeutet,
in eine Hydroxygruppe überführt, oder

b) zur Herstellung einer Verbindung der Formel I, worin $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet, eine Verbindung der allgemeinen Formel

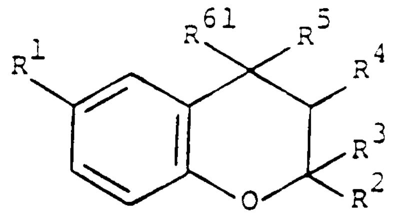

III

worin $R^1$, $R^2$, $R^3$, $R^4$ und $R^5$ die oben angegebene Bedeutung besitzen und $R^{61}$ eine 2-Pyridylgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl, 2-Pyrimidinyl, 6-Pyrimidinyl oder 2-Chinolyl bedeutet,
oxidiert, oder
c) zur Herstellung einer Verbindung der Formel I, worin $R^6$ 2-Hydroxy-3-pyridyl oder 2-Hydroxy-4-methyl-3-pyridyl bedeutet, eine Verbindung der allgemeinen Formel

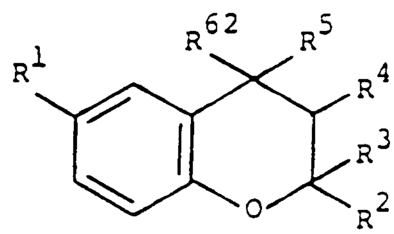

IV

worin $R^1$, $R^2$, $R^3$, $R^4$ und $R^5$ die oben angegebene Bedeutung besitzen und $R^{62}$ 3-Pyridyl-1-oxid oder 4-Methyl-3-pyridyl-1-oxid bedeutet,
mit einem nieder Alkansäureanhydrid umsetzt und das erhaltene Produkt hydrolysiert, oder
d) zur Herstellung einer Verbindung der Formel I, worin $R^4$ und $R^5$ je Wasserstoff und $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeuten, eine Verbindung der allgemeinen Formel bedeuten, eine Verbindung der allgemeinen Formel

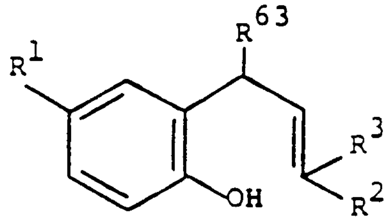

V

worin $R^1$, $R^2$, und $R^3$ die oben angegebene Bedeutung besitzen und $R^{63}$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet,
cyclisiert, oder
e) zur Herstellung einer Verbindung der Formel I, worin $R^1$ nieder Alkylsulfonyl bedeutet, eine Verbindung der Formel I, worin $R^1$ nieder Alkylthio bedeutet, oxidiert, oder

f) zur Herstellung einer Verbindung der Formel I, worin $R^1$ $C_{2-7}$-Alkanoyl oder Aroyl bedeutet, eine Verbindung der Formel I, worin $R^1$ Wasserstoff bedeutet, $C_{2-7}$-alkanoyliert oder aroyliert, oder

g) zur Herstellung einer Verbindung der Formel I, worin $R^6$ eine 2-Pyridyl-1-oxidgruppe mit einer nieder Alkoxygruppe in o- oder p-Stellung zur 1-Oxidgruppe bedeutet, eine Verbindung der Formel I, worin $R^6$ eine 2-Pyridyl-1-oxidgruppe mit einem Halogenatom in o- oder p-Stellung zur 1-Oxidgruppe bedeutet, mit einem Alkalimetall-nieder-alkoxid bei erhöhter Temperatur umsetzt, oder

h) zur Herstellung einer Verbindung der Formel I, worin $R^4$ und $R^5$ je Wasserstoff und $R^6$ eine Hydroxy-substituierte 2-Pyridyl-1-oxidgruppe bedeuten, eine Verbindung der Formel I, worin $R^4$ und $R^5$ je Wasserstoff und $R^6$ eine Benzyloxy-substituierte 2-Pyridyl-1-oxidguppe bedeuten, katalytisch hydriert, oder

i) zur Herstellung einer Verbindung der Formel I, worin $R^4$ und $R^5$ zusammen eine Kohlenstoff-Kohlenstoff-Bindung und $R^6$ eine 3-Hydroxy-4-pyridylgruppe bedeuten, ein 3-[N,N-Di(nieder alkyl)-carbamoyloxy]pyridin mit einer Verbindung der allgemeinen Formel

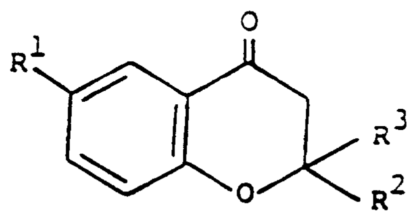

VI

worin $R^1$, $R^2$ und $R^3$ die oben angegebene Bedeutung besitzen,
in Gegenwart einer Alkalimetallalkylverbindung umsetzt, und/oder

j) erwünschtenfalls, ein erhaltenes Gemisch von diastereomeren Racematen in die entsprechenden diastereomeren Racemate oder optisch reinen Diastereomeren auftrennt, und/oder

k) erwünschtenfalls, ein erhaltenes Racemat in die beiden optischen Antipoden auftrennt, und/oder

l) erwünschtenfalls, ein erhaltenes cis/trans-Gemisch in die cis- und trans-Isomeren auftrennt, und

m) erwünschtenfalls, eine erhaltene basische Verbindung der Formel I in ein pharmazeutisch verwendbares Säureadditionssalz überführt.

**2.** Verfahren gemäss Anpruch 1, worin $R^1$ Wasserstoff, Halogen, Nitro, Cyan, nieder Alkyl, nieder Alkoxycarbonyl, nieder Alkylthio, nieder Alkylsulfonyl, $C_{2-7}$-Alkanoyl, Carbamoyl, Mono(nieder alkyl)-carbamoyl oder Di(nieder alkyl)carbamoyl, $R^2$ und $R^3$ je Wasserstoff oder nieder Alkyl und $R^4$ und $R^5$ je Wasserstoff oder zusammen eine Kohlenstoff-Kohlenstoff-Bindung bedeuten.

**3.** Verfahren gemäss Anspruch 1 oder 2, worin $R^1$ Nitro, Cyan oder $C_{2-7}$-Alkanoyl bedeutet.

**4.** Verfahren gemäss Anspruch 3, worin $R^1$ Nitro, Cyan oder Acetyl bedeutet.

**5.** Verfahren gemäss einem der Ansprüche 1-4, worin $R^2$ und $R^3$ je nieder Alkyl bedeuten.

**6.** Verfahren gemäss Anspruch 5, worin $R^2$ und $R^3$ je Methyl bedeuten.

**7.** Verfahren gemäss einem der Ansprüche 1-6, worin $R^4$ und $R^5$ je Wasserstoff oder zusammen eine Kohlenstoff-Kohlenstoff-Bindung bedeuten.

**8.** Verfahren gemäss einem der Ansprüche 1-7, worin $R^6$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeutet.

**9.** Verfahren gemäss Anspruch 8, worin $R^6$ eine 2-Pyridyl-1-oxidgruppe bedeutet, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist.

**10.** Verfahren gemäss einem der Ansprüche 1-9, worin $R^1$ Nitro, Cyan oder Acetyl, $R^2$ und $R^3$ je Methyl, $R^4$ und $R^5$ je Wasserstoff oder zusammen eine Kohlenstoff-Kohlenstoff-Bindung und $R^6$ eine 2-Pyridyl-1-oxidgruppe bedeuten, welche gegebenenfalls durch Halogen, Amino, Hydroxy, Benzyloxy, Phenyl, nieder Alkylphenyl, nieder Alkyl oder nieder Alkoxycarbonyl substituiert ist.

**11.** Verfahren gemäss Anspruch 2, dadurch gekennzeichnet, dass man 2-(3,4-Dihydro-2,2-dimethyl-6-nitro-2H-1-benzopyran-4-yl)pyridin-N-oxid herstellt.

**12.** Verfahren gemäss Anspruch 2, dadurch gekennzeichnet, dass man 2-(6-Acetyl-3,4-dihydro-2,2-dimethyl-2H-1-benzopyran-4-yl)pyridin-N-oxid herstellt.

**13.** Verfahren gemäss Anspruch 2, dadurch gekennzeichnet, dass man 2-(6-Cyan-3,4-dihydro-2,2-dimethyl-2H-1-benzopyran-4-yl)pyridin-N-oxid herstellt.

**14.** Verfahren gemäss Anspruch 2, dadurch gekennzeichnet, dass man 2-(6-Cyan-3,4-dihydro-2,2-dimethyl-2H-1-benzopyran-4-yl)pyridin-N-oxid herstellt.

**15.** Verwendung einer Verbindung der Formel I in Anspruch 1 oder eines pharmazeutisch verwendbaren Säureadditionssalzes einer solchen Verbindung, welche basisch ist, zur Herstellung eines Mittels zur Bekämpfung bzw. Verhütung von Bluthochdruck, dekompensierter Herzinsuffizienz, Angina pectoris, peripheren und cerebralen Gefässerkrankungen oder Störungen der glatten Muskulatur.

**16.** Eine Verbindung der allgemeinen Formel

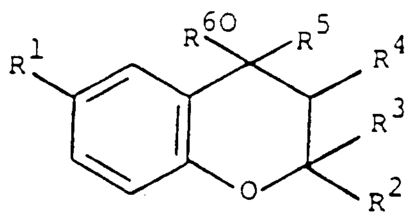

II

worin $R^1$ Wasserstoff, Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl, $R^4$ Wasserstoff oder Hydroxy und $R^5$ Wasserstoff oder $R^4$ und $R^5$ zusammen eine Kohlenstoff-Kohlenstoff-Bindung und $R^{60}$ eine Arylgruppe mit einer nieder Alkoxygruppe in 2-Stellung, 2-nieder Alkoxy-3-pyridyl, 2-nieder Alkoxy-4-methyl-3-pyridyl oder 3-nieder Alkoxy-4-pyridyl bedeuten, wobei die als "nieder" bezeichneten Reste 1-7 Kohlenstoffatome aufweisen und "Aroyl" gegebenenfalls durch Halogen oder Nitro substituiertes Benzoyl und "Aryl" gegebenenfalls durch Halogen, Cyan oder nieder Alkyl ein- oder mehrfach substituiertes Phenyl oder Naphthyl bedeuten.

**17.** Eine Verbindung der allgemeinen Formel

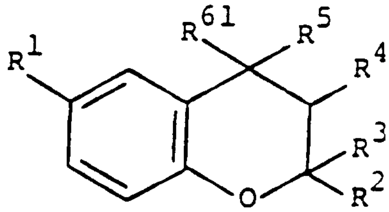

III

worin $R^1$ Wasserstoff. Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl, $R^4$ Wasserstoff oder Hydroxy und $R^5$ Wasserstoff oder $R^4$ und $R^5$ zusammen eine Kohlenstoff-Kohlenstoff-Bindung und $R^{61}$ eine 2-Pyridylgruppe, welche gegebenenfalls durch Halogen, Amino, Hydroxy,

Benzyloxy, Phenyl, nieder Alkylphenyl, nieder Alkyl, nieder Alkoxy oder nieder Alkoxycarbonyl substituiert ist, 2-Pyrazinyl, 2-Pyrimidinyl, 6-Pyrimidinyl oder 2-Chinolyl bedeuten, wobei die als "nieder" bezeichneten Reste 1-7 Kohlenstoff-atome aufweisen und "Aroyl" gegebenenfalls durch Halogen oder Nitro substituiertes Benzoyl bedeutet.

**18.** Eine Verbindung der allgemeinen Formel

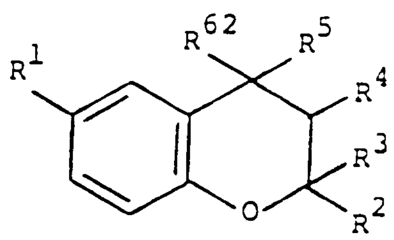

IV

worin $R^1$ Wasserstoff, Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl, $R^4$ Wasserstoff oder Hydroxy und $R^5$ Wasserstoff oder $R^4$ und $R^5$ zusammen eine Kohlenstoff-Kohlenstoff-Bindung und $R^{62}$ 3-Pyridyl-1-oxid oder 4-Methyl-3-pyridyl-1-oxid bedeuten, wobei die als "nieder" bezeichneten Reste 1-7 Kohlenstoffatome aufweisen und "Aroyl" gegebenenfalls durch Halogen oder Nitro substituiertes Benzoyl bedeutet.

**19.** Eine Verbinung der allgemeinen Formel

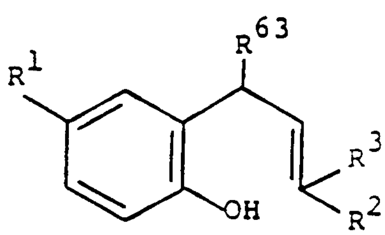

V

worin $R^1$ Wasserstoff, Halogen, Trifluormethyl, Nitro, Cyan, nieder Alkyl, nieder Alkoxycarbonyl, nieder Alkylthio, nieder Alkylsulfonyl, $C_{2-7}$-Alkanoyl, Aroyl, Carbamoyl, Mono(nieder alkyl)carbamoyl oder Di-(nieder alkyl)carbamoyl, $R^2$ Wasserstoff, nieder Alkyl oder Phenyl, $R^3$ Wasserstoff oder nieder Alkyl und $R^{63}$ eine 2-Pyridyl-1-oxidgruppe, welche gegebenenfalls durch Halogen, Amino, Hydroxy, Benzyloxy, Phenyl, nieder Alkylphenyl, nieder Alkyl, nieder Alkoxy oder nieder Alkoxycarbonyl substituiert ist, 2-Pyrazinyl-1-oxid, 2-Pyrimidinyl-1-oxid, 6-Pyrimidinyl-1-oxid oder 2-Chinolyl-1-oxid bedeuten, wobei die als "nieder" bezeichneten Reste 1-7 Kohlenstoffatome aufweisen und "Aroyl" gegebenenfalls durch Halogen oder Nitro substituiertes Benzoyl bedeutet.

## Claims

**Claims for the following Contracting States : AT, BE, CH, DE, FR, GB, IT, LI, LU, NL, SE**

**1.** Compounds of the general formula

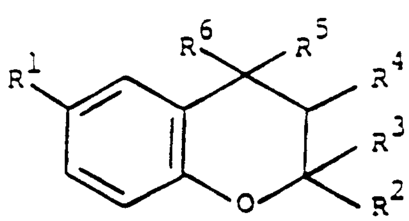

I

wherein $R^1$ represents hydrogen, halogen, trifluoromethyl, nitro, cyano, lower alkyl, lower alkoxycarbonyl, lower alkylthio, lower alkylsulphonyl, $C_{2-7}$-alkanoyl, aroyl, carbamoyl, mono(lower alkyl)-carbamoyl or di(lower alkyl)carbamoyl, $R^2$ represents hydrogen, lower alkyl or phenyl, $R^3$ represents hydrogen or lower alkyl, $R^4$ represents hydrogen or hydroxy and $R^5$ represents hydrogen or $R^4$ and $R^5$ together represent a carbon-carbon bond and $R^6$ represents an aryl group carrying a hydroxy group in the 2-position, a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-hydroxy-3-pyridyl, 2-hydroxy-4-methyl-3-pyridyl, 3-hydroxy-4-pyridyl, 2-pyrazinyl 1-oxide, 2-pyrimidinyl 1-oxide, 6-pyrimidinyl 1-oxide or 2-quinolyl 1-oxide, whereby the residues denoted as "lower" have 1-7 carbon atoms and "aroyl" signifies benzoyl optionally substituted by halogen or nitro and "aryl" signifies phenyl or naphthyl optionally mono- or multiply-substituted by halogen, cyano or lower alkyl,
and pharmaceutically acceptable acid addition salts of those compounds of formula I which are basic.

**2.** Compounds according to claim 1, wherein $R^1$ represents hydrogen, halogen, nitro, cyano, lower alkyl, lower alkoxycarbonyl, lower alkylthio, lower alkylsulphonyl, $C_{2-7}$-alkanoyl, carbamoyl, mono(lower alkyl)carbamoyl or di(lower alkylcarbamoyl), $R^2$ and $R^3$ each represent hydrogen or lower alkyl and $R^4$ and $R^5$ each represent hydrogen or together represent a carbon-carbon bond.

**3.** Compounds according to claim 1 or 2, wherein $R^1$ represents nitro, cyano or $C_{2-7}$-alkanoyl.

**4.** Compounds according to claim 3, wherein $R^1$ represents nitro, cyano or acetyl.

**5.** Compounds according to any one of claims 1-4, wherein $R^2$ and $R^3$ each represent lower alkyl.

**6.** Compounds according to claim 5, wherein $R^2$ and $R^3$ each represent methyl.

**7.** Compounds according to any one of claims 1-6, wherein $R^4$ and $R^5$ each represent hydrogen or $R^4$ and $R^5$ together represent a carbon-carbon bond.

**8.** Compounds according to any one of claims 1-7, wherein $R^6$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide.

**9.** Compounds according to claim 8, wherein $R^6$ represents a 2-pyridyl 1-oxide group which is optionally substituted by halogen, amino, hydroxy, benzyloxy, phenyl, lower alkylphenyl, lower alkyl, lower alkoxy or lower alkoxycarbonyl.

**10.** Compounds according to any one of claims 1-9, wherein $R^1$ represents nitro, cyano or acetyl, $R^2$ and $R^3$ each represent methyl, $R^4$ and $R^5$ each represent hydrogen or together represent a carbon-carbon bond and $R^6$ represents a 2-pyridyl 1-oxide group which is optionally substituted by halogen, amino, hydroxy, benzyloxy, phenyl, lower alkylphenyl, lower alkyl or lower alkoxycarbonyl.

**11.** 2-(3,4-Dihydro-2,2-dimethyl-6-nitro-2H-1-benzopyran-4-yl)pyridine N-oxide.

**12.** 2-(6-Acetyl-3,4-dihydro-2,2-dimethyl-2H-1-benzopyran-4-yl)pyridine N-oxide.

**13.** 2-(6-Cyano-3,4-dihydro-2,2-dimethyl-2H-1-benzopyran-4-yl)pyridine N-oxide.

**14.** 2-(6-Cyano-3,4-dihydro-2,2-dimethyl-2H-1-benzopyran-4-yl)-5-phenylpyridine N-oxide.

**15.** 2-(6-Acetyl-3,4-dihydro-2,2-dimethyl-2H-1-benzopyran-4-yl)-3-methylpyridine N-oxide,
2-(3,4-dihydro-2,2-dimethyl-6-nitro-2H-1-benzopyran-4-yl)-3-methylpyridine N-oxide,
2-(6-cyano-2,2-dimethyl-2H-1-benzopyran-4-yl)pyridine N-oxide,
2-(3,4-dihydro-2,2-dimethyl-2H-1-benzopyran-4-yl)pyridine N-oxide,
2-(3,4-dihydro-2,2-dimethyl-6-methylthio-2H-1-benzopyran-4-yl)pyridine N-oxide,
2-(3,4-dihydro-2,2-dimethyl-6-methylsulphonyl-2H-1-benzopyran-4-yl)pyridine N-oxide,
2-(2,2-dimethyl-2H-1-benzopyran-4-yl)pyridine N-oxide,
2-(6-bromo-3,4-dihydro-2,2-dimethyl-2H-1-benzopyran-4-yl)pyridine N-oxide,

2-[6-(methoxycarbonyl)-2,2-dimethyl-2H-1-benzopyran-4-yl]pyridine N-oxide,
2-[3,4-dihydro-6-(methoxycarbonyl)-2,2-dimethyl-2H-1-benzopyran-4-yl]pyridine N-oxide,
2-(6-carbamoyl-3,4-dihydro-2,2-dimethyl-2H-1-benzopyran-4-yl)pyridine N-oxide,
2-(6-cyano-3,4-dihydro-2,2-dimethyl-2H-1-benzopyran-4-yl)pyrimidine 1-oxide,
2-(6-cyano-2,2-dimethyl-2H-1-benzopyran-4-yl)quinoline 1-oxide,
2-(6-cyano-3,4-dihydro-2,2-dimethyl-2H-1-benzopyran-4-yl)quinoline 1-oxide,
4-(2-hydroxyphenyl)-2,2-dimethyl-2H-1-benzopyran-6-carbonitrile,
4-(5-cyano-2-hydroxyphenyl)-2,2-dimethyl-2H-1-benzopyran-6-carbonitrile or
3-(6-cyano-3,4-dihydro-2,2-dimethyl-2H-1-benzopyran-4-yl)-2(1H)-pyridone.

**16.** 2-(6-Chloro-3,4-dihydro-2,2-dimethyl-2H-1-benzopyran-4-yl)pyridine N-oxide,
2-(2,2,6-trimethyl-2H-1-benzopyran-4-yl)pyridine N-oxide,
2-[6-(trifluoromethyl)-2,2-dimethyl-2H-1-benzopyran-4-yl]pyridine N-oxide,
2-[6-(t-butyl)-3,4-dihydro-2,2-dimethyl-2H-1-benzopyran-4-yl]pyridine N-oxide,
2-(6-benzoyl-3,4-dihydro-2,2-dimethyl-2H-1-benzopyran-4-yl)pyridine N-oxide,
2-[3,4-dihydro-2,2-dimethyl-6-(4-nitrobenzoyl)-2H-1-benzopyran-4-yl]pyridine N-oxide,
2-[3,4-dihydro-2,2-dimethyl-6-(2-iodobenzoyl)-2H-1-benzopyran-4-yl]pyridine N-oxide,
2-[3,4-dihydro-2,2-dimethyl-6-(3-iodobenzoyl)-2H-1-benzopyran-4-yl]pyridine N-oxide,
2-[3,4-dihydro-2,2-dimethyl-6-(4-iodobenzoyl)-2H-1-benzopyran-4-yl]pyridine N-oxide,
2-(6-cyano-2-ethyl-2-methyl-2H-1-benzopyran-4-yl)pyridine N-oxide,
2-(6-acetyl-2-methyl-2H-1-benzopyran-4-yl)pyridine N-oxide,
2-chloro-6-(6-cyano-3,4-dihydro-2,2-dimethyl-2H-1-benzopyran-4-yl)pyridine N-oxide,
4-chloro-2-(6-cyano-3,4-dihydro-2,2-dimethyl-2H-1-benzopyran-4-yl)pyridine N-oxide,
2-(6-cyano-3,4-dihydro-2,2-dimethyl-2H-1-benzopyran-4-yl)-4-methoxypyridine N-oxide,
2-amino-6-(6-cyano-2,2-dimethyl-2H-1-benzopyran-4-yl)pyridine N-oxide,
2-amino-6-(6-cyano-3,4-dihydro-2,2-dimethyl-2H-1-benzopyran-4-yl)pyridine N-oxide,
2-(6-cyano-3,4-dihydro-2,2-dimethyl-2H-1-benzopyran-4-yl)-6-methylpyridine N-oxide,
2-(6-cyano-2,2-dimethyl-2H-1-benzopyran-4-yl)-4-methylpyridine N-oxide,
2-(6-cyano-3,4-dihydro-2,2-dimethyl-2H-1-benzopyran-4-yl)-4-methylpyridine N-oxide,
2-(6-cyano-3,4-dihydro-2,2-dimethyl-2H-1-benzopyran-4-yl)-5-(methoxycarbonyl)pyridine N-oxide,
5-amino-2-(6-cyano-2,2-dimethyl-2H-1-benzopyran-4-yl)pyridine N-oxide,
5-amino-2-(6-cyano-3,4-dihydro-2,2-dimethyl-2H-1-benzopyran-4-yl)pyridine N-oxide,
2-(6-cyano-2,2-dimethyl-2H-1-benzopyran-4-yl)-5-hydroxypyridine N-oxide,
5-chloro-2-(6-cyano-2,2-dimethyl-2H-1-benzopyran-4-yl)pyridine N-oxide,
5-chloro-2-(6-cyano-3,4-dihydro-2,2-dimethyl-2H-1-benzopyran-4-yl)pyridine N-oxide,
2-(6-cyano-2,2-dimethyl-2H-1-benzopyran-4-yl)-5-phenylpyridine N-oxide,
2-(6-acetyl-2,2-dimethyl-2H-1-benzopyran-4-yl)pyridine N-oxide,
2-(6-cyano-2,2-dimethyl-2H-1-benzopyran-4-yl)-5-methylpyridine N-oxide,
2-(6-cyano-3,4-dihydro-2,2-dimethyl-2H-1-benzopyran-4-yl)-5-methylpyridine N-oxide,
2-(6-bromo-2,2-dimethyl-2H-1-benzopyran-4-yl)pyridine N-oxide,
2-(6-cyano-2,2-dimethyl-2H-1-benzopyran-4-yl)-5-(4-methylphenyl)pyridine N-oxide,
2-(6-acetyl-2-methyl-2-phenyl-2H-1-benzopyran-4-yl)pyridine N-oxide,
5-benzyloxy-2-(6-cyano-3,4-dihydro-2,2-dimethyl-2H-1-benzopyran-4-yl)pyridine N-oxide,
2-(6-cyano-3,4-dihydro-2,2-dimethyl-2H-1-benzopyran-4-yl)-5-hydroxypyridine N-oxide,
rac-trans-2-(6-cyano-3,4-dihydro-3-hydroxy-2,2-dimethyl-2H-1-benzopyran-4-yl)-pyridine N-oxide,
rac-cis-2-(6-cyano-3,4-dihydro-3-hydroxy-2,2-dimethyl-2H-1-benzopyran-4-yl)-pyridine N-oxide,
6-(6-cyano-3,4-dihydro-2,2-dimethyl-2H-1-benzopyran-4-yl)pyrimidine 1-oxide,
2-(6-cyano-3,4-dihydro-2,2-dimethyl-2H-1-benzopyran-4-yl)pyrazine 1-oxide,
4-(6-acetyl-2,2-dimethyl-2H-1-benzopyran-4-yl)-3-pyridinol,
4-(2,2-dimethyl-2H-1-benzopyran-4-yl)-3-pyridinol,
(-)-2-(6-cyano-3,4-dihydro-2,2-dimethyl-2H-1-benzopyran-4-yl)pyridine N-oxide or
(+)-2-(6-cyano-3,4-dihydro-2,2-dimethyl-2H-1-benzopyran-4-yl)pyridine N-oxide.

**17.** A compound of the general formula

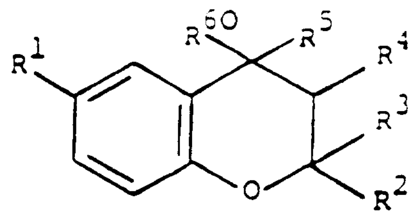

II

wherein $R^1$ represents hydrogen, halogen, trifluoromethyl, nitro, cyano, lower alkyl, lower alkoxycarbonyl, lower alkylthio, lower alkylsulphonyl, $C_{2-7}$-alkanoyl, aroyl, carbamoyl, mono(lower alkyl)-carbamoyl or di(lower alkyl)carbamoyl, $R^2$ represents hydrogen, lower alkyl or phenyl, $R^3$ represents hydrogen or lower alkyl, $R^4$ represents hydrogen or hydroxy and $R^5$ represent hydrogen or $R^4$ and $R^5$ together represent a carbon-carbon bond and $R^{60}$ represents an aryl group having a lower alkoxy group in the 2-position, 2-lower alkoxy-3-pyridyl, 2-lower alkoxy-4-methyl-3-pyridyl or 3-lower alkoxy-4-pyridyl, whereby the residues denoted as "lower" have 1-7 carbon atoms and "aroyl" signifies benzoyl optionally substituted by halogen or nitro and "aryl" signifies phenyl or naphthyl optionally mono- or multiply-substituted by halogen, cyano or lower alkyl.

**18.** A compound of the general formula

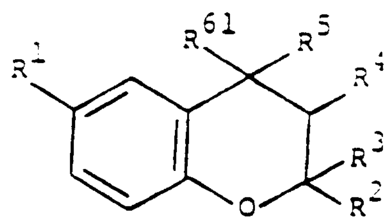

III

wherein $R^1$ represents hydrogen, halogen, trifluoromethyl, nitro, cyano, lower alkyl, lower alkoxycarbonyl, lower alkylthio, lower alkylsulphonyl, $C_{2-7}$-alkanoyl, aroyl, carbamoyl, mono(lower alkyl)-carbamoyl or di(lower alkyl)carbamoyl, $R^2$ represents hydrogen, lower alkyl or phenyl, $R^3$ represents hydrogen or lower alkyl, $R^4$ represents hydrogen or hydroxy and $R^5$ represents hydrogen or $R^4$ and $R^5$ together represent a carbon-carbon bond and $R^{61}$ represents a 2-pyridyl group, which is optionally substituted by halogen, amino, hydroxy, benzyloxy, phenyl, lower alkylphenyl, lower alkyl, lower alkoxy or lower alkoxycarbonyl, 2-pyrazinyl, 2-pyrimidinyl, 6-pyrimidinyl or 2-quinolyl, whereby the residues denoted as "lower" have 1-7 carbon atoms and "aroyl" signifies benzoyl optionally substituted by halogen or nitro.

**19.** A compound of the general formula

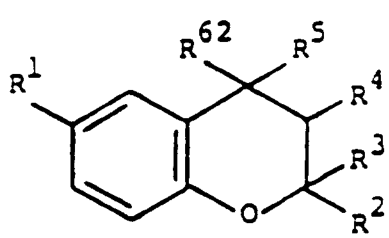

IV

wherein $R^1$ represents hydrogen, halogen, trifluoromethyl, nitro, cyano, lower alkyl, lower alkoxycarbonyl, lower alkylthio, lower alkylsulphonyl, $C_{2-7}$-alkanoyl, aroyl, carbamoyl, mono(lower alkyl)-carbamoyl or di(lower alkyl)carbamoyl, $R^2$ represents hydrogen, lower alkyl or phenyl, $R^3$ represents hydrogen or lower alkyl, $R^4$ represents hydrogen or hydroxy and $R^5$ represents hydrogen or $R^4$ and $R^5$ together represent a carbon-carbon bond and $R^{62}$ represents 3-pyridyl 1-oxide or 4-methyl-3-pyridyl 1-oxide, whereby the residues denoted as "lower" have 1-7 carbon atoms and "aroyl" signifies benzoyl

optionally substituted by halogen or nitro.

**20.** A compound of the general formula

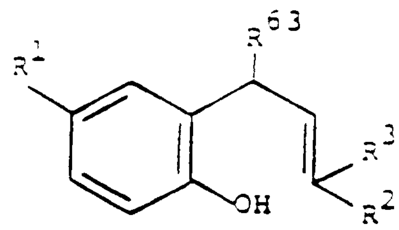

V

wherein $R^1$ represents hydrogen, halogen, trifluoromethyl, nitro, cyano, lower alkyl, lower alkoxycarbonyl, lower alkylthio, lower alkylsulphonyl, $C_{2-7}$alkanoyl, aroyl, carbamoyl, mono(lower alkyl)carbamoyl or di(lower alkyl)carbamoyl, $R^2$ represents hydrogen, lower alkyl or phenyl, $R^3$ represents hydrogen or lower alkyl and $R^{63}$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide, whereby the residues denoted as "lower" have 1-7 carbon atoms and "aroyl" signifies benzoyl optionally substituted by halogen or nitro.

**21.** A compound according to any one of claims 1 to 16 or a pharmaceutically acceptable acid addition salt of such a compound which is basic for use as a therapeutically active substance.

**22.** A compound according to any one of claims 1-16 or a pharmaceutically acceptable acid addition salt of such a compound which is basic for use in the control or prevention of hypertension, congestive heart failure, angina pectoris, peripheral and cerebral vascular disease and smooth muscle disorders.

**23.** A process for the manufacture of a compound of formula I given in claim 1 or of a pharmaceutically acceptable acid addition salt of such a compound which is basic,
characterized by

a) for the manufacture of a compound of formula I in which $R^6$ represents an aryl group having a hydroxy group in the 2-position, 2-hydroxy-3-pyridyl, 2-hydroxy-4-methyl-3-pyridyl or 3-hydroxy-4-pyridyl, converting the lower alkoxy group in a compound of the general formula

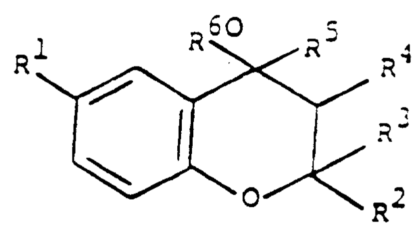

II

wherein $R^1$, $R^2$, $R^3$, $R^4$ and $R^5$ have the significance given in claim 1 and $R^{60}$ represents an aryl group having a lower alkoxy group in the 2-position, 2-lower alkoxy-3-pyridyl, 2-lower alkoxy-4-methyl-3-pyridyl or 3-lower alkoxy-4-pyridyl,
into a hydroxy group, or

b) for the manufacture of a compound of formula I in which $R^6$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide, oxidizing a compound of the general formula

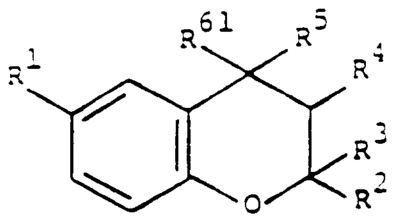

III

wherein $R^1$, $R^2$, $R^3$, $R^4$ and $R^5$ have the significance given in claim 1 and $R^{61}$ represents a 2-pyridyl group, which is optionally substituted by halogen, amino, hydroxy, benzyloxy, phenyl, lower alkylphenyl, lower alkyl, lower alkoxy or lower alkoxycarbonyl, 2-pyrazinyl, 2-pyrimidinyl, 6-pyrimidinyl or 2-quinolyl,

or

c) for the manufacture of a compound of formula I in which $R^6$ represents 2-hydroxy-3-pyridyl or 2-hydroxy-4-methyl-3-pyridyl, reacting a compound of the general formula

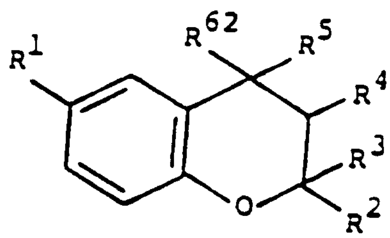

IV

wherein $R^1$, $R^2$, $R^3$, $R^4$ and $R^5$ have the significance given in claim 1 and $R^{62}$ represents 3-pyridyl 1-oxide or 4-methyl-4-pyridyl 1-oxide,

with a lower alkanoic acid anhydride and hydrolyzing the product obtained, or

d) for the manufacture of a compound of formula I in which $R^4$ and $R^5$ each represent hydrogen and $R^6$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide, cyclizing a compound of the general formula

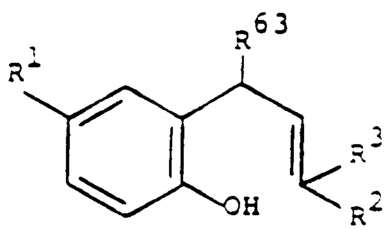

V

wherein $R^1$, $R^2$ and $R^3$ have the significance given above and $R^{63}$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide,

or

e) for the manufacture of a compound of formula I in which $R^1$ represents lower alkylsulphonyl, oxidizing a compound of formula I in which $R^1$ represents lower alkylthio, or

f) for the manufacture of a compound of formula I in which $R^1$ represents $C_{2-7}$-alkanoyl or aroyl, $C_{2-7}$-alkanoylating or aroylating a compound of formula I in which $R^1$ represents hydrogen, or

g) for the manufacture of a compound of formula I in which $R^6$ represents a 2-pyridyl 1-oxide group having a lower alkoxy group in the o- or p-position to the 1-oxide group, reacting a compound of formula I in which $R^6$ represents a 2-pyridyl 1-oxide group having a halogen atom in the o- or p-position to the 1-oxide group with an alkali metal lower alkoxide at an elevated temperature, or

h) for the manufacture of a compound of formula I in which $R^4$ and $R^5$ each represent hydrogen and $R^6$ represents a hydroxy-substituted 2-pyridyl 1-oxide group, catalytically hydrogenating a compound of formula I in which $R^4$ and $R^5$ each represent hydrogen and $R^6$ represents a benzyloxy-substituted 2-pyridyl 1-oxide group, or

i) for the manufacture of a compound of formula I in which $R^4$ and $R^5$ together represent a carbon-carbon bond and $R^6$ represents a 3-hydroxy-4-pyridyl group, reacting a 3-[N,N-di(lower alkyl)-carbamoyloxy] pyridine with a compound of the general formula

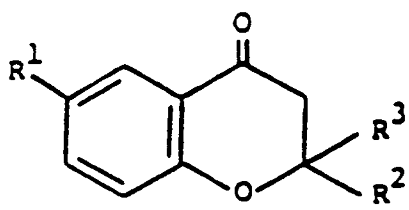

VI

wherein $R^1$, $R^2$ and $R^3$ have the significance given above,
in the presence of an alkali metal alkyl compound, and/or

j) if desired, separating a mixture of diastereoisomeric racemates obtained into the diastereoisomeric racemates or optically pure diastereoisomers, and/or

k) if desired, resolving a racemate obtained into the two optical antipodes, and/or

l) if desired, separating a cis/trans mixture obtained into the cis and trans isomers, and

m) if desired, converting a basic compound of formula I obtained into a pharmaceutically acceptable acid addition salt.

**24.** A medicament containing a compound according to any one of claims 1-16 or a pharmaceutically acceptable acid addition salt of such a compound which is basic and a therapeutically inert excipient.

**25.** A medicament for the control of hypertension, congestive heart failure, angina pectoris, peripheral and cerebral vascular disease and smooth muscle disorders, containing a compound according to any one of claims 1-16 or a pharmaceutically acceptable acid addition salt of such a compound which is basic and a therapeutically inert excipient.

**26.** The use of a compound according to any one of claims 1-16 or a pharmaceutically acceptable acid addition salt of such a compound which is basic for the manufacture of a medicament for the control or prevention of hypertension, congestive heart failure, angina pectoris, peripheral and cerebral vascular disease and smooth muscle disorders.

**Claims for the following Contracting State : ES**

**1.** A process for the manufacture of compounds of the general formula

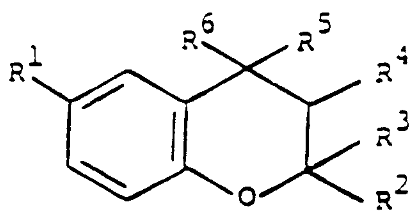

I

wherein $R^1$ represents hydrogen, halogen, trifluoromethyl, nitro, cyano, lower alkyl, lower alkoxycarbonyl, lower alkylthio, lower alkylsulphonyl, $C_{2-7}$-alkanoyl, aroyl, carbamoyl, mono(lower alkyl)-carbamoyl or di(lower alkyl)carbamoyl, $R^2$ represents hydrogen, lower alkyl or phenyl, $R^3$ represents hydrogen or lower alkyl, $R^4$ represents hydrogen or hydroxy and $R^5$ represents hydrogen or $R^4$ and $R^5$ together represent a carbon-carbon bond and $R^6$ represents an aryl group carrying a hydroxy group in the 2-position, a 2-pyridyl 1-oxide group, which is optionally substituted by halogen, amino, hydroxy,

benzyloxy, phenyl, lower alkylphenyl, lower alkyl, lower alkoxy or lower alkoxycarbonyl, 2-hydroxy-3-pyridyl, 2-hydroxy-4-methyl-3-pyridyl, 3-hydroxy-4-pyridyl, 2-pyrazinyl 1-oxide, 2-pyrimidinyl 1-oxide, 6-pyrimidinyl 1-oxide or 2-quinolyl 1-oxide, whereby the residues denoted as "lower" have 1-7 carbon atoms and "aroyl" signifies benzoyl optionally substituted by halogen or nitro and "aryl" signifies phenyl or naphthyl optionally mono- or multiply-substituted by halogen, cyano or lower alkyl,
and pharmaceutically acceptable acid addition salts of those compounds of formula I which are basic, characterized by

a) for the manufacture of a compound of formula I in which $R^6$ represents an aryl group having a hydroxy group in the 2-position, 2-hydroxy-3-pyridyl, 2-hydroxy-4-methyl-3-pyridyl or 3-hydroxy-4-pyridyl, converting the lower alkoxy group in a compound of the general formula

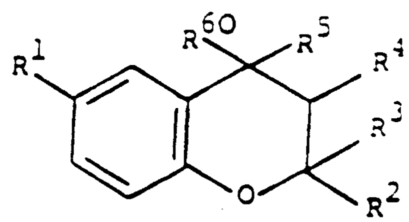

II

wherein $R^1$, $R^2$, $R^3$, $R^4$ and $R^5$ have the significance given above and $R^{60}$ represents an aryl group having a lower alkoxy group in the 2-position, 2-lower alkoxy-3-pyridyl, 2-lower alkoxy-4-methyl-3-pyridyl or 3-lower alkoxy-4-pyridyl,
into a hydroxy group, or

b) for the manufacture of a compound of formula I in which $R^6$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide, oxidizing a compound of the general formula

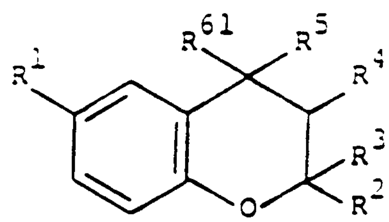

III

wherein $R^1$, $R^2$, $R^3$, $R^4$ and $R^5$ have the significance given above and $R^{61}$ represents a 2-pyridyl group, which is optionally substituted by halogen, amino, hydroxy, benzyloxy, phenyl, lower alkylphenyl, lower alkyl, lower alkoxy or lower alkoxycarbonyl, 2-pyrazinyl, 2-pyrimidinyl, 6-pyrimidinyl or 2-quinolyl,
or

c) for the manufacture of a compound of formula I in which $R^6$ represents 2-hydroxy-3-pyridyl or 2-hydroxy-4-methyl-3-pyridyl, reacting a compound of the general formula

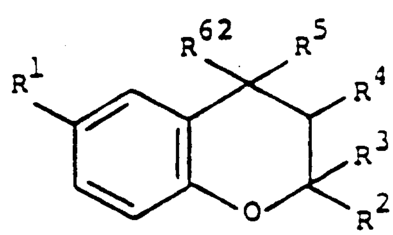

IV

wherein $R^1$, $R^2$, $R^3$, $R^4$ and $R^5$ have the significance given above and $R^{62}$ represents 3-pyridyl 1-oxide or 4-methyl-4-pyridyl 1-oxide,
with a lower alkanoic acid anhydride and hydrolyzing the product obtained, or

d) for the manufacture of a compound of formula I in which $R^4$ and $R^5$ each represent hydrogen and $R^6$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide, cyclizing a compound of the general formula

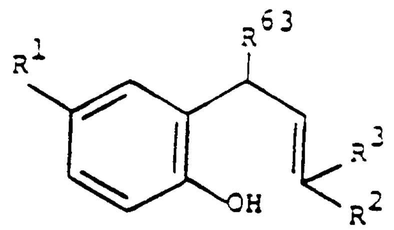

V

wherein $R^1$, $R^2$ and $R^3$ have the significance given above and $R^{63}$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide,

or

e) for the manufacture of a compound of formula I in which $R^1$ represents lower alkylsulphonyl, oxidizing a compound of formula I in which $R^1$ represents lower alkylthio, or

f) for the manufacture of a compound of formula I in which $R^1$ represents $C_{2-7}$-alkanoyl or aroyl, $C_{2-7}$-alkanoylating or aroylating a compound of formula I in which $R^1$ represents hydrogen, or

g) for the manufacture of a compound of formula I in which $R^6$ represents a 2-pyridyl 1-oxide group having a lower alkoxy group in the o- or p-position to the 1-oxide group, reacting a compound of formula I in which $R^6$ represents a 2-pyridyl 1-oxide group having a halogen atom in the o- or p-position to the 1-oxide group with an alkali metal lower alkoxide at an elevated temperature, or

h) for the manufacture of a compound of formula I in which $R^4$ and $R^5$ each represent hydrogen and $R^6$ represents a hydroxy-substituted 2-pyridyl 1-oxide group, catalytically hydrogenating a compound of formula I in which $R^4$ and $R^5$ each represent hydrogen and $R^6$ represents a benzyloxy-substituted 2-pyridyl 1-oxide group, or

i) for the manufacture of a compound of formula I in which $R^4$ and $R^5$ together represent a carbon-carbon bond and $R^6$ represents a 3-hydroxy-4-pyridyl group, reacting a 3-[N,N-di(lower alkyl)-carbamoyloxy] pyridine with a compound of the general formula

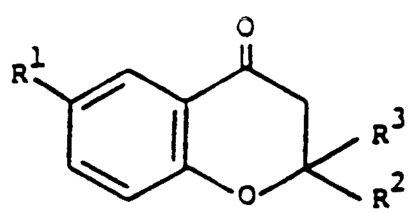

VI

wherein $R^1$, $R^2$ and $R^3$ have the significance given above,

in the presence of an alkali metal alkyl compound, and/or

j) if desired, separating a mixture of diastereoisomeric racemates obtained into the diastereoisomeric racemates or optically pure diastereoisomers, and/or

k) if desired, resolving a racemate obtained into the two optical antipodes, and/or

l) if desired, separating a cis/trans mixture obtained into the cis and trans isomers, and

m) if desired, converting a basic compound of formula I obtained into a pharmaceutically acceptable acid addition salt.

**2.** A process according to claim 1, wherein $R^1$ represents hydrogen, halogen, nitro, cyano, lower alkyl, lower alkoxycarbonyl, lower alkylthio, lower alkylsulphonyl, $C_{2-7}$-alkanoyl, carbamoyl, mono(lower alkyl)carbamoyl or di(lower alkylcarbamoyl), $R^2$ and $R^3$ each represent hydrogen or lower alkyl and $R^4$ and $R^5$ each represent hydrogen or together represent a carbon-carbon bond.

3. A process according to claim 1 or 2, wherein $R^1$ represents nitro, cyano or $C_{2-7}$-alkanoyl.

4. A process according to claim 3, wherein $R^1$ represents nitro, cyano or acetyl.

5. A process according to any one of claims 1-4, wherein $R^2$ and $R^3$ each represent lower alkyl.

6. A process according to claim 5, wherein $R^2$ and $R^3$ each represent methyl.

7. A process according to any one of claims 1-6, wherein $R^4$ and $R^5$ each represent hydrogen or together represent a carbon-carbon bond.

8. A process according to any one of claims 1-7, wherein $R^6$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide.

9. A process according to claim 8, wherein $R^6$ represents a 2-pyridyl 1-oxide group which is optionally substituted by halogen, amino, hydroxy, benzyloxy, phenyl, lower alkylphenyl, lower alkyl, lower alkoxy or lower alkoxycarbonyl.

10. A process according to any one of claims 1-9, wherein $R^1$ represents nitro, cyano or acetyl, $R^2$ and $R^3$ each represent methyl, $R^4$ and $R^5$ each represent hydrogen or together represent a carbon-carbon bond and $R^6$ represents a 2-pyridyl 1-oxide group which is optionally substituted by halogen, amino, hydroxy, benzyloxy, phenyl, lower alkylphenyl, lower alkyl or lower alkoxycarbonyl.

11. A process according to claim 2, wherein 2-(3,4-dihydro-2,2-dimethyl-6-nitro-2H-1-benzopyran-4-yl)-pyridine N-oxide is manufactured.

12. A process according to claim 2, wherein 2-(6-acetyl-3,4-dihydro-2,2-dimethyl-2H-1-benzopyran-4 -yl)-pyridine N-oxide is manufactured.

13. A process according to claim 2, wherein 2-(6-cyano-3,4-dihydro-2,2-dimethyl-2H-1-benzopyran-4-yl)-pyridine N-oxide is manufactured.

14. A process according to claim 2, wherein 2-(6-cyano-3,4-dihydro-2,2-dimethyl-2H-1-benzopyran-4-yl)-5-phenylpyridine N-oxide is manufactured.

15. The use of a compound of formula I in claim 1 or a pharmaceutically acceptable acid addition salt of such a compound which is basic for the manufacture of a medicament for the control or prevention of hypertension, congestive heart failure, angina pectoris, peripheral and cerebral vascular disease and smooth muscle disorders.

**Claims for the following Contracting State : GR**

1. A process for the manufacture of compounds of the general formula

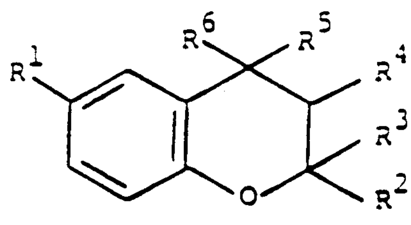

I

wherein $R^1$ represents hydrogen, halogen, trifluoromethyl, nitro, cyano, lower alkyl, lower alkoxycarbonyl, lower alkylthio, lower alkylsulphonyl, $C_{2-7}$-alkanoyl, aroyl, carbamoyl, mono(lower alkyl)-carbamoyl or di(lower alkyl)carbamoyl, $R^2$ represents hydrogen, lower alkyl or phenyl, $R^3$ represents hydrogen or lower alkyl, $R^4$ represents hydrogen or hydroxy and $R^5$ represents hydrogen or $R^4$ and $R^5$

together represent a carbon-carbon bond and $R^6$ represents an aryl group carrying a hydroxy group in the 2-position, a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-hydroxy-3-pyridyl, 2-hydroxy-4-methyl-3-pyridyl, 3-hydroxy-4-pyridyl, 2-pyrazinyl 1-oxide, 2-pyrimidinyl 1-oxide, 6-pyrimidinyl 1-oxide or 2-quinolyl 1-oxide, whereby the residues denoted as "lower" have 1-7 carbon atoms and "aroyl" signifies benzoyl optionally substituted by halogen or nitro and "aryl" signifies phenyl or naphthyl optionally mono- or multiply-substituted by halogen, cyano or lower alkyl,
and pharmaceutically acceptable acid addition salts of those compounds of formula I which are basic, characterized by

a) for the manufacture of a compound of formula I in which $R^6$ represents an aryl group having a hydroxy group in the 2-position, 2-hydroxy-3-pyridyl, 2-hydroxy-4-methyl-3-pyridyl or 3-hydroxy-4-pyridyl, converting the lower alkoxy group in a compound of the general formula

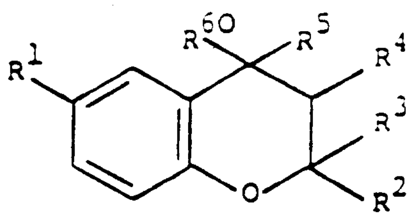

II

wherein $R^1$, $R^2$, $R^3$, $R^4$ and $R^5$ have the significance given above and $R^{60}$ represents an aryl group having a lower alkoxy group in the 2-position, 2-lower alkoxy-3-pyridyl, 2-lower alkoxy-4-methyl-3-pyridyl or 3-lower alkoxy-4-pyridyl,
into a hydroxy group, or

b) for the manufacture of a compound of formula I in which $R^6$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide, oxidizing a compound of the general formula

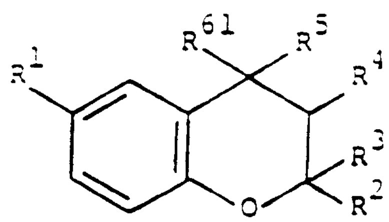

III

wherein $R^1$, $R^2$, $R^3$, $R^4$ and $R^5$ have the significance given above and $R^{61}$ represents a 2-pyridyl group, which is optionally substituted by halogen, amino, hydroxy, benzyloxy, phenyl, lower alkylphenyl, lower alkyl, lower alkoxy or lower alkoxycarbonyl, 2-pyrazinyl, 2-pyrimidinyl, 6-pyrimidinyl or 2-quinolyl,
or

c) for the manufacture of a compound of formula I in which $R^6$ represents 2-hydroxy-3-pyridyl or 2-hydroxy-4-methyl-3-pyridyl, reacting a compound of the general formula

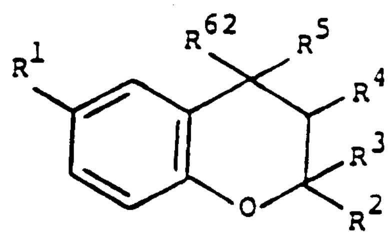

IV

wherein $R^1$, $R^2$, $R^3$, $R^4$ and $R^5$ have the significance given above and $R^{62}$ represents 3-pyridyl 1-oxide or 4-methyl-4-pyridyl 1-oxide,
with a lower alkanoic acid anhydride and hydrolyzing the product obtained, or
d) for the manufacture of a compound of formula I in which $R^4$ and $R^5$ each represent hydrogen and $R^6$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide, cyclizing a compound of the general formula

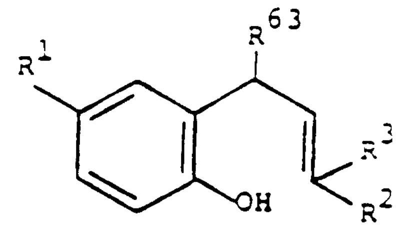

V

wherein $R^1$, $R^2$ and $R^3$ have the significance given above and $R^{63}$ represents a 2-pyridyl 1-oxide group, which is iptionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide,
or
e) for the manufacture of a compound of formula I in which $R^1$ represents lower alkylsulphonyl, oxidizing a compound of formula I in which $R^1$ represents lower alkylthio, or
f) for the manufacture of a compound of formula I in which $R^1$ represents $C_{2-7}$-alkanoyl or aroyl, $C_{2-7}$-alkanoylating or aroylating a compound of formula I in which $R^1$ represents hydrogen, or
g) for the manufacture of a compound of formula I in which $R^6$ represents a 2-pyridyl 1-oxide group having a lower alkoxy group in the o- or p-position to the 1-oxide group, reacting a compound of formula I in which $R^6$ represents a 2-pyridyl 1-oxide group having a halogen atom in the o- or p-position to the 1-oxide group with an alkali metal lower alkoxide at an elevated temperature, or
h) for the manufacture of a compound of formula I in which $R^4$ and $R^5$ each represent hydrogen and $R^6$ represents a hydroxy-substituted 2-pyridyl 1-oxide group, catalytically hydrogenating a compound of formula I in which $R^4$ and $R^5$ each represent hydrogen and $R^6$ represents a benzyloxy-substituted 2-pyridyl 1-oxide group, or
i) for the manufacture of a compound of formula I in which $R^4$ and $R^5$ together represent a carbon-carbon bond and $R^6$ represents a 3-hydroxy-4-pyridyl group, reacting a 3-[N,N-di(lower alkyl)-carbamoyloxy] pyridine with a compound of the general formula

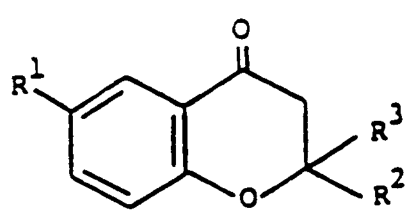

VI

wherein $R^1$, $R^2$ and $R^3$ have the significance given above,
in the presence of an alkali metal alkyl compound, and/or
j) if desired, separating a mixture of diastereoisomeric racemates obtained into the diastereoisomeric racemates or optically pure diastereoisomers, and/or
k) if desired, resolving a racemate obtained into the two optical antipodes, and/or
l) if desired, separating a cis/trans mixture obtained into the cis and trans isomers, and
m) if desired, converting a basic compound of formula I obtained into a pharmaceutically acceptable acid addition salt.

**2.** A process according to claim 1, wherein $R^1$ represents hydrogen, halogen, nitro, cyano, lower alkyl, lower alkoxycarbonyl, lower alkylthio, lower alkylsulphonyl, $C_{2-7}$-alkanoyl, carbamoyl, mono(lower

alkyl)carbamoyl or di(lower alkylcarbamoyl), $R^2$ and $R^3$ each represent hydrogen or lower alkyl and $R^4$ and $R^5$ each represent hydrogen or together represent a carbon-carbon bond.

**3.** A process according to claim 1 or 2, wherein $R^1$ represents nitro, cyano or $C_{2-7}$-alkanoyl.

**4.** A process according to claim 3, wherein $R^1$ represents nitro, cyano or acetyl.

**5.** A process according to any one of claims 1-4, wherein $R^2$ and $R^3$ each represent lower alkyl.

**6.** A process according to claim 5, wherein $R^2$ and $R^3$ each represent methyl.

**7.** A process according to any one of claims 1-6, wherein $R^4$ and $R^5$ each represent hydrogen or together represent a carbon-carbon bond.

**8.** A process according to any one of claims 1-7, wherein $R^6$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide.

**9.** A process according to claim 8, wherein $R^6$ represents a 2-pyridyl 1-oxide group which is optionally substituted by halogen, amino, hydroxy, benzyloxy, phenyl, lower alkylphenyl, lower alkyl, lower alkoxy or lower alkoxycarbonyl.

**10.** A process according to any one of claims 1-9, wherein $R^1$ represents nitro, cyano or acetyl, $R^2$ and $R^3$ each represent methyl, $R^4$ and $R^5$ each represent hydrogen or together represent a carbon-carbon bond and $R^6$ represents a 2-pyridyl 1-oxide group which is optionally substituted by halogen, amino, hydroxy, benzyloxy, phenyl, lower alkylphenyl, lower alkyl or lower alkoxycarbonyl.

**11.** A process according to claim 2, wherein 2-(3,4-dihydro-2,2-dimethyl-6-nitro-2H-1-benzopyran-4-yl)-pyridine N-oxide is manufactured.

**12.** A process according to claim 2, wherein 2-(6-acetyl-3,4-dihydro-2,2-dimethyl-2H-1-benzopyran-4 -yl)-pyridine N-oxide is manufactured.

**13.** A process according to claim 2, wherein 2-(6-cyano-3,4-dihydro-2,2-dimethyl-2H-1-benzopyran-4-yl)-pyridine N-oxide is manufactured.

**14.** A process according to claim 2, wherein 2-(6-cyano-3,4-dihydro-2,2-dimethyl-2H-1-benzopyran-4-yl)-5-phenylpyridine N-oxide is manufactured.

**15.** The use of a compound of formula I in claim 1 or a pharmaceutically acceptable acid addition salt of such a compound which is basic for the manufacture of a medicament for the control or prevention of hypertension, congestive heart failure, angina pectoris, peripheral and cerebral vascular disease and smooth muscle disorders.

**16.** A compound of the general formula

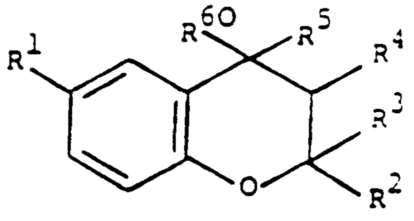

II

wherein $R^1$ represents hydrogen, halogen, trifluoromethyl, nitro, cyano, lower alkyl, lower alkoxycarbonyl, lower alkylthio, lower alkylsulphonyl, $C_{2-7}$-alkanoyl, aroyl, carbamoyl, mono(lower alkyl)-

carbamoyl or di(lower alkyl)carbamoyl, $R^2$ represents hydrogen, lower alkyl or phenyl, $R^3$ represents hydrogen or lower alkyl, $R^4$ represents hydrogen or hydroxy and $R^5$ represent hydrogen or $R^4$ and $R^5$ together represent a carbon-carbon bond and $R^{60}$ represents an aryl group having a lower alkoxy group in the 2-position, 2-lower alkoxy-3-pyridyl, 2-lower alkoxy-4-methyl-3-pyridyl or 3-lower alkoxy-4-pyridyl, whereby the residues denoted as "lower" have 1-7 carbon atoms and "aroyl" signifies benzoyl optionally substituted by halogen or nitro and "aryl" signifies phenyl or naphthyl optionally mono- or multiply-substituted by halogen, cyano or lower alkyl.

**17.** A compound of the general formula

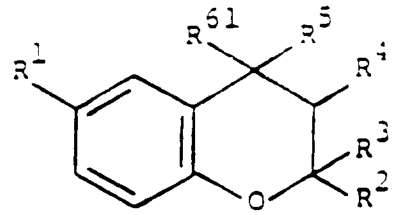

III

wherein $R^1$ represents hydrogen, halogen, trifluoromethyl, nitro, cyano, lower alkyl, lower alkoxycarbonyl, lower alkylthio, lower alkylsulphonyl, $C_{2-7}$-alkanoyl, aroyl, carbamoyl, mono(lower alkyl)-carbamoyl or di(lower alkyl)carbamoyl, $R^2$ represents hydrogen, lower alkyl or phenyl, $R^3$ represents hydrogen or lower alkyl, $R^4$ represents hydrogen or hydroxy and $R^5$ represents hydrogen or $R^4$ and $R^5$ together represent a carbon-carbon bond and $R^{61}$ represents a 2-pyridyl group, which is optionally substituted by halogen, amino, hydroxy, benzyloxy, phenyl, lower alkylphenyl, lower alkyl, lower alkoxy or lower alkoxycarbonyl, 2-pyrazinyl, 2-pyrimidinyl, 6-pyrimidinyl or 2-quinolyl, whereby the residues denoted as "lower" have 1-7 carbon atoms and "aroyl" signifies benzoyl optionally substituted by halogen or nitro.

**18.** A compound of the general formula

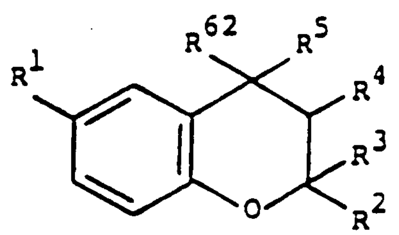

IV

wherein $R^1$ represents hydrogen, halogen, trifluoromethyl, nitro, cyano, lower alkyl, lower alkoxycarbonyl, lower alkylthio, lower alkylsulphonyl, $C_{2-7}$-alkanoyl, aroyl, carbamoyl, mono(lower alkyl)-carbamoyl or di(lower alkyl)carbamoyl, $R^2$ represents hydrogen, lower alkyl or phenyl, $R^3$ represents hydrogen or lower alkyl, $R^4$ represents hydrogen or hydroxy and $R^5$ represents hydrogen or $R^4$ and $R^5$ together represent a carbon-carbon bond and $R^{62}$ represents 3-pyridyl 1-oxide or 4-methyl-3-pyridyl 1-oxide, whereby the residues denoted as "lower" have 1-7 carbon atoms and "aroyl" signifies benzoyl optionally substituted by halogen or nitro.

**19.** A compound of the general formula

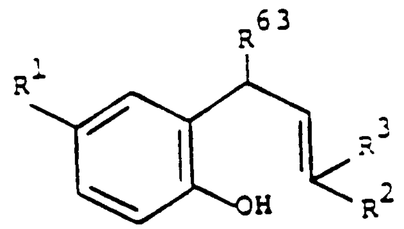

V

wherein $R^1$ represents hydrogen, halogen, trifluoromethyl, nitro, cyano, lower alkyl, lower alkoxycarbonyl, lower alkylthio, lower alkylsulphonyl, $C_{2-7}$-alkanoyl, aroyl, carbamoyl, mono(lower alkyl)-carbamoyl or di(lower alkyl)carbamoyl, $R^2$ represents hydrogen, lower alkyl or phenyl, $R^3$ represents hydrogen or lower alkyl and $R^{63}$ represents a 2-pyridyl 1-oxide group, which is optionally substituted by halogen, amino, hydroxy, benzyloxy, phenyl, lower alkylphenyl, lower alkyl, lower alkoxy or lower alkoxycarbonyl, 2-pyrazinyl 1-oxide, 2-pyrimidinyl 1-oxide, 6-pyrimidinyl 1-oxide or 2-quinolyl 1-oxide, whereby the residues denoted as "lower" have 1-7 carbon atoms and "aroyl" signifies benzoyl optionally substituted by halogen or nitro.

**Revendications**

**Revendications pour les Etats contractants suivants : AT, BE, CH, DE, FR, GB, IT, LI, LU, NL, SE**

**1.** Composés de formule générale

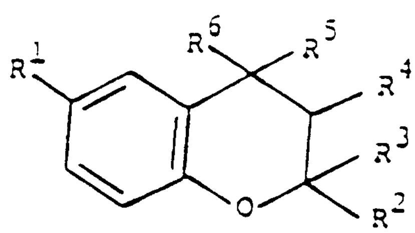

I

dans laquelle $R^1$ représente un hydrogène, un halogène, un trifluorométhyle, un nitro, un cyano, un alcoyle inférieur, un alcoxy inférieur carbonyle, un alcoyle inférieur thio, un alcoyle inférieur sulfonyle, un alcanoyle en $C_{2-7}$, un aroyle, un carbamoyle, un mono(alcoyle inférieur)carbamoyle ou un di(alcoyle inférieur)carbamoyle, $R^2$ représente un hydrogène, un alcoyle inférieur ou un phényle, $R^3$ représente un hydrogène ou un alcoyle inférieur, $R^4$ représente un hydrogène ou un hydroxy et $R^5$ représente un hydrogène, ou $R^4$ et $R^5$ représentent ensemble une liaison carbone-carbone et $R^6$ représente un groupe aryle,

qui porte un groupe hydroxy en position 2, un groupe 2-pyridyl-1-oxyde, qui est éventuellement substitué par un halogène, un amino, un hydroxy, un benzyloxy, un phényle, un alcoyle inférieur-phényle, un alcoyle inférieur, un alcoxy inférieur ou un alcoxy inférieur carbonyle, ou encore un 2-hydroxy-3-pyridyle, 2-hydroxy-4-méthyl-3-pyridyle, 3-hydroxy-4-pyridyle, 2-pyrazinyl-1-oxyde, 2-pyrimidinyl-1-oxyde, 6-pyrimidinyl-1-oxyde ou 2-quinoléyl-1-oxyde, où les radicaux décrits comme "inférieurs" présentent de 1 à 7 atomes de carbone et "aroyle" représente un benzoyle éventuellement substitué par un halogène ou un nitro et "aryle" un phényle ou un naphtyle éventuellement mono- ou polysubstitué par un halogène, un cyano ou un alcoyle inférieur,

et les sels d'addition d'acides pharmaceutiquement acceptables des composés de formule I qui sont basiques.

**2.** Composés selon la revendication 1, où $R^1$ représente un hydrogène, un halogène, un nitro, un cyano, un alcoyle inférieur, un alcoxy inférieur carbonyle, un alcoyle inférieur thio, un alcoyle inférieur sulfonyle, un alcanoyle en $C_{2-7}$, un carbamoyle, un mono(alcoyle inférieur)carbamoyle, ou un di-(alcoyle inférieur)carbamoyle, $R^2$ et $R^3$ représentent chacun un hydrogène ou un alcoyle inférieur et $R^4$

et $R^5$ représentent chacun un hydrogène ou représentent ensemble une liaison carbone-carbone.

**3.** Composés selon la revendication 1 ou 2, où $R^1$ représente un nitro, un cyano ou un alcanoyle en $C_{2-7}$.

**4.** Composés selon la revendication 3, où $R^1$ représente un nitro, un cyano ou un acétyle.

**5.** Composés selon l'une des revendications 1-4, où $R^2$ et $R^3$ représentent chacun un alcoyle inférieur.

**6.** Composés selon la revendication 5, où $R^2$ et $R^3$ représentent un méthyle.

**7.** Composés selon l'une des revendications 1-6, où $R^4$ et $R^5$ représentent chacun un hydrogène ou $R^4$ et $R^5$ représentent ensemble une liaison carbone-carbone.

**8.** Composés selon l'une des revendications 1-7, où $R^6$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur-carbonyle, ou encore un 2-pyrazinyl-1-oxyde, un 2-pyrimidinyl-1-oxyde, un 6-pyrimidinyl-1-oxyde ou un 2-quinoléyl-1-oxyde.

**9.** Composés selon la revendication 8, où $R^6$ représente un groupe 2-pyridyl-1-oxyde, qui est éventuellement substitué par un halogène, un amino, un hydroxy, un benzyloxy, un phényle, un alcoyle inférieur-phényle, un alcoyle inférieur, un alcoxy inférieur ou un alcoxy inférieur carbonyle.

**10.** Composés selon l'une des revendications 1-9, où $R^1$ représente un nitro, un cyano ou un acétyle, $R^2$ et $R^3$ représentent chacun un méthyle, $R^4$ et $R^5$ représentent un hydrogène ou ensemble une liaison carbone-carbone et $R^6$ un groupe 2-pyridyl-1-oxyde, qui est éventuellement substitué par un halogène, un amino, un hydroxy, un benzyloxy, un phényle, un alcoyle inférieur phényle, un alcoyle inférieur ou un alcoxy inférieur-carbonyle.

**11.** 2-(3,4-dihydro-2,2-diméthyl-6-nitro-2H-1-benzopyran-4-yl)pyridine-N-oxyde.

**12.** 2-(6-acétyl-3,4-dihydro-2,2-diméthyl-2H-1-benzopyran-4-yl)pyridine-N-oxyde.

**13.** 2-(6-cyano-3,4-dihydro-2,2-diméthyl-2H-1-benzopyran-4-yl)pyridine-N-oxyde.

**14.** 2-(6-cyano-3,4-dihydro-2,2-diméthyl-2H-1-benzopyran-4-yl)-5-phénylpyridine-N-oxyde.

**15.** 2-(6-acétyl-3,4-dihydro-2,2-diméthyl-2H-1-benzopyran-4-yl)-3-méthylpyridine-N-oxyde,
2-(3,4-dihydro-2,2-diméthyl-6-nitro-2H-1-benzopyran-4-yl)-3-méthylpyridine-N-oxyde,
2-(6-cyano-2,2-diméthyl-2H-1-benzopyran-4-yl)pyridine-N-oxyde,
2-(3,4-dihydro-2,2-diméthyl-2H-1-benzopyran-4-yl)pyridine-N-oxyde,
2-(3,4-dihydro-2,2-diiméthyl-6-méthylthio-2H-1-benzopyran-4-yl)pyridine-N-oxyde,
2-(3,4-dihydro-2,2-diméthyl-6-méthylsulfonyl-2H-1-benzopyran-4-yl)pyridine-N-oxyde,
2-(2,2-diméthyl-2H-1-benzopyran-4-yl)pyridine-N-oxyde,
2-(6-bromo-3,4-dihydro-2,2-diméthyl-2H-1-benzopyran-4-yl)pyridine-N-oxyde,
2-[6-(méthoxycarbonyl)-2,2-diméthyl-2H-1-benzopyran-4-yl)]pyridine-N-oxyde,
2-[3,4-dihydro-6-(méthoxycarbonyl)-2,2-diméthyl-2H-1-benzopyran-4-yl]pyridine-N-oxyde,
2-(6-carbamoyl-3,4-dihydro-2,2-diméthyl-2H-1-benzopyran-4-yl)pyridine-N-oxyde,
2-(6-cyano-3,4-dihydro-2,2-diméthyl-2H-1-benzopyran-4-yl)pyrimidine-1-oxyde,
2-(6-cyano-2,2-diméthyl-2H-1-benzopyran-4-yl)quinoléine-1-oxyde,
2-(6-cyano-3,4-dihydro-2,2-diméthyl-2H-1-benzopyran-4-yl)quinoléine-1-oxyde,
4-(2-hydroxyphényl)-2,2-diméthyl-2H-1-benzopyran-6-carbonitrile,
4-(5-cyano-2-hydroxyphényl)-2,2-diméthyl-2H-1-benzopyran-6-carbonitrile ou
3-(6-cyano-3,4-dihydro-2,2-diméthyl-2H-1-benzopyran-4-yl)-2(1H)-pyridone.

**16.** 2-(6-chloro-3,4-dihydro-2,2-diméthyl-2H-1-benzopyran-4-yl)pyridine-N-oxyde,
2-(2,2,6-triméthyl-2H-1-benzopyran-4-yl)pyridine-N-oxyde,
2-[6-(trifluorométhyl)-2,2-diméthyl-2H-1-benzopyran-4-yl]pyridine-N-oxyde,
2-[6-(t-butyl)-3,4-dihydro-2,2-diméthyl-2H-1-benzopyran-4-yl]pyridine-N-oxyde,

2-(6-benzoyl-3,4-dihydro-2,2-diméthyl-2H-1-benzopyran-4-yl)pyridine-N-oxyde,
2-[3,4-dihydro-2,2-diméthyl-6-(4-nitrobenzoyl)-2H-1-benzopyran-4-yl]pyridine-N-oxyde,
2-[3,4-dihydro-2,2-diméthyl-6-(2-iodobenzoyl)-2H-1-benzopyran-4-yl]pyridine-N-oxyde,
2-[3,4-dihydro-2,2-diméthyl-6-(3-iodobenzoyl)-2H-1-benzopyran-4-yl]pyridine-N-oxyde,
2-[3,4-dihydro-2,2-diméthyl-6-(4-iodobenzoyl)-2H-1-benzopyran-4-yl]pyridine-N-oxyde,
2-(6-cyano-2-éthyl-2-méthyl-2H-1-benzopyran-4-yl)pyridine-N-oxyde,
2-(6-acétyl-2-méthyl-2H-1-benzopyran-4-yl)pyridine-N-oxyde,
2-chloro-6-(6-cyano-3,4-dihydro-2,2-diméthyl-2H-1-benzopyran-4-yl)pyridine-N-oxyde,
4-chloro-2-(6-cyano-3,4-dihydro-2,2-diméthyl-2H-1-benzopyran-4-yl)pyridine-N-oxyde,
2-(6-cyano-3,4-dihydro-2,2-diméthyl-2H-1-benzopyran-4-yl)-4-méthoxypyridine-N-oxyde,
2-amino-6-(6-cyano-2,2-diméthyl-2H-1-benzopyran-4-yl)pyridine-N-oxyde,
2-amino-6-(6-cyano-3,4-dihydro-2,2-diméthyl-2H-1-benzopyran-4-yl)pyridine-N-oxyde,
2-(6-cyano-3,4-dihydro-2,2-diméthyl-2H-1-benzopyran-4-yl)-6-méthylpyridine-N-oxyde,
2-(6-cyano-2,2-diméthyl-2H-1-benzopyran-4-yl)-4-méthylpyridine-N-oxyde,
2-(6-cyano-3,4-dihydro-2,2-diméthyl-2H-1-benzopyran-4-yl)-4-méthylpyridine-N-oxyde,
2-(6-cyano-3,4-dihydro-2,2-diméthyl-2H-1-benzopyran-4-yl)-5-(méthoxycarbonyl)pyridine-N-oxyde,
5-amino-2-(6-cyano-2,2-diméthyl-2H-1-benzopyran-4-yl)pyridine-N-oxyde,
5-amino-2-(6-cyano-3,4-dihydro-2,2-diméthyl-2H-1-benzopyran-4-yl)pyridine-N-oxyde,
2-(6-cyano-2,2-diméthyl-2H-1-benzopyran-4-yl)-5-hydroxypyridine-N-oxyde,
5-chloro-2-(6-cyano-2,2-diméthyl-2H-1-benzopyran-4-yl)pyridine-N-oxyde,
5-chloro-2-(6-cyano-3,4-dihydro-2,2-diméthyl-2H-1-benzopyran-4-yl)pyridine-N-oxyde,
2-(6-cyano-2,2-diméthyl-2H-1-benzopyran-4-yl)-5-phénylpyridine-N-oxyde,
2-(6-acétyl-2,2-diméthyl-2H-1-benzopyran-4-yl)pyridine-N-oxyde,
2-(6-cyano-2,2-diméthyl-2H-1-benzopyran-4-yl)-5-méthylpyridine-N-oxyde,
2-(6-cyano-3,4-dihydro-2,2-diméthyl-2H-1-benzopyran-4-yl)-5-méthylpyridine-N-oxyde,
2-(6-bromo-2,2-diméthyl-2H-1-benzopyran-4-yl)pyridine-N-oxyde,
2-(6-cyano-2,2-diméthyl-2H-1-benzopyran-4-yl)-5-(4-méthylphényl)pyridine-N-oxyde,
2-(6-acétyl-2-méthyl-2-phényl-2H-1-benzopyran-4-yl)pyridine-N-oxyde,
5-benzyloxy-2-(6-cyano-3,4-dihydro-2,2-diméthyl-2H-1-benzopyran-4-yl)pyridine-N-oxyde.
2-(6-cyano-3,4-dihydro-2,2-diméthyl-2H-1-benzopyran-4-yl)-5-hydroxypyridine-N-oxyde.
rac-trans-2-(6-cyano-3,4-dihydro-3-hydroxy-2,2-diméthyl-2H-1-benzopyran-4-yl)pyridine-N-oxyde.
rac-cis-2-(6-cyano-3,4-dihydro-3-hydroxy-2,2-diméthyl-2H-1-benzopyran-4-yl)pyridine-N-oxyde.
6-(6-cyano-3,4-dihydro-2,2-diméthyl-2H-1-benzopyran-4-yl)pyrimidine-1-oxyde.
2-(6-cyano-3,4-dihydro-2,2-diméthyl-2H-1-benzopyran-4-yl)pyrazine-1-oxyde,
4-(6-acétyl-2,2-diméthyl-2H-1-benzopyran-4-yl)-3-pyridinol.
4-(2,2-diméthyl-2H-1-benzopyran-4-yl)-3-pyridinol.
(-)-2-(6-cyano-3,4-dihydro-2,2-diméthyl-2H-1-benzopyran-4-yl)pyridine-N-oxyde ou
(+)-2-(6-cyano-3,4-dihydro-2,2-diméthyl-2H-1-benzopyran-4-yl)pyridine-N-oxyde.

**17.** Composé de formule générale

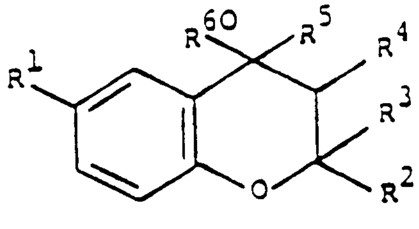

II

dans laquelle $R^1$ représente un hydrogène, un halogène, un trifluorométhyle, un nitro, un cyano, un alcoyle inférieur, un alcoxy inférieur-carbonyle, un alcoyle inférieur-thio, un alcoyle inférieur sulfonyle, un alcoyle en $C_{2-7}$, un aroyle, un carbamoyle, un mono(alcoyle inférieur)carbamoyle ou un di(alcoyle inférieur)carbamoyle, $R^2$ représente un hydrogène, un alcoyle inférieur ou un phényle, $R^3$ représente un hydrogène ou un alcoyle inférieur, $R^4$ représente un hydrogène ou un hydroxy et $R^5$ un hydrogène, ou $R^4$ et $R^5$ représentent ensemble une liaison carbone-carbone, et $R^{60}$ un groupe aryle avec un groupe alcoxy inférieur en position 2, un 2-alcoxy inférieur-3-pyridyle, un 2-alcoxy inférieur-4-méthyl-3-pyridyle, ou un 3-alcoxy inférieur-4-pyridyle, où les radicaux décrits comme "inférieurs"présentent de 1 à 7

atomes de carbone et "aroyle" représente un benzoyle éventuellement substitué par un halogène ou un nitro et "aryle" un phényle ou un naphtyle éventuellement mono- ou polysubstitué par un halogène, un cyano ou un alcoyle inférieur.

**18.** Composé de formule générale

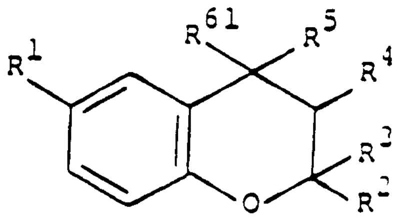

III

dans laquelle $R^1$ représente un hydrogène, un halogène, un trifluorométhyle, un nitro, un cyano, un alcoyle inférieur, un alcoxy inférieur carbonyle, un alcoyle inférieur thio, un alcoyle inférieur sulfonyle, un alcanoyle en $C_{2-7}$, un aroyle, un carbamoyle, un mono(alcoyle inférieur)carbamoyle ou un di(alcoyle inférieur)carbamoyle, $R^2$ représente un hydrogène, un alcoyle inférieur ou un phényle, $R^3$ représente un hydrogène ou un alcoyle inférieur, $R^4$ représente un hydrogène ou un hydroxy et $R^5$ représente un hydrogène, ou $R^4$ et $R^5$ représentent ensemble une liaison carbone-carbone et $R^{61}$ représente un groupe 2-pyridyle, qui est éventuellement substitué par un halogène, un amino, un hydroxy, un benzyloxy, un phényle, un alcoyle inférieur-phényle, un alcoyle inférieur, un alcoxy inférieur ou un alcoxy inférieur-carbonyle, ou encore un 2-pyrazinyle, un 2-pyrimidinyle, un 6-pyrimidinyle ou un 2-quinoléyle, où les radicaux décrits comme "inférieurs" présentent de 1 à 7 atomes de carbone et "aroyle" représente un benzoyle éventuellement substitué par un halogène ou un nitro.

**19.** Composé de formule générale

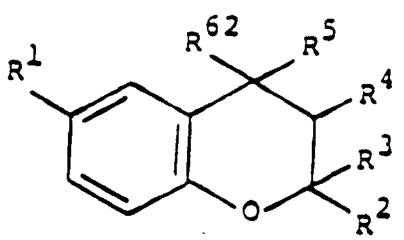

IV

dans laquelle $R^1$ représente un hydrogène, un halogène, un trifluorométhyle, un nitro, un cyano, un alcoyle inférieur, un alcoxy inférieur-carbonyle, un alcoyle inférieur-thio, un alcoyle inférieur-sulfonyle, un alcanoyle en $C_{2-7}$, un aroyle, un carbamoyle, un mono(alcoyle inférieur)carbamoyle ou un di(alcoyle inférieur)carbamoyle, $R^2$ représente un hydrogène, un alcoyle inférieur ou un phényle, $R^3$ représente un hydrogène ou un alcoyle inférieur, $R^4$ représente un hydrogène ou un hydroxy et $R^5$ représente un hydrogène ou $R^4$ et $R^5$ représentent ensemble une liaison carbone-carbone et $R^{62}$ représente un 3-pyridyl-1-oxyde ou un 4-méthyl-3-pyridyl-1-oxyde, où les radicaux décrits comme "inférieurs" présentent de 1 à 7 atomes de carbone et "aroyle" représente un benzoyle éventuellement substitué par un halogène ou un nitro.

**20.** Composé de formule générale

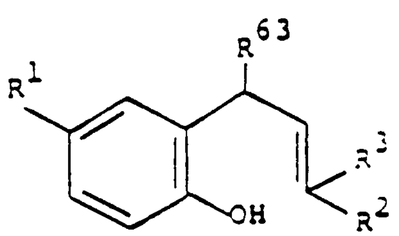

V

dans laquelle $R^1$ représente un hydrogène, un halogène, un trifluorométhyle, un nitro, un cyano, un alcoyle inférieur, un alcoxy inférieur-carbonyle, un alcoyle inférieur-thio, un alcoyle inférieur-sulfonyle, un alcanoyle en $C_{2-7}$, un aroyle, un carbamoyle, un mono(alcoyle inférieur)carbamoyle, ou un di-(alcoyle inférieur)carbamoyle, $R^2$ représente un hydrogène, un alcoyle inférieur ou un phényle, $R^3$ représente un hydrogène ou un alcoyle inférieur et $R^{63}$ représente un groupe 2-pyridyl-1-oxyde, qui est éventuellement substitué par un halogène, un amino, un hydroxy, un benzyloxy, un phényle, un alcoyle inférieur-phényle, un alcoyle inférieur, un alcoxy inférieur ou un alcoxy inférieur-carbonyle, ou encore un 2-pyrazinyl-1-oxyde, un 2-pyrimidinyl-1-oxyde, un 6-pyrimidinyl-1-oxyde ou un 2-quinoléyl-1-oxyde, où les radicaux décrits comme "inférieurs" présentent de 1 à 7 atomes de carbone et "aroyle" représente un benzoyle éventuellement substitué par un halogène ou un nitro.

**21.** Composé selon l'une des revendications 1-16 ou un sel d'addition d'acides pharmaceutiquement acceptable d'un tel composé, qui est basique, aux fins d'application comme substance active thérapeutique.

**22.** Composé selon l'une des revendications 1-16 ou un sel d'addition d'acides pharmaceutiquement acceptable d'un tel composé, qui est basique, aux fins d'application pour combattre ou prévenir l'hypertension artérielle, l'insuffisance cardiaque décompensée, l'angine de poitrine, les maladies vasculaires périphériques ou cérébrales ou les désordres de la musculature lisse.

**23.** Procédé de préparation d'un composé de formule I indiqué dans la revendication 1 ou d'un des sels d'addition d'acides pharmaceutiquement acceptable d'un tel composé, qui est basique, caractérisé en ce que :

a) pour préparer un composé de formule I dans laquelle $R^6$ représente un aryle avec un groupe hydroxy en position 2, un 2-hydroxy-3-pyridyle, un 2-hydroxy-4-méthyl-3-pyridyle, ou un 3-hydroxy-4-pyridyle, on transforme en un groupe hydroxy le groupe alcoxy inférieur dans un composé de formule générale

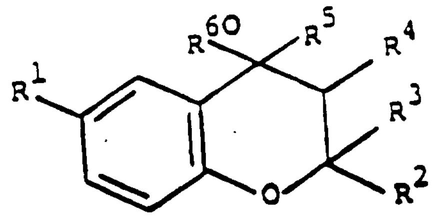

II

dans laquelle $R^1$, $R^2$, $R^3$, $R^4$ et $R^5$ ont la signification donnée dans la revendication 1 et $R^{60}$ représente un groupe aryle avec un groupe alcoxy inférieur en position 2, un 2-alcoxy inférieur-3-pyridyle, un 2-alcoxy inférieur-4-méthyl-3-pyridyle ou un 3-alcoxy inférieur-4-pyridyle, ou

b) pour préparer un composé de formule I dans laquelle $R^6$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyl-1-oxyde, un 2-pyrimidinyl-1-oxyde, un 6-pyrimidinyl-1-oxyde ou un 2-quinoléyl-1-oxyde, on oxyde un composé de formule générale

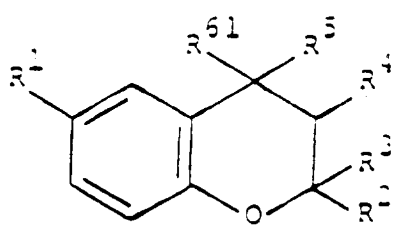

III

dans laquelle $R^1$, $R^2$, $R^3$, $R^4$ et $R^5$ ont la signification donnée dans la revendication 1 et $R^{61}$ représente un groupe 2-pyridyle, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyle, un 2-pyrimidinyle, un 6-pyrimidinyle ou un 2-quinoléyle, ou

c) pour préparer un composé de formule I, dans laquelle $R^6$ représente un 2-hydroxy-3-pyridyle ou un 2-hydroxy-4-méthyl-3-pyridyle, on fait réagir avec un anhydride d'acide alcanoïque inférieur un composé de formule générale

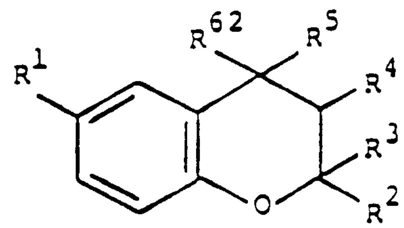

IV

dans laquelle $R^1$, $R^2$, $R^3$, $R^4$ et $R^5$ ont la signification donnée dans la revendication 1 et $R^{62}$ représente un 3-pyridyl-1-oxyde ou un 4-méthyl-3-pyridyl-1-oxyde, et on hydrolyse le produit obtenu, ou

d) pour préparer un composé de formule I dans laquelle $R^4$ et $R^5$ représentent chacun un hydrogène et $R^6$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyl-1-oxyde, un 2-pyrimidinyl-1-oxyde, un 6-pyrimidinyl-1-oxyde ou un 2-quinoléyl-1-oxyde, on cyclise un composé de formule générale

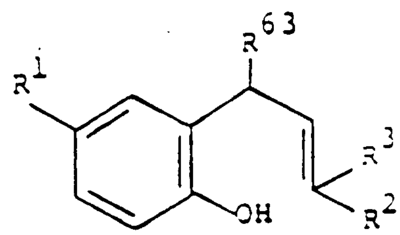

V.

dans laquelle $R^1$, $R^2$ et $R^3$ ont la signification donnée ci-dessus et $R^{63}$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyl-1-oxyde, un 2-pyrimidinyl-1-oxyde, un 6-pyrimidinyl-1-oxyde ou un 2-quinoléyl-1-oxyde, ou

e) pour préparer un composé de formule I dans laquelle $R^1$ représente un alcoyle inférieur sulfonyle, en oxyde un composé de formule I dans laquelle $R^1$ représente un alcoyle inférieur thio, ou

f) pour préparer un composé de formule I dans laquelle $R^1$ représente un alcanoyle en $C_{2-7}$ ou un aroyle, on traite par un agent donnant un alcanoyle en $C_{2-7}$ ou un aroyle un composé de formule I dans laquelle $R^1$ représente un hydrogène, ou

g) pour préparer un composé de formule I dans laquelle $R^6$ représente un groupe 2-pyridyl-1-oxyde avec un groupe alcoxy inférieur en position o ou p, par référence au groupe 1-oxyde, on fait réagir un composé de formule I, dans laquelle $R^6$ représente un groupe 2-pyridyl-1-oxyde avec un atome d'halogène en position o ou p par référence au groupe 1-oxyde, avec un alcoxyde inférieur de métal alcalin à température augmentée, ou

h) pour préparer un composé de formule I dans laquelle $R^4$ et $R^5$ représentent chacun un hydrogène et $R^6$ un groupe 2-pyridyl-1-oxyde substitué par un hydroxy, on hydrogène de façon catalytique un composé de formule I dans laquelle $R^4$ et $R^5$ représentent chacun un hydrogène et $R^6$ un groupe 2-pyridyl-1-oxyde substitué par un benzyloxy, ou

i) pour préparer un composé de formule I, dans laquelle $R^4$ et $R^5$ représentent ensemble une liaison carbone-carbone et $R^6$ un groupe 3-hydroxy-4-pyridyle, on fait réagir en présence d'un composé de métal alcalin-alcoyle une 3-[N,N-di(alcoyle inférieur)carbamoyloxy]pyridine avec un composé de formule générale

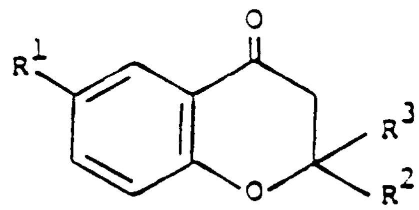

VI

dans laquelle $R^1$, $R^2$ et $R^3$ ont la signification donnée ci-dessus,
et/ou
j) si on le désire, on dédouble un mélange de racémates diastéréomères obtenus en les racémates des stéréomères correspondants ou en diastéréomères optiquement purs,
et/ou
k) si on le désire, on sépare un racémate obtenu en les deux antipodes optiques, et/ou
l) si on le désire, on dédouble un mélange cis/trans obtenu en les isomères cis et trans, et
m) si on le désire, on transforme un composé basique de formule I obtenu en un sel d'addition d'acide pharmaceutiquement acceptable.

**24.** Médicament contenant un composé selon l'une des revendications 1-16 ou un sel d'addition d'acides pharmaceutiquement acceptable d'un tel composé, qui est basique, et un excipient thérapeutiquement inerte.

**25.** Agent pour combattre ou prévenir l'hypertension artérielle, l'insuffisance cardiaque décompensée, l'angine de poitrine, les maladies vasculaires périphériques et cérébrales ou les désordres de la musculature lisse, contenant un composé selon l'une des revendications 1-16 ou un sel d'addition d'acides pharmaceutiquement acceptable d'un tel composé qui est basique, et un excipient thérapeutiquement inerte.

**26.** Application d'un composé selon l'une des revendications 1-16 ou d'un sel d'addition d'acides pharmaceutiquement acceptable d'un tel composé, qui est basique, à la préparation d'un agent pour combattre ou prévenir l'hypertension artérielle, l'insuffisance cardiaque décompensée, l'angine de poitrine, les maladies vasculaires périphériques et cérébrales ou les désordres de la musculature lisse.

**Revendications pour l'Etat contractant suivant : ES**

**1.** Procédé de préparation de composés de formule générale

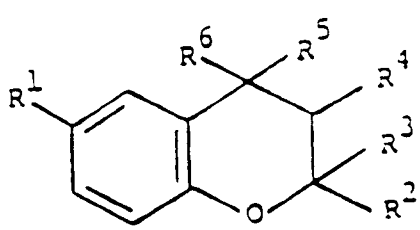

I

dans laquelle $R^1$ représente un hydrogène, un halogène, un trifluorométhyle, un nitro, un cyano, un alcoyle inférieur, un alcoxy inférieur carbonyle, un alcoyle inférieur thio, un alcoyle inférieur sulfonyle, un alcanoyle en $C_{2-7}$, un aroyle, un carbamoyle, un mono(alcoyle inférieur)carbamoyle ou un di(alcoyle inférieur)carbamoyle, $R^2$ représente un hydrogène, un alcoyle inférieur ou un phényle, $R^3$ représente un hydrogène ou un alcoyle inférieur, $R^4$ représente un hydrogène ou un hydroxy et $R^5$ représente un hydrogène, ou $R^4$ et $R^5$ représentent ensemble une liaison carbone-carbone et $R^6$ représente un groupe aryle,

qui porte un groupe hydroxy en position 2, un groupe 2-pyridyl-1-oxyde, qui est éventuellement substitué par un halogène, un amino, un hydroxy, un benzyloxy, un phényle, un alcoyle inférieur-phényle, un alcoyle inférieur, un alcoxy inférieur ou un alcoxy inférieur carbonyle, ou encore un 2-hydroxy-3-pyridyle, 2-hydroxy-4-méthyl-3-pyridyle, 3-hydroxy-4-pyridyle, 2-pyrazinyl-1-oxyde, 2-pyrimidinyl-1-oxyde, 6-pyrimidinyl-1-oxyde ou 2-quinoléyl-1-oxyde, où les radicaux décrits comme "inférieurs" présentent de 1 à 7 atomes de carbone et "aroyle" représente un benzoyle éventuellement substitué par un halogène ou un nitro et "aryle" un phényle ou un naphtyle éventuellement mono- ou polysubstitué par un halogène, un cyano ou un alcoyle inférieur,

et les sels d'addition d'acides pharmaceutiquement acceptables des composés de formule I qui sont basiques, caractérisé en ce que

a) pour préparer un composé de formule I dans laquelle $R^6$ représente un aryle

avec un groupe hydroxy en position 2, un 2-hydroxy-3-pyridyle, un 2-hydroxy-4-méthyl-3-pyridyle, ou un 3-hydroxy-4-pyridyle, on transforme en un groupe hydroxy le groupe alcoxy inférieur dans un composé de formule générale

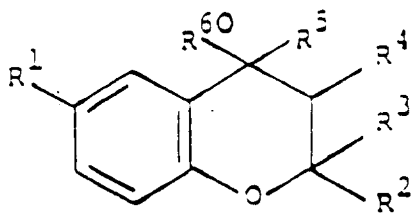

II

dans laquelle $R^1$, $R^2$, $R^3$, $R^4$ et $R^5$ ont la signification données dans la revendication 1 et $R^{60}$ représente un groupe aryle avec un groupe alcoxy inférieur en position 2, un 2-alcoxy inférieur-3-pyridyle, un 2-alcoxy inférieur-4-méthyl-3-pyridyle ou un 3-alcoxy inférieur-4-pyridyle, ou

b) pour préparer un composé de formule I dans laquelle $R^6$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyl-1-oxyde, un 2-pyrimidinyl-1-oxyde, un 6-pyrimidinyl-1-oxyde ou un 2-quinoléyl-1-oxyde, on oxyde un composé de formule générale

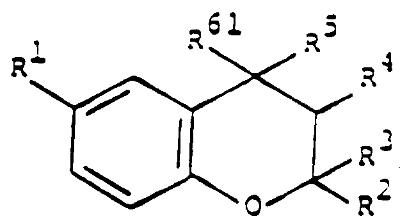

III

dans laquelle $R^1$, $R^2$, $R^3$, $R^4$ et $R^5$ ont la signification donnée dans la revendication 1 et $R^{61}$ représente un groupe 2-pyridyle, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyle, un 2-pyrimidinyle, un 6-pyrimidinyle ou un 2-quinoléyle, ou

c) pour préparer un composé de formule I, dans laquelle $R^6$ représente un 2-hydroxy-3-pyridyle ou un 2-hydroxy-4-méthyl-3-pyridyle, on fait réagir avec un anhydride d'acide alcanoïque inférieur un composé de formule générale

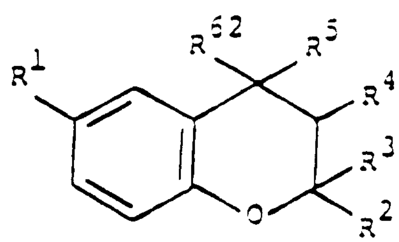

IV

dans laquelle $R^1$, $R^2$, $R^3$, $R^4$ et $R^5$ ont la signification donnée dans la revendication 1 et $R^{62}$

représente un 3-pyridyl-1-oxyde ou un 4-méthyl-3-pyridyl-1-oxyde, et on hydrolyse le produit obtenu, ou

d) pour préparer un composé de formule I dans laquelle $R^4$ et $R^5$ représentent chacun un hydrogène et $R^6$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyl-1-oxyde, un 2-pyrimidinyl-1-oxyde, un 6-pyrimidinyl-1-oxyde ou un 2-quinoléyl-1-oxyde, on cyclise un composé de formule générale

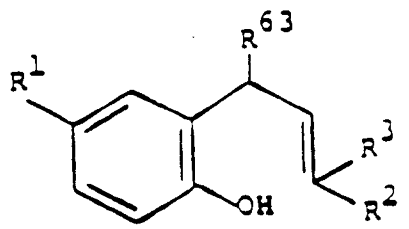

V

dans laquelle $R^1$, $R^2$ et $R^3$ ont la signification donnée ci-dessus et $R^{63}$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyl-1-oxyde, un 2-pyrimidinyl-1-oxyde, un 6-pyrimidinyl-1-oxyde ou un 2-quinoléyl-1-oxyde, ou

e) pour préparer un composé de formule I dans laquelle $R^1$ représente un alcoyle inférieur sulfonyle, en oxyde un composé de formule I dans laquelle $R^1$ représente un alcoyle inférieur thio, ou

f) pour préparer un composé de formule I dans laquelle $R^1$ représente un alcanoyle en $C_{2-7}$ ou un aroyle, on traite par un agent donnant un alcanoyle en $C_{2-7}$ ou un aroyle un composé de formule I dans laquelle $R^1$ représente un hydrogène, ou

g) pour préparer un composé de formule I dans laquelle $R^6$ représente un groupe 2-pyridyl-1-oxyde avec un groupe alcoxy inférieur en position o ou p, par référence au groupe 1-oxyde, on fait réagir un composé de formule I, dans laquelle $R^6$ représente un groupe 2-pyridyl-1-oxyde avec un atome d'halogène en position o ou p par référence au groupe 1-oxyde, avec un alcoxyde inférieur de métal alcalin à température augmentée, ou

h) pour préparer un composé de formule I dans laquelle $R^4$ et $R^5$ représentent chacun un hydrogène et $R^6$ un groupe 2-pyridyl-1-oxyde substitué par un hydroxy, on hydrogène de façon catalytique un composé de formule I dans laquelle $R^4$ et $R^5$ représentent chacun un hydrogène et $R^6$ un groupe 2-pyridyl-1-oxyde substitué par un benzyloxy, ou

i) pour préparer un composé de formule I, dans laquelle $R^4$ et $R^5$ représentent ensemble une liaison carbone-carbone et $R^6$ un groupe 3-hydroxy-4-pyridyle, on fait réagir en présence d'un composé de métal alcalin-alcoyle une 3-[N,N-di(alcoyle inférieur)carbamoyloxy]pyridine avec un composé de formule générale

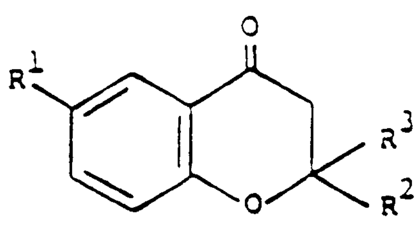

VI

dans laquelle $R^1$, $R^2$ et $R^3$ ont la signification donnée ci-dessus,

et/ou

j) si on le désire, on dédouble un mélange de racémates diastéréomères obtenus en les racémates des stéréomères correspondants ou en diastéréomères optiquement purs,

et/ou

k) si on le désire, on sépare un racémate obtenu en les deux antipodes optiques, et/ou

l) si on le désire, on dédouble un mélange cis/trans obtenu en les isomères cis et trans, et

m) si on le désire, on transforme un composé basique de formule I obtenu en un sel d'addition d'acide pharmaceutiquement acceptable.

**2.** Procédés selon la revendication 1, où $R^1$ représente un hydrogène, un halogène, un nitro, un cyano, un alcoyle inférieur, un alcoxy inférieur carbonyle, un alcoyle inférieur thio, un alcoyle inférieur sulfonyle, un alcanoyle en $C_{2-7}$, un carbamoyle, un mono(alcoyle inférieur)carbamoyle, ou un di(alcoyle inférieur)carbamoyle, $R^2$ et $R^3$ représentent chacun un hydrogène ou un alcoyle inférieur et $R^4$ et $R^5$ représentent chacun un hydrogène ou représentent ensemble une liaison carbone-carbone.

**3.** Procédés selon la revendication 1 ou 2, où $R^1$ représente un nitro, un cyano ou un alcanoyle en $C_{2-7}$.

**4.** Procédés selon la revendication 3, où $R^1$ représente un nitro, un cyano ou un acétyle.

**5.** Procédés selon l'une des revendications 1-4, où $R^2$ et $R^3$ représentent chacun un alcoyle inférieur.

**6.** Procédés selon la revendication 5, où $R^2$ et $R^3$ représentent un méthyle.

**7.** Procédés selon l'une des revendications 1-6, où $R^4$ et $R^5$ représentent chacun un hydrogène ou $R^4$ et $R^5$ représentent ensemble une liaison carbone-carbone.

**8.** Procédés selon l'une des revendications 1-7, où $R^6$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur-carbonyle, ou encore un 2-pyrazinyl-1-oxyde, un 2-pyrimidinyl-1-oxyde, un 6-pyrimidinyl-1-oxyde ou un 2-quinoléyl-1-oxyde.

**9.** Procédés selon la revendication 8, où $R^6$ représente un groupe 2-pyridyl-1-oxyde, qui est éventuellement substitué par un halogène, un amino, un hydroxy, un benzyloxy, un phényle, un alcoyle inférieur-phényle, un alcoyle inférieur, un alcoxy inférieur ou un alcoxy inférieur carbonyle.

**10.** Procédés selon l'une des revendications 1-9, où $R^1$ représente un nitro, un cyano ou un acétyle, $R^2$ et $R^3$ représentent chacun un méthyle, $R^4$ et $R^5$ représentent un hydrogène ou ensemble une liaison carbone-carbone et $R^6$ un groupe 2-pyridyl-1-oxyde, qui est éventuellement substitué par un halogène, un amino, un hydroxy, un benzyloxy, un phényle, un alcoyle inférieur phényle, un alcoyle inférieur ou un alcoxy inférieur-carbonyle.

**11.** Procédé selon la revendication 2, caractérisé en ce qu'on prépare le 2-(3,4-dihydro-2,2-diméthyl-6-nitro-2H-1-benzopyran-4-yl)pyridine-N-oxyde.

**12.** Procédé selon la revendication 2, caractérisé en ce qu'on prépare le 2-(6-acétyl-3,4-dihydro-2,2-diméthyl-2H-1-benzopyran-4-yl)pyridine-N-oxyde.

**13.** Procédé selon la revendication 2, caractérisé en ce qu'on prépare le 2-(6-cyano-3,4-dihydro-2,2-diméthyl-2H-1-benzopyran-4-yl)pyridine-N-oxyde.

**14.** Procédé selon la revendication 2, caractérisé en ce qu'on prépare le 2-(6-cyano-3,4-dihydro-2,2-diméthyl-2H-1-benzopyran-4-yl)-5-phénylpyridine-N-oxyde.

**15.** Application d'un composé de formule I de la revendication 1 ou d'un sel d'addition d'acides pharmaceutiquement acceptable d'un tel composé, qui est basique, à la préparation d'un agent pour combattre ou prévenir l'hypertension artérielle, l'insuffisance cardiaque décompensée, l'angine de poitrine, les maladies vasculaires périphériques et cérébrales ou les désordres de la musculature lisse.

**Revendications pour l'Etat contractant suivant : GR**

**1.** Procédé de préparation de composés de formule générale

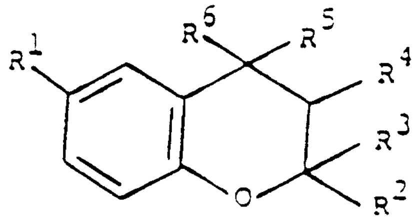

I

dans laquelle $R^1$ représente un hydrogène, un halogène, un trifluorométhyle, un nitro, un cyano, un alcoyle inférieur, un alcoxy inférieur carbonyle, un alcoyle inférieur thio, un alcoyle inférieur sulfonyle, un alcanoyle en $C_{2-7}$, un aroyle, un carbamoyle, un mono(alcoyle inférieur)carbamoyle ou un di(alcoyle inférieur)carbamoyle, $R^2$ représente un hydrogène, un alcoyle inférieur ou un phényle, $R^3$ représente un hydrogène ou un alcoyle inférieur, $R^4$ représente un hydrogène ou un hydroxy et $R^5$ représente un hydrogène, ou $R^4$ et $R^5$ représentent ensemble une liaison carbone-carbone et $R^6$ représente un groupe aryle,

qui porte un groupe hydroxy en position 2, un groupe 2-pyridyl-1-oxyde, qui est éventuellement substitué par un halogène, un amino, un hydroxy, un benzyloxy, un phényle, un alcoyle inférieur-phényle, un alcoyle inférieur, un alcoxy inférieur ou un alcoxy inférieur carbonyle, ou encore un 2-hydroxy-3-pyridyle, 2-hydroxy-4-méthyl-3-pyridyle, 3-hydroxy-4-pyridyle, 2-pyrazinyl-1-oxyde, 2-pyrimidinyl-1-oxyde, 6-pyrimidinyl-1-oxyde ou 2-quinoléyl-1-oxyde, où les radicaux décrits comme "inférieurs" présentent de 1 à 7 atomes de carbone et "aroyle" représente un benzoyle éventuellement substitué par un halogène ou un nitro et "aryle" un phényle ou un naphtyle éventuellement mono- ou polysubstitué par un halogène, un cyano ou un alcoyle inférieur,

et les sels d'addition d'acides pharmaceutiquement acceptables des composés de formule I qui sont basiques, caractérisé en ce que

a) pour préparer un composé de formule I dans laquelle $R^6$ représente un aryle

avec un groupe hydroxy en position 2, un 2-hydroxy-3-pyridyle, un 2-hydroxy-4-méthyl-3-pyridyle, ou un 3-hydroxy-4-pyridyle, on transforme en un groupe hydroxy le groupe alcoxy inférieur dans un composé de formule générale

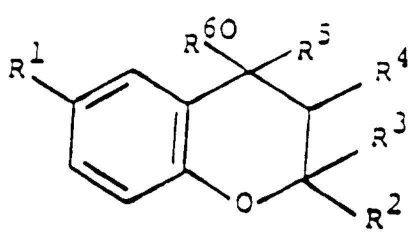

II

dans laquelle $R^1$, $R^2$, $R^3$, $R^4$ et $R^5$ ont la signification donnée dans la revendication 1 et $R^{60}$ représente un groupe aryle avec un groupe alcoxy inférieur en position 2, un 2-alcoxy inférieur-3-pyridyle, un 2-alcoxy inférieur-4-méthyl-3-pyridyle, ou un 3-alcoxy inférieur-4-pyridyle, ou

b) pour préparer un composé de formule I dans laquelle $R^6$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyl-1-oxyde, un 2-pyrimidinyl-1-oxyde, un 6-pyrimidinyl-1-oxyde ou un 2-quinoléyl-1-oxyde, on oxyde un composé de formule générale

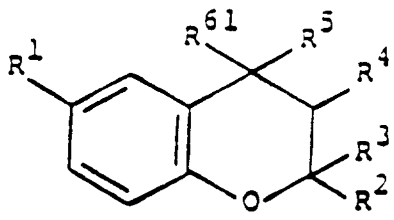

. III

dans laquelle $R^1$, $R^2$, $R^3$, $R^4$ et $R^5$ ont la signification donnée dans la revendication 1 et $R^{61}$ représente un groupe 2-pyridyle, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyle, un 2-pyrimidinyle, un 6-pyrimidinyle ou un 2-quinoléyle, ou

c) pour préparer un composé de formule I, dans laquelle $R^6$ représente un 2-hydroxy-3-pyridyle ou un 2-hydroxy-4-méthyl-3-pyridyle, on fait réagir avec un anhydride d'acide alcanoïque inférieur un composé de formule générale

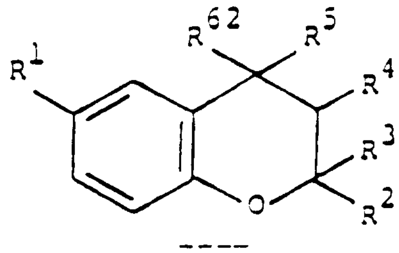

IV

dans laquelle $R^1$, $R^2$, $R^3$, $R^4$ et $R^5$ ont la signification donnée dans la revendication 1 et $R^{62}$ représente un 3-pyridyl-1-oxyde ou un 4-méthyl-3-pyridyl-1-oxyde, et on hydrolyse le produit obtenu, ou

d) pour préparer un composé de formule I dans laquelle $R^4$ et $R^5$ représentent chacun un hydrogène et $R^6$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyl-1-oxyde, un 2-pyrimidinyl-1-oxyde, un 6-pyrimidinyl-1-oxyde ou un 2-quinoléyl-1-oxyde, on cyclise un composé de formule générale

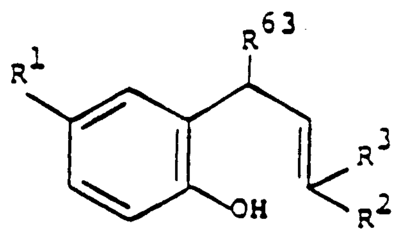

V

dans laquelle $R^1$, $R^2$ et $R^3$ ont la signification donnée ci-dessus et $R^{63}$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur carbonyle, ou encore un 2-pyrazinyl-1-oxyde, un 2-pyrimidinyl-1-oxyde, un 6-pyrimidinyl-1-oxyde ou un 2-quinoléyl-1-oxyde, ou

e) pour préparer un composé de formule I dans laquelle $R^1$ représente un alcoyle inférieur sulfonyle, en oxyde un composé de formule I dans laquelle $R^1$ représente un alcoyle inférieur thio, ou

f) pour préparer un composé de formule I dans laquelle $R^1$ représente un alcanoyle en $C_{2-7}$ ou un aroyle, on traite par un agent donnant un alcanoyle en $C_{2-7}$ ou un aroyle un composé de formule I dans laquelle $R^1$ représente un hydrogène, ou

g) pour préparer un composé de formule I dans laquelle $R^6$ représente un groupe 2-pyridyl-1-oxyde avec un groupe alcoxy inférieur en position o ou p, par référence au groupe 1-oxyde, on fait réagir un composé de formule I, dans laquelle $R^6$ représente un groupe 2-pyridyl-1-oxyde avec un atome d'halogène en position o ou p par référence au groupe l-oxyde, avec un alcoxyde inférieur de métal alcalin à température augmentée, ou

h) pour préparer un composé de formule I dans laquelle $R^4$ et $R^5$ représentent chacun un hydrogène et $R^6$ un groupe 2-pyridyl-1-oxyde substitué par un hydroxy, on hydrogène de façon catalytique un composé de formule I dans laquelle $R^4$ et $R^5$ représentent chacun un hydrogène et $R^6$ un groupe 2-pyridyl-1-oxyde substitué par un benzyloxy, ou

i) pour préparer un composé de formule I, dans laquelle $R^4$ et $R^5$ représentent ensemble une liaison carbone-carbone et $R^6$ un groupe 3-hydroxy-4-pyridyle, on fait réagir en présence d'un composé de métal alcalin-alcoyle une 3-[N,N-di(alcoyle inférieur)carbamoyloxy]pyridine avec un composé de formule générale

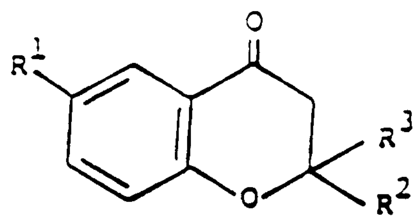

VI

dans laquelle $R^1$, $R^2$ et $R^3$ ont la signification donnée ci-dessus,

et/ou

j) si on le désire, on dédouble un mélange de racémates diastéréomères obtenus en les racémates des stéréomères correspondants ou en diastéréomères optiquement purs,

et/ou

k) si on le désire, on sépare un racémate obtenu en les deux antipodes optiques, et/ou

l) si on le désire, on dédouble un mélange cis/trans obtenu en les isomères cis et trans, et

m) si on le désire, on transforme un composé basique de formule I obtenu en un sel d'addition d'acide pharmaceutiquement acceptable.

**2.** Procédés selon la revendication 1, où $R^1$ représente un hydrogène, un halogène, un nitro, un cyano, un alcoyle inférieur, un alcoxy inférieur carbonyle, un alcoyle inférieur thio, un alcoyle inférieur sulfonyle, un alcanoyle en $C_{2-7}$, un carbamoyle, un mono(alcoyle inférieur)carbamoyle, ou un di(alcoyle inférieur)carbamoyle, $R^2$ et $R^3$ représentent chacun un hydrogène ou un alcoyle inférieur et $R^4$ et $R^5$ représentent chacun un hydrogène ou représentent ensemble une liaison carbone-carbone.

**3.** Procédés selon la revendication 1 ou 2, où $R^1$ représente un nitro, un cyano ou un alcanoyle en $C_{2-7}$.

**4.** Procédés selon la revendication 3, où $R^1$ représente un nitro, un cyano ou un acétyle.

**5.** Procédés selon l'une des revendications 1-4, où $R^2$ et $R^3$ représentent chacun un alcoyle inférieur.

**6.** Procédés selon la revendication 5, où $R^2$ et $R^3$ représentent un méthyle.

**7.** Procédés selon l'une des revendications 1-6, où $R^4$ et $R^5$ représentent chacun un hydrogène ou $R^4$ et $R^5$ représentent ensemble une liaison carbone-carbone.

**8.** Procédés selon l'une des revendications 1-7, où $R^6$ représente un groupe 2-pyridyl-1-oxyde, qui est éventuellement substitué par un halogène, un amino, un hydroxy, un benzyloxy, un phényle, un alcoyle inférieur phényle, un alcoyle inférieur, un alcoxy inférieur ou un alcoxy inférieur-carbonyle, ou encore un 2-pyrazinyl-1-oxyde, un 2-pyrimidinyl-1-oxyde, un 6-pyrimidinyl-1-oxyde ou un 2-quinoléyl-1-oxyde.

**9.** Procédés selon la revendication 8, où $R^6$ représente un groupe 2-pyridyl-1-oxyde, qui est éventuellement substitué par un halogène, un amino, un hydroxy, un benzyloxy, un phényle, un alcoyle inférieur-phényle, un alcoyle inférieur, un alcoxy inférieur ou un alcoxy inférieur carbonyle.

**10.** Procédés selon l'une des revendications 1-9, où $R^1$ représente un nitro, un cyano ou un acétyle, $R^2$ et $R^3$ représentent chacun un méthyle, $R^4$ et $R^5$ représentent un hydrogène ou ensemble une liaison carbone-carbone et $R^6$ un groupe 2-pyridyl-1-oxyde, qui est éventuellement substitué par un halogène, un amino, un hydroxy, un benzyloxy, un phényle, un alcoyle inférieur phényle, un alcoyle inférieur ou un alcoxy inférieur-carbonyle.

**11.** Procédé selon la revendication 2, caractérisé en ce qu'on prépare le 2-(3,4-dihydro-2,2-diméthyl-6-nitro-2H-1-benzopyran-4-yl)pyridine-N-oxyde.

**12.** Procédé selon la revendication 2, caractérisé en ce qu'on prépare le 2-(6-acétyl-3,4-dihydro-2,2-diméthyl-2H-1-benzopyran-4-yl)pyridine-N-oxyde.

**13.** Procédé selon la revendication 2, caractérisé en ce qu'on prépare le 2-(6-cyano-3,4-dihydro-2,2-diméthyl-2H-1-benzopyran-4-yl)pyridine-N-oxyde.

**14.** Procédé selon la revendication 2, caractérisé en ce qu'on prépare le 2-(6-cyano-3,4-dihydro-2,2-diméthyl-2H-1-benzopyran-4-yl)-5-phénylpyridine-N-oxyde.

**15.** Application d'un composé de formule I de la revendication 1 ou d'un sel d'addition d'acides pharmaceutiquement acceptable d'un tel composé, qui est basique, à la préparation d'un agent pour combattre ou prévenir l'hypertension artérielle, l'insuffisance cardiaque décompensée, l'angine de poitrine, les maladies vasculaires périphériques et cérébrales ou les désordres de la musculature lisse.

**16.** Composé de formule générale

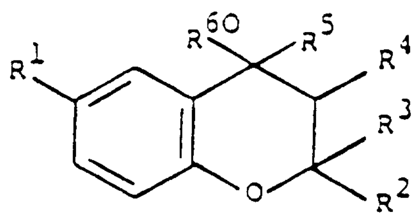

II

dans laquelle $R^1$ représente un hydrogène, un halogène, un trifluorométhyle, un nitro, un cyano, un alcoyle inférieur, un alcoxy inférieur-carbonyle, un alcoyle inférieur-thio, un alcoyle inférieur sulfonyle, un alcoyle en $C_{2-7}$, un aroyle, un carbamoyle, un mono(alcoyle inférieur)carbamoyle ou un di(alcoyle inférieur)carbamoyle, $R^2$ représente un hydrogène, un alcoyle inférieur ou un phényle, $R^3$ représente un hydrogène ou un alcoyle inférieur, $R^4$ représente un hydrogène ou un hydroxy et $R^5$ un hydrogène, ou $R^4$ et $R^5$ représentent ensemble une liaison carbone-carbone, et $R^{60}$ un groupe aryle avec un groupe alcoxy inférieur en position 2, un 2-alcoxy inférieur-3-pyridyle, un 2-alcoxy inférieur-4-méthyl-3-pyridyle, ou un 3-alcoxy inférieur-4-pyridyle, où les radicaux décrits comme "inférieurs" présentent de 1 à 7 atomes de carbone et "aroyle" représente un benzoyle éventuellement substitué par un halogène ou un nitro et "aryle" un phényle ou un naphtyle éventuellement mono- ou polysubstitué par un halogène, un cyano ou un alcoyle inférieur.

**17.** Composé de formule générale

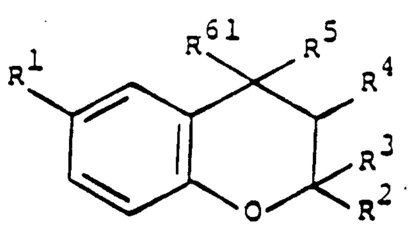

III

dans laquelle $R^1$ représente un hydrogène, un halogène, un trifluorométhyle, un nitro, un cyano, un

alcoyle inférieur, un alcoxy inférieur carbonyle, un alcoyle inférieur thio, un alcoyle inférieur sulfonyle, un alcanoyle en $C_{2-7}$, un aroyle, un carbamoyle, un mono(alcoyle inférieur)carbamoyle ou un di(alcoyle inférieur)carbamoyle, $R^2$ représente un hydrogène, un alcoyle inférieur ou un phényle, $R^3$ représente un hydrogène ou un alcoyle inférieur, $R^4$ représente un hydrogène ou un hydroxy et $R^5$ représente un hydrogène, ou $R^4$ et $R^5$ représentent ensemble une liaison carbone-carbone et $R^{61}$ représente un groupe 2-pyridyle, qui est éventuellement substitué par un halogène, un amino, un hydroxy, un benzyloxy, un phényle, un alcoyle inférieur-phényle, un alcoyle inférieur, un alcoxy inférieur ou un alcoxy inférieur-carbonyle, ou encore un 2-pyrazinyle, un 2-pyrimidinyle, un 6-pyrimidinyle ou un 2-quinoléyle, où les radicaux décrits comme "inférieurs" présentent de 1 à 7 atomes de carbone et "aroyle" représente un benzoyle éventuellement substitué par un halogène ou un nitro.

**18.** Composé de formule générale

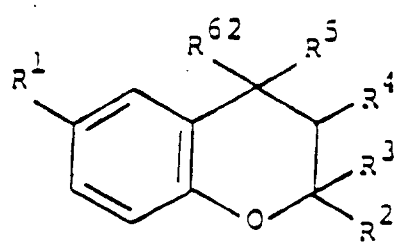

IV

dans laquelle $R^1$ représente un hydrogène, un halogène, un trifluorométhyle, un nitro, un cyano, un alcoyle inférieur, un alcoxy inférieur-carbonyle, un alcoyle inférieur-thio, un alcoyle inférieur-sulfonyle, un alcanoyle en $C_{2-7}$, un aroyle, un carbamoyle, un mono(alcoyle inférieur)carbamoyle ou un di(alcoyle inférieur)carbamoyle, $R^2$ représente un hydrogène, un alcoyle inférieur ou un phényle, $R^3$ représente un hydrogène ou un alcoyle inférieur, $R^4$ représente un hydrogène ou un hydroxy et $R^5$ représente un hydrogène ou $R^4$ et $R^5$ représentent ensemble une liaison carbone-carbone et $R^{62}$ représente un 3-pyridyl-1-oxyde ou un 4-méthyl-3-pyridyl-1-oxyde, où les radicaux décrits comme "inférieurs" présentent de 1 à 7 atomes de carbone et "aroyle" représente un benzoyle éventuellement substitué par un halogène ou un nitro.

**19.** Composé de formule générale

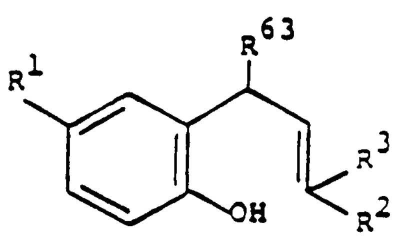

V

dans laquelle $R^1$ représente un hydrogène, un halogène, un trifluorométhyle, un nitro, un cyano, un alcoyle inférieur, un alcoxy inférieur-carbonyle, un alcoyle inférieur-thio, un alcoyle inférieur-sulfonyle, un alcanoyle en $C_{2-7}$, un aroyle, un carbamoyle, un mono(alcoyle inférieur)carbamoyle, ou un di-(alcoyle inférieur)carbamoyle, $R^2$ représente un hydrogène, un alcoyle inférieur ou un phényle, $R^3$ représente un hydrogène ou un alcoyle inférieur et $R^{63}$ représente un groupe 2-pyridyl-1-oxyde, qui est éventuellement substitué par un halogène, un amino, un hydroxy, un benzyloxy, un phényle, un alcoyle inférieur-phényle, un alcoyle inférieur, un alcoxy inférieur ou un alcoxy inférieur-carbonyle, ou encore un 2-pyrazinyl-1-oxyde, un 2-pyrimidinyl-1-oxyde, un 6-pyrimidinyl-1-oxyde ou un 2-quinoléyl-1-oxyde, où les radicaux décrits comme "inférieurs" présentent de 1 à 7 atomes de carbone et "aroyle" représente un benzoyle éventuellement substitué par un halogène ou un nitro.